# EUROPEAN PATENT SPECIFICATION

(11) **EP 1 805 519 B1**
(45) Date of publication and mention of the grant of the patent: **06.11.2013**
(21) Application number: 05812548.5
(22) Date of filing: 20.09.2005
(51) Int. Cl.: G01N 33/53, C07K 16/00

(54) **WNT PROTEINS AND DETECTION AND TREATMENT OF CANCER**
WNT-PROTEINE SOWIE ERKENNUNG UND BEHANDLUNG VON KREBS
PROTÉINES WNT ET DÉTECTION ET TRAITEMENT DU CANCER

(30) Priority: 21.09.2004 US 612098 P; 05.01.2005 WO PCT/US2005/000267
(43) Date of publication of application: 11.07.2007
(73) Proprietor: RHODE ISLAND HOSPITAL, Providence, RI 02902 (US)
(72) Inventor: WANDS, Jack, R., Providence, RI 02903 (US); KIM, Miran, North Providence, RI 02911 (US)
(74) Representative: Dörries, Hans Ulrich
(86) International application number: PCT/US2005/033775
(87) International publication number: WO 2006/034328

(56) References cited:
- WO-A-03/092705
- US-A- 6 043 053
- WILLERT JENNIFER ET AL: "A transcriptional response to Wnt protein in human embryonic carcinoma cells" BMC DEVELOPMENTAL BIOLOGY, BIOMED CENTRAL LTD., LONDON, GB, vol. 2, no. 1, 2 July 2002 (2002-07-02), page 8, XP021014141 ISSN: 1471-213X
- MERLE ET AL: "Functional consequences of frizzled-7 receptor overexpression in human hepatocellular carcinoma" GASTROENTEROLOGY, ELSEVIER, PHILADELPHIA, PA, vol. 127, no. 4, 9 July 2004 (2004-07-09), pages 1110-1122, XP005313031 ISSN: 0016-5085
- KIRIKOSHI H ET AL: "Up-regulation of Frizzled-7 (FZD7) in human gastric cancer" INTERNATIONAL JOURNAL OF ONCOLOGY, DEMETRIOS A. SPANDIDOS ED. & PUB, GR, vol. 19, no. 1, 1 July 2001 (2001-07-01), pages 111-115, XP008112314 ISSN: 1019-6439
- VINCAN E: "Frizzled/WNT signalling: the insidious promoter of tumour growth and progression" FRONTIERS IN BIOSCIENCE, FRONTIERS IN BIOSCIENCE, ALBERTSON, NY, US, vol. 9, 1 May 2004 (2004-05-01), pages 1023-1034, XP002398992 ISSN: 1093-9946
- LUSTIG B ET AL: "The Wnt pathway and its role in tumor development" JOURNAL OF CANCER RESEARCH AND CLINICAL ONCOLOGY, SPRINGER INTERNATIONAL, BERLIN, DE, vol. 129, 1 April 2003 (2003-04-01), pages 199-221, XP002983435 ISSN: 0171-5216
- KIM M ET AL: "Functional interaction between Wnt3 and Frizzled-7 leads to activation of the Wnt/beta-catenin signaling pathway in hepatocellular carcinoma cells" JOURNAL OF HEPATOLOGY, MUNKSGAARD INTERNATIONAL PUBLISHERS, COPENHAGEN, DK, vol. 48, no. 5, 1 May 2008 (2008-05-01), pages 780-791, XP022627181 ISSN: 0168-8278 [retrieved on 2008-02-07]
- BRANDA MARK ET AL: "Upregulation of Wnt and Frizzled genes are biomarkers for hepatocellular carcinoma" HEPATOLOGY, WILLIAMS AND WILKINS, BALTIMORE, MD, US, vol. 44, no. 4, Suppl. 1, 31 October 2006 (2006-10-31), page 514A, XP009123761 ISSN: 0270-9139
- KATOH, M.: 'Regulation of WNT3 and WNT3A mRNA in human cancer cell lines NT2, MCF-7, and MKN45' INTL J ONCOLOGY vol. 20, pages 373 - 377, XP008089315

## Description

### STATEMENT AS TO FEDERALLY SPONSORED RESEARCH

This invention was made with Government support under National Institutes of Health Grant Nos. CA035711 and AA002666. The Government has certain rights in this invention.

### CROSS-REFERENCE TO RELATED APPLICATIONS

This application is a continuation-in-part of International Application No. PCT/US05/000267, filed January 5, 2005, which claims the benefit of U.S. Provisional Application No. 60/612,098, filed September 21, 2004.

### TECHNICAL FIELD

This invention relates to detection and treatment of liver cancer.

### BACKGROUND

Hepatocellular carcinoma (HCC) is the major primary malignant tumor of the liver. Although viral etiological factors have been identified, the molecular mechanisms that contribute to tumor progression during hepatocarcinogenesis remain largely unknown. The Frizzled family of proteins is composed of ten or more seven-transmembrane proteins that act as receptors for Wnt proteins. The Wnt/Frizzled signaling network influences diverse biological processes ranging from cell fate determination to cell motility and proliferation.

β-catenin is a multifactorial protein with a role in cell-cell adhesion that involves strengthening the linkage of cadherin and α-catenin to the actin cytoskeleton. In the absence of Wnt/Frizzled signaling, β-catenin is phosphorylated by interactions with glycogen synthase kinase (GSK)-3β, and forms a complex with axin and the adenomatous polyposis coli protein (APC). Subsequently β-catenin is targeted for degradation by the ubiquitinproteasome system. In contrast, binding of a Wnt ligand to its Frizzled receptor stabilizes intracellular β-catenin through the inhibition of GSK-3β enzymatic activity. Subsequently, β-catenin translocates into the nucleus in association with high mobility group domain factors such as Tcf/Lef. This complex is associated with transcriptional up-regulation of growth regulatory and cell migration related genes.

Involvement of the Wnt/β-catenin signal transduction pathway in molecular pathogenesis of human hepatocellular carcinoma (HCC) has been described by Philippe Merle et al., (Gastroenterotogy 2004; 127:1110-1122) and overexpression of human Frizzled-7 receptor (FDZ7) in HCC was reported. However, molecular mechanisms that may lead to up-regulation of the FZD7 gene were unknown and specific receptor-ligand interactions of FZD7 were not discussed.

FZD7 was further described by Jennifer Willert et al., (BMC Developmental Biology 2002, 2:8) as a Wnt3A responsive gene in human embryonic carcinoma cells. Other Wnt proteins, such as Wnt3 or Wnt11, have not been analyzed in this study. Also, this disclosure is confined to human embryonic carcinoma cells, without mentioning or suggesting any role or expression of Wnt3A or other Wnt proteins in HCC or liver cells.

Finally, a requirement of Wnt11 activity for regulation of gastrulation movements in vertebrate has been described in WO 03/092705 A1 and Wnt11 was believed to signal through FZD7. Expression of Wnt11 in other cells, such as liver cells, or down-regulation of FZD7 through Wnt11 is not mentioned.

### SUMMARY

The present invention is based, in part, on the discovery that Wnt 3, 8b and 11 are ligands for Frizzled 7, which is commonly overexpressed at the mRNA and protein level in HCC, for example, in hepatitis B virus (HBV) related HCC. Liver cancer cells that overexpress Frizzled 7 exhibit enhanced cell motility and migration. Overexpression appears to be an early event during the multi-step process of hepatocyte transformation, and therefore Frizzled 7 and Wnt 3, 8b and 11 are novel molecular targets for therapy of liver cancer.

Accordingly, in one aspect, the invention provides a method for identifying an anti-liver cancer agent. The method includes selecting a test compound that binds to a polypeptide comprising the amino acid sequence of a Wnt 3 or Wnt 11 protein or a FZD-binding fragment thereof; and determining whether the test compound is capable of (i) reducing Wnt/FZD 7 signaling in a liver cell, (ii) reducing liver cancer cell motility, (iii) reducing β-catenin accumulation in a liver cancer cell; or (iv) treating liver cancer in vitro or in a non-human animal in vivo; wherein a test compound that is capable of at least one of (i) to (iv) is an anti-liver cancer agent. In some embodiments, selecting a test compound can include providing a polypeptide comprising the amino acid sequence of a Wnt 3 or Wnt 11 polypeptide or a FZD-binding fragment thereof, contacting the polypeptide with a test compound, detecting binding between the polypeptide and the test compound; and selecting the test compound if it binds to the polypepide. The polypeptide to which a test compound binds can be a (i) naturally occurring polypeptide, a (ii) recombinant polypeptide, (iii) a polypeptide expressed on the surface of a cell or (iv) an isolated polypeptide. Where the polypeptide includes the amino acid sequence of a Wnt 3 protein, the polypeptide can include any one of SBQ ID NO:8 to 12. Where the polypeptide includes the amino acid sequence of a Wnt 11 protein, the polypeptide can include any one of SEQ ID NO: 22 to 26. In certain embodiments, the polypeptide includes any one of SEQ ID NO:7 to 13 and 21 to 27 and at least one non-Wnt sequence. The test comp ound can be selected from the group consisting of polypeptides, ribonucleic acids, small molecules (e.g., small organic molecules), and deoxyribonucleic acids.

Anti-cancer agents identified by the methods of identifying an anti-liver cancer agent described herein include, but are not limited to, an anti-Wnt antibody, e.g., a monoclonal antibody, FZD7 receptors, Wnt-binding fragments of FZD7 receptors, and other Wnt-binding compounds. Anti-cancer agents identified by these methods can bet used in the treatment of cancer, e.g., liver cancer. Additionally, an anti-cancer agent identified by these methods can be used to manufacture a medicament for treating liver cancer or reducing the motility of liver cancer cells in a patient.

In another aspect, the invention includes a method of identifying a candidate anti-liver cancer agent. The method includes (a) providing a first polypeptide that: (i) comprises a FZD 7 polypeptide or a fragment thereof; and (ii) displays Wnt (e.g., Wnt 3 or 11)-binding ability; (b) providing a second polypeptide that: (i) comprises a Wnt 3 or Wnt 11 polypeptide or a fragment thereof; and (ii) displays FZD 7 binding ability; (c) contacting the first and second polypeptides in the presence of a test compound; and (d) comparing the level of binding between the first and second polypeptides in the presence of the test compound with the level of binding in the absence of the test compound, wherein a reduced level of binding in the presence of the test compound than in its absence indicates that the test compound is a candidate anti-cancer agent. The method can further include: (e) determining whether the candidate anti-liver cancer agent is capable of: (i) reducing Wnt/FZD 7 signaling in a cell that expresses FZD 7; (ii) reducing liver cancer cell motility; (iii) reducing β-catenin accumulation in a liver cancer cell; or (iv) treating liver cancer *in vitro* or in a non-human animal *in vivo*; wherein a candidate that is capable of at least one of (i) to (iv) is an anti-liver cancer agent. The test compound can be selected from the group consisting of polypeptides, ribonucleic acids, small molecules (e.g., small organic molecules), and deoxyribonucleic acids. The Wnt polypeptide can include, e.g., SEQ ID NO:8 to 12 and/or 22 to 26. The FZD polypeptide can include, e.g., SEQ ID NO:1, 2 and/or 3.

In certain embodiments, the first polypeptide is a first fusion protein comprising a FZD polypeptide (e.g., FZD 7 polypeptide) fused to (i) a transcription activation : domain of a transcription factor or (ii) a DNA-binding domain of a transcription factor; the second polypeptide is a second fusion protein comprising a Wnt polypeptide (e.g., a Wnt 3, or 11 polypeptide) fused to (i) a transcription activation domain of a transcription factor or (ii) a DNA-binding domain of a transcription factor, wherein the Wnt polypeptide is fused to a domain different from that fused to the Wnt polypeptide; and binding of the first and second polypeptides is detected as reconstitution of a transcription factor.

Anti-cancer agents (and/or candidate anticancer agents) identified by the methods described herein can be used in the treatment of cancer, for example, liver cancer. Additionally, anti-cancer agents (and/or candidate anticancer agents) identified by the methods described herein can be used in the manufacture of a medicament for treating cancer, for example, liver cancer.

In still another aspect, the invention provides a method of determining whether a liver cell is, or is at risk for becoming, a cancer cell. The method includes (a) providing a test liver cell (b) determining whether the cell's level of Wnt3 expression is higher than that of a control cell; and (c) classifying the test cell as (i) a cancer cell or (ii) at risk for becoming a cancer cell, if the test cell's level of Wnt3 expression is higher than that of the control cell. Where the method includes determining the cell's s level of Wnt3 expression, the method can further include: (c) determining whether the test cell's level of Wnt11 expression is lower than that of a control cell, wherein a lower level of expression of Wnt11 indicates that the test cell is, or is at risk for becoming, a cancer cell. Where the method includes determining the cell's level of Wnt11 expression, the method can further include: (c) determining whether the test cell's level of FZD7 expression is higher than that of a control cell, wherein a higher level of expression of FZD7 indicates that the test cell is, or is at risk for becoming, a cancer cell.

In a further aspect, the invention provides a method of determining whether a patient is suffering from or at risk for liver cancer, e.g., whether a test tissue sample comes from a patient that is suffering from or at risk for cancer. The method can include: providing a liver test tissue sample (such as tumerous or peritumorous liver tissue) obtained from a patient, and (b) determining whether the level of Wnt3 expression is higher, and optionally further whether the level of Wnt11 expression is lower, in the test tissue sample than that in a comparable tissue sample obtained from a healthy individual, wherein a higher level of expression of Wnt3 and/or a lower level of expression of Wnt11 in the test tissue sample is an indication that the sample is from a patient suffering from or at risk for cancer. Where the method includes determining the level of Wnt3 expression, the method can further include: (c) determining whether the level of Wnt11 expression in the test tissue sample is lower than that in a tissue sample obtained from a healthy individual, wherein a lower level of expression of Wnt11 is an indication that the sample comes from a patient who is suffering from or at risk for cancer. Where the method includes determining the level of Wnt11 expression, the method can further include: (c) determining whether the level of Wnt3 expression in the test tissue sample is higher than that in a tissue sample obtained from a healthy individual, wherein a higher level of expression of Wnt3 is an indication that the patient is suffering from or at risk for cancer.

In any of the methods described herein, determining the level of Wnt3 or Wnt11 expression can include determining the amount of Wnt3 or Wnt11 mRNA in the cell, e.g., using a Northern blot assay or an RT-PCR assay. In other embodiments, determining the level of expression can include determining the amount of Wnt3 or Wnt11 protein in the cell, e.g., using an anti-Wnt antibody, e.g., an antibody that binds to SEQ ID NOS:7 or 80.

In still another aspect, the invention includes an antibody or a composition for use in treating liver cancer in a patient. The use includes administering to the patient an effective amount of a compound that reduces Wnt/ FZD7 signaling in FZD7-expressing cells of the patient and that is optionally non-lethal to the FZD7-expressing cells. In one embodiment, the compound is a compound that reduces Wnt3 expression in the patient and/or increases Wnt11 expression in the patient. In another embodiment, the compound is a compound that binds Wnt 3 in the patient. The compound can be, e.g., an antisense oligonucleotide, a double stranded RNA (dsRNA) that includes a nucleotide sequence that hybridizes under physiological conditions to a Wnt nucleotide sequence, a genetic construct encoding a Wnt polypeptide (e.g., a Wnt11 polypeptide) and/or anti-Wnt antibody (e.g., anti-Wnt3 antibody). The compound can be administered by any route, e.g., by administration to the patient's liver. In certain embodiments, the compound is an antibody that binds to SEQ ID NO: 80. In other embodiments, the compound is a siRNA comprising SEQ ID NO:81, 82 or 83.

In yet another aspect, the invention includes a compound for use in treating liver cancer or for use in motility of a liver cancer cell in a patient. The method includes administering to the cell an effective amount of a compound capable of reducing Wnt/FZD7 signaling in the cell and which is optionally non-lethal to the cell. In one embodiment, the compound is a compound that reduces Wnt3 expression in the patient. In another embodiment, the compound is a compound that binds Wnt 3. The compound can be, e.g., an antisense oligonucleotide, a double stranded RNA (dsRNA) that includes a nucleotide sequence that hybridizes under physiological conditions to a Wnt nucleotide sequence, an siRNA that reduces expression of Wnt3 in the patient or an anti-Wnt3 antibody. The compound can be administered by any route, e.g., by administration to the patient's liver. In certain embodiments, the compound is an antibody that binds to SEQ ID NO: 80. In other embodiments, the compound is a siRNA comprising SEQ ID NO:81, 82 or 83.

Also described herein is the use of a compound that reduces Wnt/ FZD7 signaling in FZD7-expressing cells in the manufacture of (i) a medicament for the treatment of liver cancer or (ii) a medicament that reduces the motility of liver cancer cells. Optionally, the medicament is non-lethal to FZD7 expressing cells. In one embodiment, the compound is a compound that reduces FZD7 and/or Wnt3 expression in the patient and/or increases Wnt11 expression in the patient. The compound may be a compound that binds Wnt 3, Wnt 8b, Wnt 11, FZD7 or FZD8 in the patient The compound can be, e.g., an antisense oligonucleotide, a double stranded RNA (dsRNA) that includes a nucleotide sequence that hybridizes under physiological conditions to a Wnt nucleotide sequence, an isolated FZD7 receptor or a Wnt3 binding fragment thereof, a genetic construct encoding a Wnt polypeptide (e.g., a Wnt11 polypeptide) or truncated form of FZD7 (e.g., a form of FZD7 lacks FZD7's intracellular and/or transmembrane domain), and/or an anti-FZD and/or anti-Wnt antibody (e.g., anti-Wnt3 antibody).

In certain aspects, the invention includes an anti-Wnt3 antibody that binds to the amino acid sequence LRAKYSLFKPPTERDL (SEQ NO:80). The antibody can be included in a pharmaceutical composition suitable for administration to a patient.

Also described is the use of any of the compounds described herein in the preparation of a pharmaceutical composition for the treatment or prevention of a condition described herein, e.g., cancer, e.g., liver cancer. The composition can be used in treating cancer and/or for reducing motility of a cancer cell in accordance with the uses described herein. The composition can be in any form described herein, e.g., a liquid or solid composition. In certain embodiments, the compound is an antibody that binds to the amino acid sequence LRAKYSLFKPPTERDL (SEQ NO:80). In other embodiments, the compound is an siRNA, e.g., an siRNA comprising the nucleic acid sequence WNT3-1: 5'-GGAAAAAUGCCACUGCAUC-3' (SEQ NO:81), WNT3-2: 5'-GGAGUGUAUUCGCAUCUAC-3' (SEQ ID NO:82), and/or WNT3-3: 5'-GGCUUAUCUUUGCACAUGU-3' (SEQ NO:83)).

Also described herein are nucleic acids (e.g., a primer described in Table 1, below) that are useful for detecting Wnt proteins.

Unless otherwise defined, all technical and scientific terms used herein have the same meaning as commonly understood by one of ordinary skill in the art to which this invention belongs. Suitable methods and materials are described below, although methods and materials similar or equivalent to those described herein can be used in the practice or testing of the present invention. The materials, methods, and examples are illustrative only and not intended to be limiting.

Other features and advantages of the invention will be apparent from the following detailed description, and from the claims.

### DESCRIPTION OF DRAWINGS

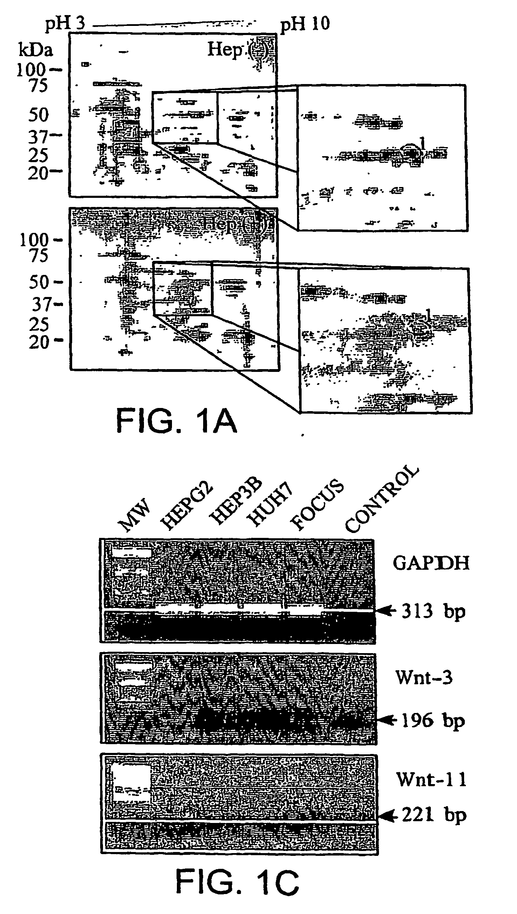

Fig. 1A is a set of pictures of silver-stained two-dimensional SDS-PAGE gels illustrating a pattern of fractionated heparan sulfate proteoglycans (HSPGs) from Huh7 cells following heparin treatment (Hep +) or no treatment (Hep -) of the Huh7 cells. The 0.25 M NaCl fractions from heparin affinity chromatography were separated onto first-dimensional pH 3-10 nonlinear IPG gels and second-dimensional 4-12% gradient NuPAGE gels. A protein spot (circled; hereinafter referred to as "spot 1") from the heparin-untreated fraction showed increased expression.

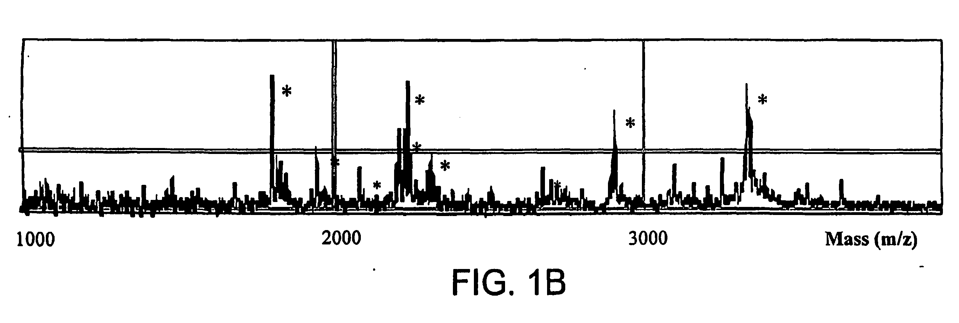

Fig. 1B is a mass-spectrum of tryptic peptides obtained from spot 1. Spot I was excised, destained, and digested with trypsin. The peptide masses were analyzed on a PBSII instrument. The marked peaks (*) represent peptides that matched the calculated masses of human Wnt-11 peptides.
Fig. 1C are pictures of agarose gels that illustrate the detection of Wnt ligand mRNA in various hepatocellular carcinoma cell lines using RT-PCR. Wnt-3 mRNA was detected in all HCC cell lines and Wnt-11 mRNA was detected in 3 HCC lines but not Focus cells. No other Wnt mRNAs were detectable by RT-PCR.

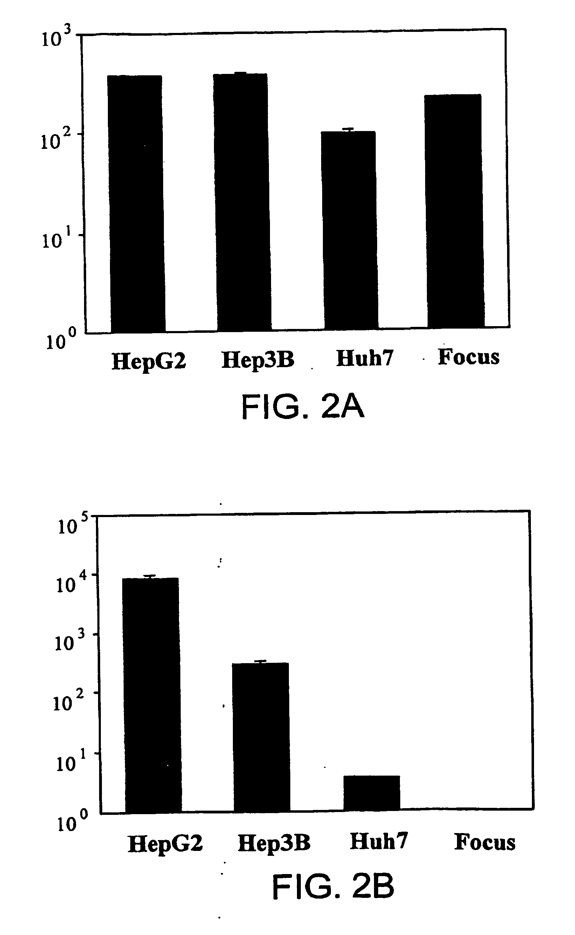

Fig. 2A is a bar graph illustrating Wnt3 mRNA levels as determined by qRT-PCR in HCC cell lines. 18SrRNA levels were used as internal controls, and Wnt3 and Wnt11 mRNA levels were expressed as copy numbers per 10⁹ 18SrRNA. The expression levels (mean ± SE) of Wnt3 mRNA in HCC cell lines were 370.0 ± 10.3 in HepG2, 381.3 ± 12.7 in Hep3B, 95.2 ± 6.3 in Huh7 and 210.4 ± 9.5 copies per 10⁹ 18SrRNA.
Fig. 2B is a bar graph illustrating Wnt11 MRNA levels as determined by qRT-PCR in HCC cell lines. 18SrRNA levels were used as internal controls, and Wnt11 mRNA levels were expressed as copy numbers per 10⁹ 18SrRNA. Wnt11 mRNA expression levels were 8,499.3 ± 845.0 in HepG2, 290.9 ± 40.1 in Hep3B and 3.57 ± 0.2 copies per 10⁹ 18SrRNA in Huh7 cells. Wnt11 MRNA could not be detected in focus cells with qRT-PCR, consistent with the result of conventional RT-PCR.

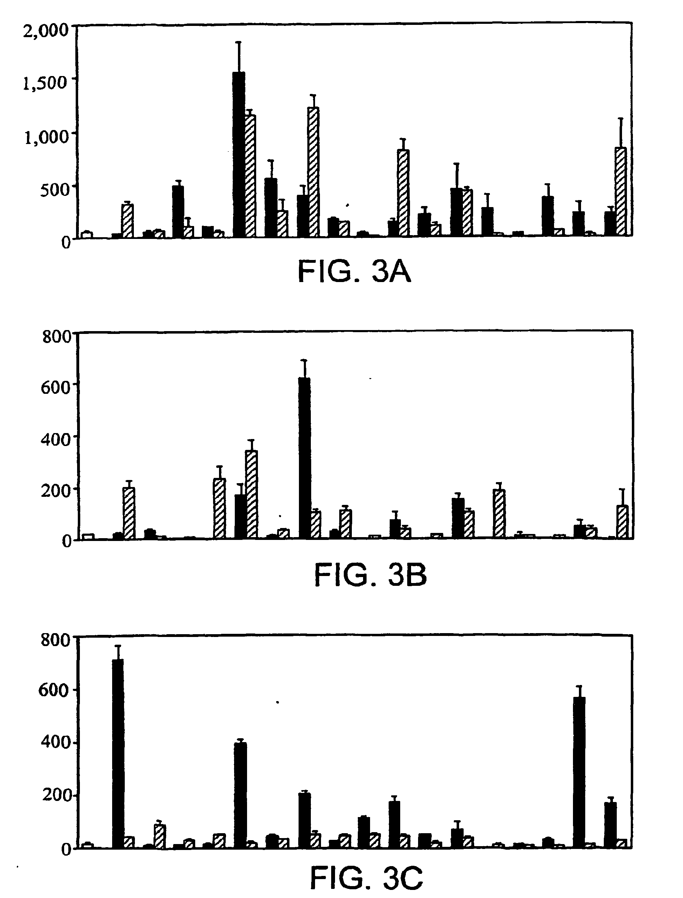

Fig. 3A is a bar graph illustrating expression of Wnt3 mRNA in human HCC tissues. The mRNA levels were measured by qRT-PCR. White bars represent mRNA levels in normal liver tissues. Black bars represent mRNA levels in HCC tissues. Gray bars represent mRNA levels in corresponding peritumoral tissues. Experiments were performed in duplicate and data are expressed as mean ± SD. Seventy-seven percent of HCCs and 59% of peritumoral tissues showed increased Wnt3 mRNA expression levels above the value of mean ± 3 SD in normal liver tissues. Seventy-one percent of HCC tissues.had increased Wnt3 mRNA expression levels compared to those in the corresponding peritumoral tissues.
Fig. 3B is a bar graph illustrating expression of Wnt11 MRNA in human HCC tissues. The mRNA levels were measured by qRT-PCR. White bars represent mRNA levels in normal liver tissues. Black bars represent mRNA levels in HCC tissues. Gray bars represent mRNA levels in corresponding peritumoral tissues. Experiments were performed in duplicate and data are expressed as mean ± SD. Forty-one percent of HCC tissues showed decreased expression even below the lower cut-off level of normal liver tissues, while none did in peritumoral tissues (P = .0036 by Fischer's exact test). Sixty-five percent of paired samples also showed decreased expression of Wnt11 mRNA in tumors compared with corresponding peritumoral tissues.
Fig. 3C is a bar graph illustrating expression of FZD7 mRNA in human HCC tissues. The mRNA levels were measured by qRT-PCR. White bars represent mRNA levels in normal liver tissues. Black bars represent mRNA levels in HCC tissues. Gray bars represent mRNA levels in corresponding peritumoral tissues. Experiments were performed in duplicate and data are expressed as mean ± SD. Fifty-nine percent of both HCC and peritumoral tissues showed increased FZD7 mRNA expressions compared to those in normal liver tissues. Seventy-one percent of paired samples showed increased expression of FZD7 mRNA in tumors compared with corresponding, peritumoral tissues (*P* = .031 by Wilcoxon signed-ranks test).

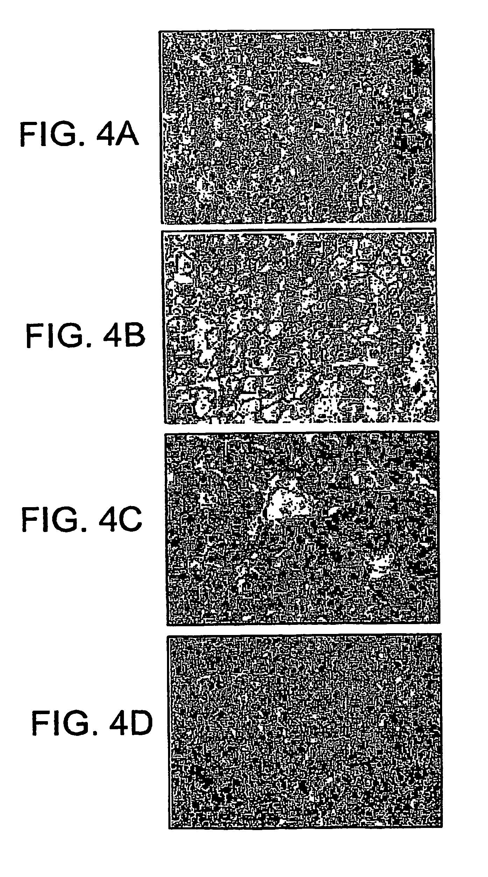

Figs 4A-4D are pictures of immunohistochemically stained human HCC and peritumoral tissues (magnification x400). Fig. 4A: Negative control. Fig. 4B: β-catenin stained peritumoral tissues. The hepatocytes showed typical membranous staining. Fig. 4C: β-catenin stained HCC tissues, which display nuclear accumulation of β-catenin. Note the nuclear staining as well as the increased cytoplasmic staining of β-catenin. Fig. 4D: HCC tissues, which display no nuclear or cytoplasmic accumulation of β-catenin.

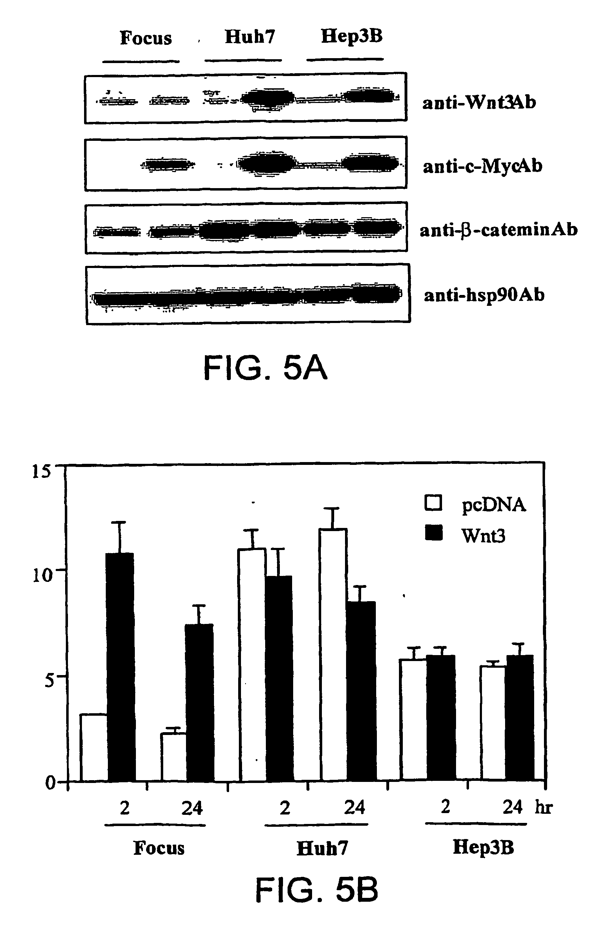

Fig. 5A is a composite picture of Western blots illustrating the effect of Wnt3 plasmid transfection on T-cell factor (Tcf) transcriptional activities in HCC cell lines. Focus, Huh7 and Hep3B cells were cotransfected with myc-tagged Wnt3 plasmid or pcDNA3.1/*myc*-His A plasmid, pSUPER8xTOPFLASH or pSUPER8xFOPFLASH, and β-galactosidase plasmid. Twenty-four hours after transfection, the cells were serum-starved for 24 h, and then stimulated with 1% FBS MEM. The cells were harvested at 2 h and 24 h after the stimulation and subjected to a luciferase assay and Western blot analysis for Wnt3 and β-catenin. Western blot analyses with rabbit polyclonal anti-Wnt3 antibody showed an increase of Wnt3 protein after transfection. Specificity of the polyclonal anti-Wnt3 antibody was verified with monoclonal anti-*myc* antibody. Note the increase of cellular β-catenin levels in Focus cells. Hsp90 protein was used as internal loading controls.
Fig. 5B is a bar graph illustrating changes in Tcf transcriptional activity in HCC cell lines after transfection with Wnt3 plasmid. The Tcf transcriptional activities were increased 3 fold in Focus cells following transfection, as compared to those in Focus cells transfected with control plasmid. Tcf transcriptional activities were slightly decreased in Huh7 cells and not changed in Hep3B cells following transfection. White bars denote cells transfected with the control plasmid (pcDNA) and black bars denote cells transfected with Wnt3 plasmid.

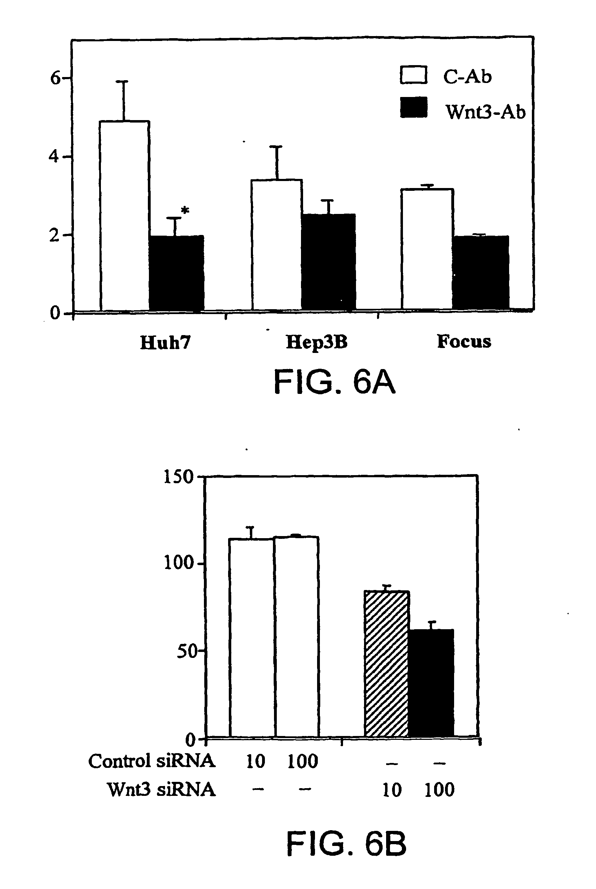

Fig. 6A is a bar graph illustrating the effects of anti-Wnt3 antibodies on Tcf transcriptional activities in HCC cell lines. HCC cells were seeded in 12-well plates and transfected with pSUPER8xTOPFLASH or pSUPER8xFOPFLASH with β-galactosidase plasmid. Cells were deprived of serum for 24 h and the cells were subsequently incubated with 1% FBS MEM containing either anti-Wnt3 antibodies (Wnt3-Ab; black bars) or control antibodies (10 µg/ml), and harvested 24 h after incubation. Normal rabbit IgG was used as a control antibody (C-Ab; white bars). Tcf transcriptional activities were decreased by 60% in Huh7, 26% in Hep3B, and 40% in Focus cells with polyclonal anti-Wnt3 antibody treatment.
Fig. 6B is a bar graph illustrating the effect of siRNAs on endogenous Wnt3 mRNA expression. Control siRNA and Wnt3 siRNA (WNT3-3) at a concentration of 10 or 100 nM were transfected into HCC cells. Forty-eight hours after transfection, cells were harvested and Wnt3 mRNA expression levels were measured using qRT-PCR siRNA Wnt3-3 caused a decrease of mRNA levels by 50-60% on average at a concentration of 100 nM in all of the 3 cell lines. Fig. 6B is representative of this effect in Huh7 cells.

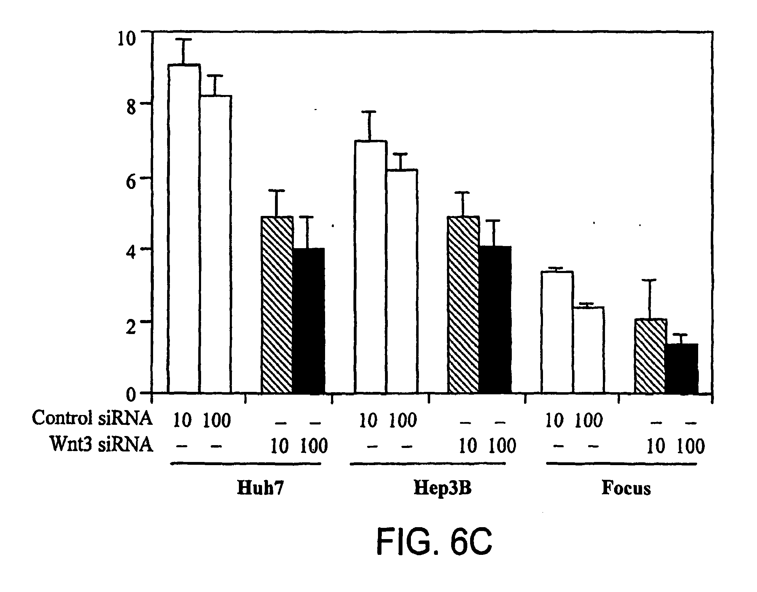

Fig. 6C is a bar graph illustrating the effects of Wnt3 siRNA on the Tcf transcriptional activities or HCC cell lines. Wnt3 siRNA or control siRNA was cotransfected in the presence of Tcf reporter at the indicated concentrations (nM). The Tcf transcriptional activities were decreased by 48.5% in Huh7, 33% in Hep3B, and 43.5% in Focus cells.

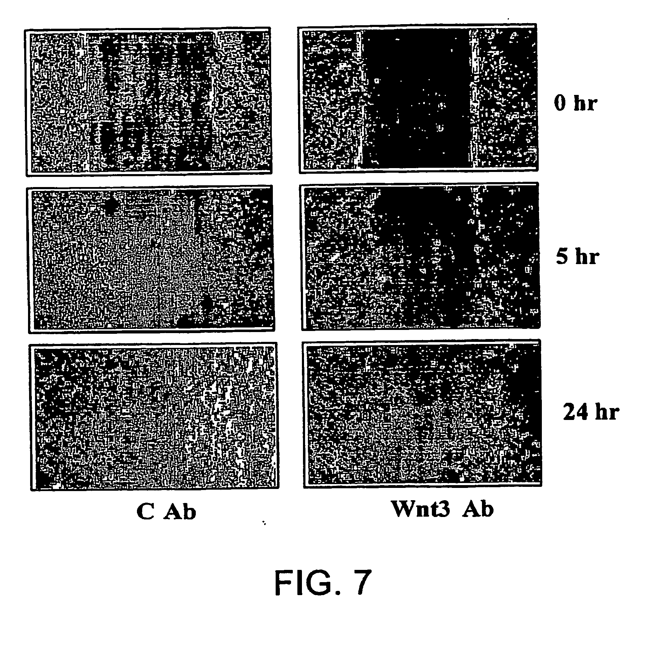

Fig. 7 are pictures of cell cultures illustrating delayed wound healing in Focus cells treated with anti-Wnt3 antibodies. Focus cells were plated in a 6-well plate. Confluent monolayer cells were wounded with sterile plastic 200 µl micropipette tips. The cells were then treated with media with either anti-Wnt3 Ab or rabbit IgG (control antibodies (C Ab); 10 µg/ml), and photographed at different time points. Focus cells treated with anti-Wnt3 Ab showed delayed wound healing. This effect was most prominent at 24 h. At 24 h, most of the wound was covered with migrating and/or proliferating cells in cells treated with C Ab, while it persisted in those treated with anti-Wnt3 Ab.

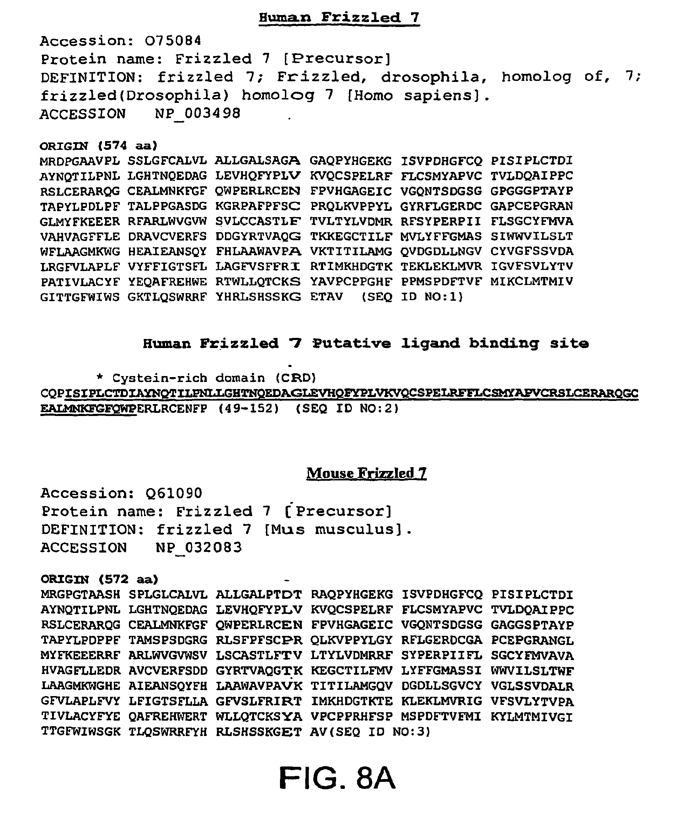

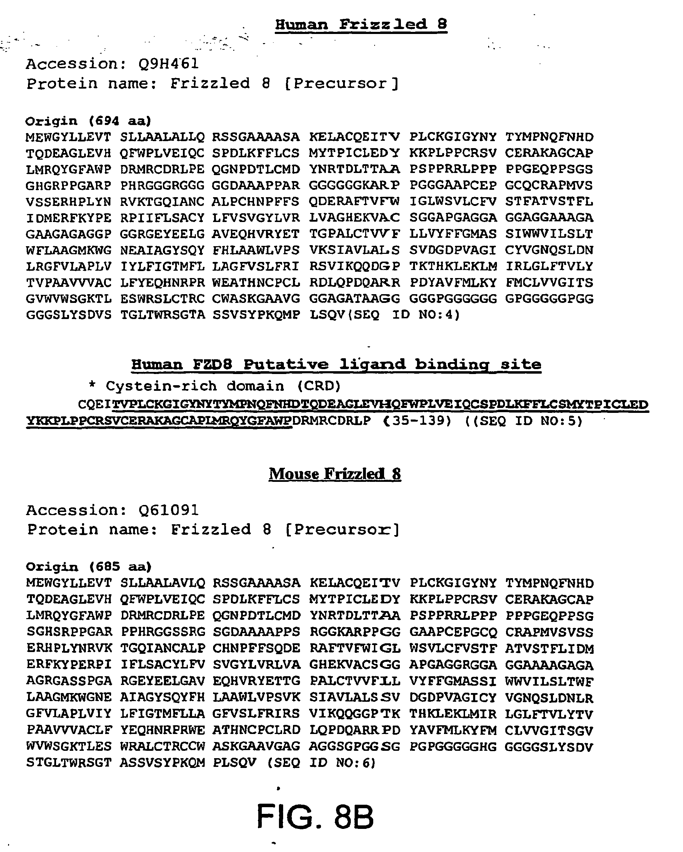

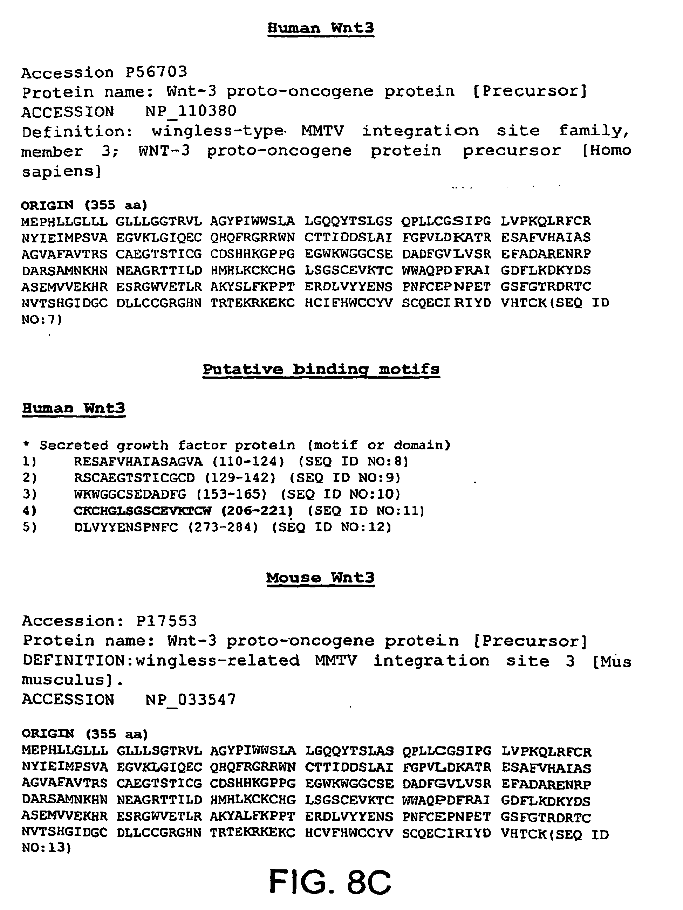

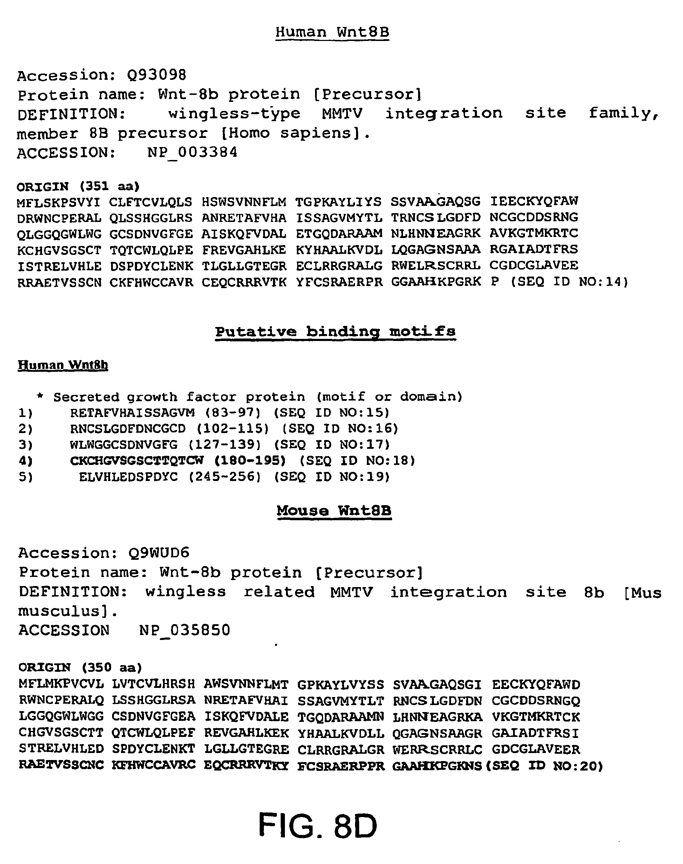

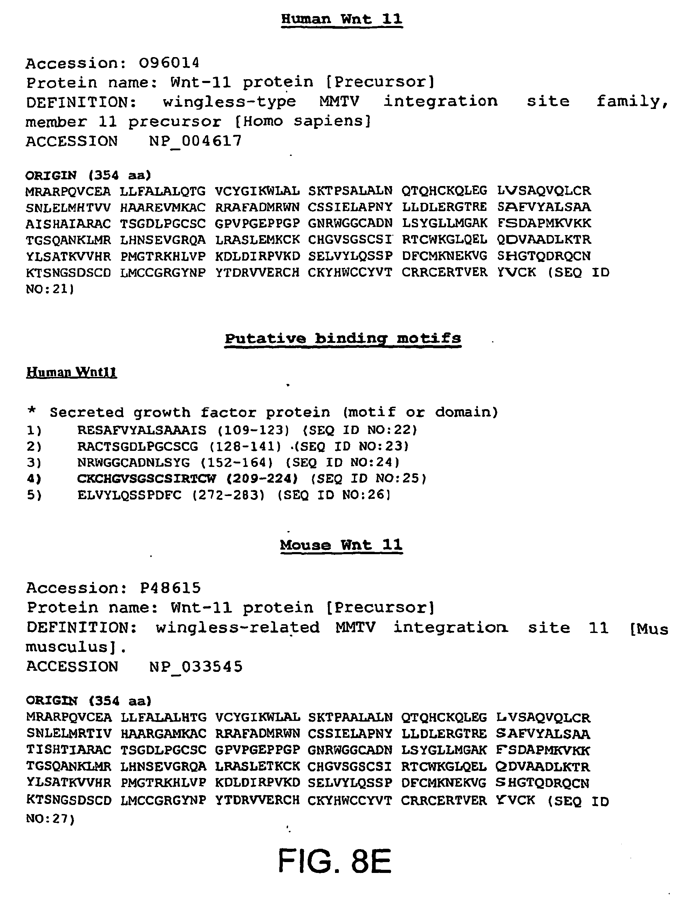

Figs. 8A-8E illustrate exemplary FZD7, FZD8, Wnt3, Wnt 8b and Wnt 11 human and mouse amino acid sequences, including putative binding motifs.

### DETAILED DESCRIPTION

This invention is based, at least in part, on the discovery that particular Frizzled (FZD) proteins, e.g., FZD 7 and 8, are associated with certain cancers, such as liver cancer, and that Wnt 3, 8b and 11 are FZD ligands. Accordingly, the present specification provides, *inter alia,* methods of using Wnt and FZD proteins, genes, FZD-specific antibodies and probes in diagnosis and treatment of cancer and for screening test compounds for an ability to treat cancer. Also disclosed are compounds useful for treating cancer such as liver cancer.

### I. Nucleic Acids, Proteins, Vectors, and Host Cells

The terms "Frizzled," "FZD," "Frizzled protein" and "Frizzled receptor" refer to a family of mammalian proteins related to the Drosophila Frizzled genes, which play a role in the development of tissue polarity. The Frizzled family comprises at least 10 mammalian genes. Exemplary human Frizzled receptors include Frizzled 1, Frizzled 2, Frizzled 3, Frizzled 4, Frizzled 5, Frizzled 6, Frizzled 7, Frizzled 8, Frizzled 9 and Frizzled 10. Frizzled receptors are involved in a dynamic model of transmembrane signal transduction analogous to G-protein-coupled receptors with amino-terminal ligand binding domains.

The terms "Wnt protein," "Wnt ligand" and "Wnt" refer to a family of mammalian proteins related to the Drosophila segment polarity gene, wingless. In humans, the Wnt family of genes typically encode 38 to 43 kDa cysteine rich glycoproteins having hydrophobic signal sequence and a conserved asparagine-linked oligosaccharide consensus sequence (see e.g., Shimizu et al., Cell Growth Differ 8:1349-1358 (1997)). The Wnt family contains at least 19 mammalian members. Exemplary Wnt proteins include Wnt-1, Wnt-2, Wnt-2b (also known as Wnt-13), Wnt-3, Wnt-3A, Wnt-4, Wnt-5A, Wnt-5B, Wnt-6, Wnt-7A, Wnt-7B, Wnt-8A, Wnt-8B, Wnt-10A, Wnt-10B, Wnt-11, Wnt 14, Wnt 15, and Wnt 16.

In addition to Wnt ligands, a family of secreted Frizzled-related proteins (sFRPs) has been isolated. sFRPs appear to function as soluble endogenous modulators of Wnt signaling by competing with the membrane-spanning Frizzled receptors for the binding of secreted Wnt ligands. sFRPs can either antagonize Wnt function by binding the protein and blocking access to its cell surface signaling receptor, or they can enhance Wnt activity by facilitating the presentation of ligand to the Frizzled receptors.

The term "Wnt/FZD signaling pathway" refers to an intracellular signal transduction pathway that is initiated by an interaction between a Frizzled receptor, e.g., FZD 7, and one or more of its ligands, e.g., a Wnt protein, e.g., Wnt 3, 8b or 11. Typically, a Wnt/FZD interaction involves binding of a Wnt protein, e.g., Wnt 3, 8b or 11, to a Frizzled receptor, e.g., FZD 7, leading to activation of a signal transduction pathway. In some instances, activation of the Wnt/Frizzled signaling pathway will lead to induction of downstream-Wnt and/or FZD-inducible genes. A "downstream Wnt/FZD regulated gene product" is a protein or RNA that is regulated (e.g., up- or down-regulated) as a result of signaling by a Wnt/FZD signaling pathway.

The invention may include the use of certain FZD and Wnt nucleic acids. For example, the present invention may include the use of certain FZD 7 nucleic acids, such as those that encode the amino acid sequences of the exemplary human and mouse FZD 7 (SEQ ID NOs:1 and 3, respectively) receptors set forth in Fig. 8A. As another example, the invention includes the use of certain Wnt 3, and 11 nucleic acids, such as those that encode the amino acid sequences of the exemplary human and mouse Wnt 3 (SEQ ID NOs:7 and 13, respectively), and 11 (SEQ ID NOs:21 and 27, respectively) proteins set forth in Figs. 8A to 8E.

Also described and/or claimed is the use of certain fragments of FZD and Wnt nucleic acids, e.g., a fragment of a nucleic acid sequence that encodes SEQ ID NOs:1, 3, 4, 6, 7, 13, 14, 20, 21, or 27. Fragments of FZD or Wnt nucleic acids encode at least one useful fragment of a FZD or Wnt polypeptide (e.g., a human or rodent polypeptide), respectively, such as a binding domain (e.g., a CRD domain) or other useful fragment. For example, a useful fragment of a FZD nucleic acid may encode a fragment of a FZD receptor having binding activity, e.g., a fragment corresponding to SEQ ID NO:3 or 5. As another example, a useful fragment of an Wnt nucleic acid may encode a fragment of a Wnt polypeptide having binding activity, e.g., a fragment corresponding to any one or more of SEQ ID NOs:8 to 12, 15 to 19 and 22 to 26.

FZD and Wnt nucleic acids described herein include both RNA and DNA, including genomic DNA and synthetic (e.g., chemically synthesized) DNA. Nucleic acids can be double-stranded or single-stranded. Where single-stranded, the nucleic acid can be a sense strand or an antisense strand. Nucleic acids can be synthesized using oligonucleotide analogs or derivatives (e.g., inosine or phosphorothioate nucleotides). Such oligonucleotides can be used, for example, to prepare nucleic acids that have altered base-pairing abilities or increased resistance to nucleases.

An "isolated nucleic acid" is a nucleic acid the structure of which is not identical to that of any naturally occurring nucleic acid or to that of any spanning more than three separate genes. The term therefore covers, for example, (a) a DNA which has the sequence of part of a naturally occurring genomic DNA molecule but is not flanked by both of the coding sequences that flank that part of the molecule in the genome of the organism in which it naturally occurs; (b) a nucleic acid incorporated into a vector or into the genomic DNA of a prokaryote or eukaryote in a manner such that the resulting molecule is not identical to any naturally occurring vector or genomic DNA; (c) a separate molecule such as a cDNA, a genomic fragment, a fragment produced by polymerase chain reaction (PCR), or a restriction fragment; and (d) a recombinant nucleotide sequence that is part of a hybrid gene, i.e., a gene encoding a fusion protein. Specifically excluded from this definition are nucleic acids present in mixtures of (i) DNA molecules, (ii) transfected cells; and (iii) cell clones, e.g., as these occur in a DNA library such as a cDNA or genomic DNA library.

In some embodiments, the invention includes the use of nucleic acid sequences that are substantially homologous to a FZD or Wnt nucleic acid. A nucleic acid sequence that is "substantially homologous" to a FZD or Wnt nucleic acid is at least 75% homologous to FZD or Wnt nucleic acid sequences that encode any one of SEQ ID NOs:1 to 27. For example, substantially homologous nucleic acid sequences can be at least about 80%, 85%, 90%, 95%, 98%, or at least about 99% homologous to sequences that encode SEQ ID NOs:1 to 27. For purposes of comparison of nucleic acids, the length of the reference nucleic acid sequence will be at least 50 nucleotides, but can be longer, e.g., at least 60 nucleotides, or more nucleotides.

As used herein, "percent homology" of two amino acid sequences or two nucleic acid sequences is determined using the algorithm of Karlin and Altschul (1990) Proc. Nat'l Acad. Sci. USA 87:2264-2268, modified as in Karlin and Altschul (1993) Proc. Nat'l Acad. Sci. USA 90:5873-5877. Such an algorithm is incorporated into the NBLAST and XBLAST programs of Altschul, et al. (1990); J. Mol. Biol. 215:403-410. BLAST nucleotide searches are performed with the NBLAST program, score = 100, wordlength = 12 to obtain nucleotide sequences homologous to FZD or Wnt nucleic acid molecules used in the invention. BLAST protein searches are performed with the XBLAST program, score = 50, wordlength = 3 to obtain amino acid sequences homologous to a reference polypeptide. To obtain gapped alignments for comparison purposes, Gapped BLAST is utilized as described in Altschul et al., (1997) Nucleic Acids Res. 25:3389-3402. When utilizing BLAST and Gapped BLAST programs, the default parameters of the respective programs (e.g., XBLAST and NBLAST) are used. See the World Wide Web at address ncbi.nlm.nih.gov.

The invention also includes the use of nucleic acids that hybridize under stringent hybridization conditions (as defined herein) to all or a portion of nucleotide sequences that encode any of SEQ ID NOs:1 to 27, or to a complement of such nucleic acid sequences. The hybridizing portion of the hybridizing nucleic acids is typically at least 15 (e.g., 20, 25, 30, or 50) nucleotides in length. The hybridizing portion of the hybridizing nucleic acid is at least about 75% (e.g., at least 80%, 90%, 95% or 98%) identical to the sequence of a portion or all of a nucleic acid encoding an FZD or Wnt polypeptide, or to its complement. Hybridizing nucleic acids of the type described herein can be used, for example, as a cloning probe, a primer (e.g., a PCR primer), or a diagnostic probe. Hybridization of the oligonucleotide probe to a nucleic acid sample typically is performed under stringent conditions. Nucliec acid duplex or hybrid stability is expressed as the melting temperature or Tm, which is the temperature at which a probe dissociates from a target DNA. This melting temperature is used to define the required stringency conditions. IF sequenes are to be identified that are related and substantially identical to the probe, rather than identical, then it is useful to first estabilish the lowest termperature at which only homologous hybridization occurs with a particular concentration of salt (e.g., SSC or SSPE).

Then, assuming that 1% mismatching results in a 1°C decrease in the Tm, the temperature of the final wash in the hybridization reaction is reduced accordingly (for example, if sequences having > 95% identity with the probe are sought, the final wash temperature is decreased by 5°C). In practice, the change in Tm can be between 0.5°C and 1.5°C per 1% mismatch. Stringent conditions involve hybridizing at 68°C in 5x SSC/5x Denhardt's solution/1.0% SDS, and washing in 0.2x SSC/0.1 % SDS at room temperature. Moderately stringent conditions include washing in 3x SSC at 42°C. The parameters of salt concentration and temperature can be varied to achieve the optimal level of identity between the probe and the target nucleic acid. Additional guidance regarding such conditions is readily available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

Nucleic acids that hybridize to nucleotide sequence that encode any of SEQ ID NOs:1 to 27 are considered "antisense oligonucleotides."

Also included in the invention are genetic constructs (e.g., vectors and plasmids) that include a FZD and/or Wnt nucleic acid described herein, operably linked to a transcription and/or translation sequence to enable expression, e.g., expression vectors. A selected nucleic acid, e.g., a DNA molecule encoding a FZD or Wnt polypeptide, is "operably linked" to another nucleic acid molecule, e.g., a promoter, when it is positioned in such a way that the other molecule can direct transcription and/or translation of the selected nucleic acid. For example, the selected nucleic acid can be positioned adjacent to the other nucleic acid molecule.

Also included in the invention are various engineered cells which contain a FZD and/or Wnt nucleic acid described herein. For example, the invention includes transformed host cells, i.e., cells into which (or into an ancestor of which) has been introduced, by means of recombinant DNA techniques, a nucleic acid encoding a FZD and/or Wnt polypeptide. Both prokaryotic and eukaryotic cells are included, e.g., mammalian cells (e.g., liver cells), fungi, and bacteria (such as *Escherichia coli*), and the like. An engineered cell exemplary of the type included in the invention is a liver cell that overexpresses a FZD 7 transgene.

A cell that "overexpresses FZD" is a cancer cell and/or transgenic cell in which expression of a particular FZD protein, such as FZD 7 and/or 8, is at least about 1.5 times, e.g., at least about 2, 3, 4 or 5 times, the level of expression in a non-cancer cell or non-transgenic cell, respectively, from the same tissue type. In some embodiments, FZD expression in a cell can be compared to expression in a non-cancer or non-transgenic cell of a different tissue-type or a panel of non-cancer or non-transgenic cells of a different tissue type. In addition, expression of one type of FZD protein (e.g., FZD 7) can be compared to other FZD proteins in the same cell. Methods for determining the level of expression of a particular gene are well known in the art. Such methods include, but are not limited to, RT-PCR, real time PCR and use of antibodies against the gene products.

The use of certain FZD and Wnt polypeptides are also included within the present invention. Examples of FZD polypeptides used in the present invention are human and mouse FZD polypeptides, such as those shown in SEQ ID NOs:1 and 3, respectively. Examples of Wnt polypeptides used in the present invention are human and mouse Wnt 3 and 11 polypeptides, such as those shown in SEQ ID NOs:7, 13 21 and 27. Also included used in the present invention are certain fragments of FZD and Wnt polypeptides, e.g., fragments of SEQ ID NOs:1, 3, 7, 13, 21 and 27. Fragments of FZD and Wnt polypeptides may include at least one binding domain, or other useful portion of a full-length FZD and Wnt polypeptide. For example, useful fragments of FZD and Wnt polypeptides include, but are not limited to, fragments having binding activity (e.g., SEQ ID NOs: 2, 5, 8 to 12, and 22 to 26).

The terms "protein" and "polypeptide" both refer to any chain of amino acids, regardless of length or post-translational modification (e.g., glycosylation or phosphorylation). Thus, the terms "Frizzled protein," "Wnt protein," "Frizzled polypeptide," and "Wnt polypeptide" include full-length naturally occurring isolated proteins, as well as recombinantly or synthetically produced polypeptides that correspond to the full-length naturally occurring proteins, or to a fragment of the full-length naturally occurring or synthetic polypeptide.

As discussed above, the terms "Frizzled polypeptide," and "Wnt polypeptide" include biologically active fragments of naturally occurring or synthetic FZD and Wnt polypeptides, respectively. Fragments of a protein can be produced by any of a variety of methods known to those skilled in the art, e.g., recombinantly, by proteolytic digestion, or by chemical synthesis. Internal or terminal fragments of a polypeptide can be generated by removing one or more nucleotides from one end (for a terminal fragment) or both ends (for an internal fragment) of a nucleic acid that encodes the polypeptide. Expression of such mutagenized DNA can produce polypeptide fragments. Digestion with "end-nibbling" endonucleases can generate DNAs that encode an array of fragments. DNAs that encode fragments of a protein can also be generated, e.g., by random shearing, restriction digestion, chemical synthesis of oligonucleotides, amplification of DNA using the polymerase chain reaction, or a combination of the above-discussed methods. Fragments can also be chemically synthesized using techniques known in the art, e.g., conventional Merrifield solid phase FMOC or t-Boc chemistry.

A purified or isolated compound is a composition that is at least 60% by weight the compound of interest, e.g., a FZD polypeptide, Wnt polypeptide, or antibody. For example, the preparation can be at least 75% (e.g., at least 90%, 95%, or even 99%) by weight the compound of interest. Purity can be measured by any appropriate method known in the art, e.g., column chromatography, polyacrylamide gel electrophoresis, or HPLC analysis.

In certain embodiments, FZD and Wnt polypeptides include sequences substantially identical to all or portions of a naturally occurring FZD and Wnt polypeptides. Polypeptides "substantially homologous" to the FZD and Wnt polypeptide sequences described herein have an amino acid sequence that is at least 65% (e.g., at least 75%, 80%, 85%, 90%, 95% or 99%, e.g., 100%), homologous to an amino acid sequence represented by SEQ ID NOs:1 to 27 (measured as described herein). For purposes of comparison, the length of the reference FZD and Wnt polypeptide sequence can be at least 16 amino acids, e.g., at least 20 or 25 amino acids.

A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

The invention also includes the use of fusion proteins (and nucleic acids that encode such fusion proteins) in which a portion of a FZD (e.g., FZD 7) or Wnt (e.g., Wnt 3, and/or 11) polypeptide is fused to an unrelated polypeptide (e.g., a marker polypeptide or a fusion partner) to create a fusion protein. For example, the polypeptide can be fused to a hexa-histidine tag or a FLAG tag to facilitate purification of bacterially expressed polypeptides or to a hemagglutinin tag or a FLAG tag to facilitate purification of polypeptides expressed in eukaryotic cells. The invention also includes, for example, the use of isolated polypeptides (and the nucleic acids that encode these polypeptides) that include a first portion and a second portion, wherein the first portion includes, e.g., a FZD or Wnt polypeptide, and the second portion includes an unrelated polypeptide, e.g., an immunoglobulin constant (Fc) region or a detectable marker.

The fusion partner can be, for example, a polypeptide that facilitates secretion, e.g., a secretory sequence. Such a fused polypeptide is typically referred to as a preprotein. The secretory sequence can be cleaved by the host cell to form the mature protein. Also within the invention are nucleic acids that encode a FZD and/or Wnt polypeptide fused to a polypeptide sequence to produce an inactive preprotein. Preproteins can be converted into the active form of the protein by removal of the inactivating sequence.

### II. Methods for Detecting Cancer

Without being bound by theory, it appears that various FZD proteins, e.g., FZD 7 and 8, and FZD ligands, e.g., Wnt3 and 11, are important in cancer, e.g., liver cancer. In particular, hepatocytes appear to overexpress FZD 7 and Wnt3 early during the process of transformation, e.g., prior to the development of HCC. Similarly, such cells often underexpress FZD 8 and/or Wnt 11. It appears that Wnt 3, 8b and 11 are FZD 7 ligands.

Accordingly, the present invention provides methods of detecting cancer cells, facilitating the diagnosis of the presence and severity (e.g., tumor grade, tumor burden, and the like) of cancer in a patient, facilitating a determination of the prognosis of a patient and assessing the responsiveness of the patient to therapy (e.g., by providing a measure of therapeutic effect through, for example, assessing tumor burden during or following a chemotherapeutic regimen).

Detection can be based on detection of a polynucleotide (e.g., a Wnt 3 and/or Wnt 11 polynucleotide) that is differentially expressed in a cancer cell (e.g., as compared to a non-cancer cell) and/or detection of a polypeptide (e.g., a Wnt 3 and/or Wnt 11 polypeptide) encoded by a polynucleotide that is differentially expressed in a cancer cell. The detection methods of the invention can be conducted *in vitro* or *in vivo,* on a biological sample, e.g., isolated cells and/or whole tissues.

A "biological sample" as used herein is a sample of biological tissue or fluid that contains nucleic acids or polypeptides, e.g., a FZD 7 protein, polynucleotide or transcript. Such samples include, but are not limited to, tissue obtained from, e.g., liver, lung, lymph nodes, colon, stomach, pancreas, bile duct, small bowel and/or esophagus. Biological samples may also include sections of tissues such as biopsy and autopsy samples, frozen sections taken for histologic purposes, blood, plasma, serum, sputum, stool, tears, mucus, bile, saliva, lymph, hair, skin, etc. Biological samples also include explants and primary and/or transformed cell cultures derived from patient tissues. A biological sample is typically obtained from a eukaryotic organism, e.g., a primate such as a chimpanzee or human; cow; horse; goat; sheep; dog; cat; a rodent, e.g., guinea pig, rat or mouse; rabbit; bird; reptile; or fish. A sample is usually provided by removing a sample of cells from an animal, but can also be accomplished by providing previously isolated cells (e.g., isolated by another person, at another time and/or for another purpose), or by performing the methods of the invention *in vivo.* Archival tissues, having treatment or outcome history, can be used.

In some embodiments, methods are provided for detecting a cancer cell by detecting expression in the cell of a transcript (e.g., a Wnt3 and/or Wnt11 transcript) that is differentially expressed in a cancer cell. Any of a variety of known methods can be used for detection including but not limited to, detection of a transcript by hybridization of mRNA with an appropriate hybridization probe; detection of a transcript by a polymerase chain reaction using specific oligonucleotide primers; and *in situ* hybridization using an appropriate hybridization probe. The methods can be used to detect and/or measure mRNA levels of a gene that is differentially expressed in a cancer cell. In some embodiments, the methods comprise: a) contacting a sample with a polynucleotide that corresponds to a differentially expressed gene described herein under conditions that allow hybridization; and b) detecting hybridization, if any.

Detection of differential hybridization, when compared to a suitable control, is an indication of the presence in the sample of a polynucleotide that is differentially expressed in a cancer cell. Appropriate controls include, for example, a sample that is not a cancer cell, a sample that is known not to contain a polynucleotide that is differentially expressed in a cancer cell, and use of a labeled polynucleotide of the same "sense" as the polynucleotide that is differentially expressed in the cancer cell. Conditions that allow hybridization are known in the art and have been described in more detail above. Detection can also be accomplished by any known method, including, but not limited to, *in situ* hybridization, PCR (polymerase chain reaction) and/or RT-PCR (reverse transcription-PCR), or combinations of known techniques. A variety of labels and labeling methods for polynucleotides are known in the art and can be used in the assay methods of the invention. Specificity of hybridization can be determined by comparison to appropriate controls.

Polynucleotides generally comprising at least 10 nt, at least 12 nt or at least 15 contiguous nucleotides of a polynucleotide described herein, such as those having the sequence as depicted herein, can be used for a variety of purposes, such as probes or PCR primers for detection and/or measurement of transcription levels of a polynucleotide that is differentially expressed in a cancer cell. As will be appreciated by the skilled artisan, the probe can be detectably labeled and contacted with, for example, an array comprising immobilized polynucleotides obtained from a test sample (e.g., mRNA). Alternatively, the probe can be immobilized on an array and the test sample detectably labeled. The use of these and other variations of the methods of the invention are well within the skill in the art.

Nucleotide probes can be used to detect expression of a gene corresponding to the provided polynucleotide. In Northern blots, mRNA is separated electrophoretically and contacted with a probe. A probe is detected as hybridizing to an mRNA species of a particular size. The amount of hybridization can be quantified to determine relative amounts of expression. Probes can be used for *in situ* hybridization to cells to detect expression. Probes can also be used *in vivo* for diagnostic detection of hybridizing sequences. Probes can be labeled with a radioactive isotope or other types of detectable labels, e.g., chromophores, fluorophores and/or enzymes. Other examples of nucleotide hybridization assays are described in WO92/02526 and U.S. Pat. No. 5,124,246.

PCR is another means for detecting small amounts of target nucleic acids (see, e.g., Mullis etal., Meth. Enzymol. (1987) 155:335; U.S. Pat. No. 4,683,195; and U.S. Pat. No. 4,683,202). Two primer oligonucleotides that hybridize with the target nucleic acids can be used to prime the reaction. The primers can be composed of sequence within or 3' and 5' to the polynucleotides described herein. After amplification of the target by standard PCR methods, the amplified target nucleic acids can be detected by methods known in the art, e.g., Southern blot. mRNA or cDNA can also be detected by traditional blotting techniques (e.g., Southern blot, Northern blot, etc.) described in Sambrook et al., "Molecular Cloning: A Laboratory Manual" (New York, Cold Spring Harbor Laboratory, 1989) (e.g., without PCR amplification). In general, mRNA or cDNA generated from mRNA using a polymerase enzyme can be purified and separated using gel electrophoresis, and transferred to a solid support, such as nitrocellulose. The solid support can be exposed to a labeled probe and washed to remove any unhybridized probe. Duplexes containing the labeled probe can then be detected.

Methods using PCR amplification can be performed on the DNA from one or more cells. The use of the polymerase chain reaction is described in Saiki et al. (1985) Science 239:487, and a review of techniques may be found in Sambrook et al., "Molecular Cloning: A Laboratory Manual" (New York, Cold Spring Harbor Laboratory, 1989; pp.14.2-14.33). A detectable label may be included in the amplification reaction. Suitable detectable labels include fluorochromes, (e.g. fluorescein isothiocyanate (FITC), rhodamine, Texas Red, phycoerythrin, allophycocyanin, 6-carboxyfluorescein (6-FAM), 2',7'-dimethoxy-4',5'-dichloro-6-carboxyfluorescein, 6-carboxy-X-rhodamine (ROX), 6-carboxy- 2',4,4',5',7,7'-hexachlorofluorescein (HEX), 5-carboxyfluorescein (5-FAM) or N,N,N',N'-tetramethyl-6-carboxyrho- damine (TAMRA)), radioactive labels, (e.g., ³²P, ³⁵S, ³H, etc.), and the like. The label may be a two stage system, where the polynucleotide is conjugated to biotin, haptens, etc. having a high affinity binding partner, e.g. avidin, specific antibodies, etc., where the binding partner is conjugated to a detectable label. The label may be conjugated to one or both of the primers. Alternatively, the pool of nucleotides used in the amplification is labeled, so as to incorporate the label into the amplification product.

In one embodiment, expression level is assessed by using real time PCR. RNA is isolated from a sample of interest PCR primers are designed to amplify the specific gene of interest. PCR product accumulation is measured using a dual-labeled flourogenic oligonucleotide probe. The probe is labeled with two different flourescent dyes, the 5' terminus reporter dye and the 3' terminus quenching dye. The oligonucleotide probe is selected to be homologous to an internal target sequence present in the PCR amplicon. When the probe is intact, energy transfer occurs between the two flourophors, and the fluorescent emission is quenched. During the extension phase of PCR, the probe is cleaved by 5' nuclease activity of Taq polymerase. Therefore, the reporter is no longer in proximity to the quencher, and the increase in emission intensity is measured. An exemplary method for detecting FZD expression using real time PCR is provided in the Examples section, below. The primers can also be used in other methods, for example RT-PCR. This assay provides a quantitative measure of nucleic acid.

In other embodiments, methods are provided for detecting a cancer cell by detecting expression of a protein (e.g., a Wnt3 and/or Wnt11 protein) that is differentially expressed by the cell. Any of a variety of known methods can be used for detection, including but not limited to methods that employ binding compounds, e.g., antibodies or antigen binding fragments thereof, e.g., as is useful in ELISA and/or Western blotting methods. Such antibodies can be polyclonal or monoclonal and can be labeled with a detectable marker (e.g., fluorophore, chromophore or isotope, etc). Where appropriate, the compound can be attached to a solid support such as a bead, plate, filter, resin, etc. Determination of formation of the compound/target complex can be effected by contacting the complex with a further compound (e.g., a secondary antibody) that specifically binds to the first compound (or complex). Like the first compound, the further compound can be attached to a solid support and/or can be labeled with a detectable marker.

The materials needed to perform the detection methods described herein can be provided as part of a kit Thus, the invention further provides kits for detecting the presence and/or a level of a polynucleotide that is differentially expressed in a cancer cell (e.g., by detection of an mRNA encoded by the differentially expressed gene of interest), and/or a polypeptide encoded thereby, in a biological sample. Procedures using these kits can be performed by clinical laboratories, experimental laboratories, medical practitioners or private individuals. The kits of the invention for detecting a polypeptide encoded by a polynucleotide that is differentially expressed in a cancer cell may comprise a moiety, such as an antibody, that specifically binds the polypeptide. The kits of the invention used for detecting a polynucleotide that is differentially expressed in a cancer cell may comprise a moiety that specifically hybridizes to such a polynucleotide. The kit may optionally provide additional components that are useful in the procedure including, e.g., buffers, developing reagents, labels, reacting surfaces, means for detection, control samples, standards, instructions, and interpretive information.

The present invention further relates to methods of detecting/diagnosing a neoplastic or preneoplastic condition in a mammal (for example, a human). "Diagnosis" as used herein generally includes determination of a patient's susceptibility to a disease or disorder, determination as to whether a subject is presently affected by a disease or disorder, prognosis of a subject affected by a disease or disorder (e.g., identification of pre-metastatic or metastatic cancerous states, stages of cancer, or responsiveness of cancer to therapy), and therametrics (e.g., monitoring a subject's condition to provide information as to the effect or efficacy of therapy).

One exemplary detection/diagnostic method includes: (a) obtaining from a mammal (e.g., a human) a biological sample (e.g., liver tissue), (b) detecting in the sample the presence of a Wnt 3 and/or Wnt 11 gene product (e.g., protein or mRNA), and (c) comparing the amount of Wnt 3 and/or Wnt 11 gene product present with that in a control sample. In accordance with this method, the presence in the sample of elevated levels of Wnt 3 gene product and/or reduced levels of Wnt 11 gene product indicates that the subject has a neoplastic or preneoplastic condition, e.g., liver cancer or a risk for developing liver cancer.

The identification of elevated levels of FZD 7 and/or Wnt 3 protein and/or reduced levels of FZD 8 and/or Wnt 11 protein makes possible the identification of patients that are likely to benefit from specialized therapy. For example, a biological sample from a post primary therapy subject (e.g., subject having undergone surgery) can be screened for the presence of elevated levels of FZD 7 and/or Wnt 3 and/or reduced levels of FZD 8 and/or Wnt 11 protein, such levels being indicative of residual tumor tissue. Similarly, tissue surrounding the cut site of a surgically removed tumor (e.g., peritumorous tissue) can be examined (e.g., by immunofluorescence), the presence of elevated levels of FZD 7 and/or Wnt 3 and/or reduced levels of FZD 8 and/or Wnt 11 (relative to the surrounding tissue) being indicative of potential development of disease in this tissue or incomplete removal of the tumor. The ability to identify such patients makes it possible to tailor therapy to the needs of the particular patient. Subjects undergoing non-surgical therapy, e.g., chemotherapy or radiation therapy, can also be monitored, the presence in samples from such subjects of elevated levels of FZD 7 and/or Wnt 3 and/or reduced levels of FZD 8 and/or Wnt 11 being indicative of the need for continued treatment. Skilled practitioners will also appreciate that staging of cancer (e.g., liver cancer) for purposes of optimizing treatment regimens can be performed using the methods described herein.

### III. Methods for Identifying Compounds Capable of Treating Cancer

The invention provides methods for screening test compounds for an ability to treat liver cancer. A "test compound" as described herein is any compound that can be screened using the methods described herein. For example, a test compound can be, e.g., a small organic or inorganic molecule (M.W. less than 1,000 Da). Alternatively or in addition, the test compound can be a polypeptide (e.g., a polypeptide having a random or predetermined amino acid sequence or a naturally-occurring or synthetic polypeptide) or a nucleic acid, such as a DNA or RNA molecule. A test compound can be naturally occurring (e.g., an herb or a natural product), or synthetic, or can include both natural and synthetic components. A test compound can have a formula weight of less than about 10,000 grams per mole, less than 5,000 grams per mole, less than 1,000 grams per mole, or less than about 500 grams per mole. The test compound can be, for example, any organic or inorganic compound (e.g., heteroorganic or organometallic compound), an amino acid, amino acid analog, polypeptide, peptidomimetic (e.g., peptoid), oligopeptide (e.g., from about 5 to about 25 amino acids in length, preferably from about 10 to 20 or 12 to 18 amino acids in length, preferably 12, 15, or 18 amino acids in length), nucleotide, nucleotide analog, polynucleotide, polynucleotide analog, ribonucleic acid, deoxyribonucleic acid, antisense oligonucleotide, ribozyme, saccharide, lipid (e.g., a sphingolipid), and/or a fatty acid, or any combination thereof.

The terms "antagonist" or "inhibitor" of Wnt/FZD signaling (e.g., Wnt/FZD 7 signaling) refer to compounds that, e.g., bind to Wnt proteins (e.g., Wnt 3, 8, and/or 11) and/or FZD receptors (e.g., FZD 7) and/or partially or totally block or inhibit Wnt/FZD signaling (e.g., Wnt/FZD 7 signaling) as measured in known assays for Wnt/FZD signaling (e.g., measurement of β-catenin levels, oncogene expression controlled by Tcf and Lef transcription factors or other downstream Wnt/Frizzled regulated gene products). Inhibitors include, e.g., antibodies directed against Wnt or FZD proteins (one example of an anti-Wnt3 antibody is described in the Examples section, below), modified versions of Wnt or FZD proteins, naturally occurring and synthetic ligands, antagonists, agonists, antibodies, small chemical molecules, and the like. Assays for detecting inhibitors or antagonists are described in more detail below.

### Libraries of Test Compounds

In certain embodiments, screens of the present invention utilize libraries of test compounds. A "library" is a collection of compounds (e.g., as a mixture or as physically separated individual compounds) synthesized from various combinations of one or more starting components. At least some of the compounds must differ from at least some of the other compounds in the library. A library can include, e.g., 5, 10, 50, 100, 1000, or even 10,000, 50,000, or 100,000, or more different compounds (i.e., not simply multiple copies of the same compounds, although some compounds in the library may be duplicated or represented more than once). Each of the different compounds will be present in an amount such that its presence can be determined by some means, e.g., can be isolated, analyzed, and/or detected with a receptor or suitable probe. The actual quantity of each different compound needed so that its presence can be determined will vary due to the actual procedures used and may change as the technologies for isolation, detection, and analysis advance. When the compounds are present in a mixture in substantially equimolar amounts, for example, an amount of 100 picomoles of each compound can often be detected. Libraries can include both libraries of individual compounds (e.g., present substantially as a single type of compound-per-well, made via parallel synthesis or the pool and split pool method) and mixtures containing substantially equimolar amounts of each desired compound (i.e., wherein no single compound dominates). Either library format can allow identification of an active compound discovered in an assay.

Test compounds can be screened individually or in parallel. An example of parallel screening is a high throughput drug screen of large libraries of chemicals. Such libraries of candidate compounds can be generated or purchased, e.g., from Chembridge Corp., San Diego, CA. Alternatively, prior experimentation and anecdotal evidence can suggest a class or category of compounds of enhanced potential. A library can be designed and synthesized to cover such a class of chemicals.

The synthesis of combinatorial libraries is well known in the art and has been reviewed (see, e.g., E.M. Gordon et al., J. Med. Chem. (1994) 37:1385-1401 ; DeWitt, S. H.; Czarnik, A. W. Acc. Chem. Res. (1996) 29:114; Armstrong, R. W.; Combs, A. P.; Tempest, P. A.; Brown, S. D.; Keating, T. A. Acc. Chem. Res. (1996) 29:123; Ellman, J. A. Acc. Chem. Res. (1996) 29:132; Gordon, E. M.; Gallop, M. A.; Pa.tel, D. V. Acc. Chem. Res. (1996) 29:144; Lowe, G. Chem. Soc. Rev. (1995) 309, Blondelle et al. Trends Anal. Chem. (1995) 14:83; Chen et al. J. Am. Chem. Soc. (1994) 116=2661; U.S. Patents 5,359,115, 5,362,899, and 5,288,514; PCT Publication Nos. WO92/10092, WO93/09668, WO91/07087, WO93/20242, WO94/08051).

Libraries of compounds can be prepared according to a variety of methods, some of which are known in the art. For example, a "split-pool" strategy can be implemented in the following way: beads of a functionalized polymeric support are placed in a plurality of reaction vessels; a variety of polymeric supports suitable for solid-phase peptide synthesis are known, and some are commercially available (for examples, see, e.g., M. Bodansky "Principles of Peptide Synthesis", 2nd edition, Springer-Verlag, Berlin (1993)). To each aliquot of beads is added a solution of a different activated amino acid, and the reactions are allowed to proceed to yield a plurality of immobilized amino acids, one in each reaction vessel. The aliquots of derivatized beads are then washed, "pooled" (i.e., recombined), and the pool of beads is again divided, with each aliquot being placed in a separate reaction vessel. Another activated amino acid is then added to each aliquot of beads. The cycle of synthesis is repeated until a desired peptide length is obtained. The amino acid residues added at each synthesis cycle can be randomly selected; alternatively, amino acids can be selected to provide a "biased" library, e.g., a library in which certain portions of the inhibitor are selected non-randomly, e.g., to provide an inhibitor having known structural similarity or homology to a known peptide capable of interacting with an antibody, e.g., the an anti-idiotypic antibody antigen binding site. It will be appreciated that a wide variety of peptidic, peptidomimetic, or non-peptidic compounds can be readily generated in this way.

The "split-pool" strategy can result in a library of peptides, e.g., modulators, which can be used to prepare a library of test compounds. In another illustrative synthesis, a "diversomer library" is created by the method of Hobbs DeWitt et al. (Proc. Natl. Acad. Sci. U.S.A. 90:6909 (1993)). Other synthesis methods, including the "tea-bag" technique of Houghten (see, e.g., Houghten et al., Nature 354:84-86 (1991)) can also be used to synthesize libraries of compounds. .

Libraries of compounds can be screened to determine whether any members of the library have a desired activity, and, if so, to identify the active species. Methods of screening combinatorial libraries have been described (see, e.g., Gordon et al., J Med. Chem.*, supra*). Soluble compound libraries can be screened by affinity chromatography with an appropriate receptor to isolate ligands for the receptor, followed by identification of the isolated ligands by conventional techniques (e.g., mass spectrometry, NMR, and the like). Immobilized compounds can be screened by contacting the compounds with a soluble receptor; preferably, the soluble receptor is conjugated to a label (e.g., fluorophores, colorimetric enzymes, radioisotopes, luminescent compounds, and the like) that can be detected to indicate ligand binding. Alternatively, immobilized compounds can be selectively released and allowed to diffuse through a membrane to interact with a receptor. Exemplary assays useful for screening libraries of test compounds are described above.

### Screening Methods

The invention provides methods for identifying compounds capable of treating cancer, e.g., liver cancer. Although applicants do not intend to be bound by any particular theory as to the biological mechanism involved, such compounds are thought to modulate specifically (1) Wnt/FZD signaling (e.g., by binding to FZD 7, Wnt 3 and/or Wnt 11 polypeptides and/or reducing (e.g., preventing) Wnt/FZD-mediated transcription) and/or (2) expression of FZD 7, Wnt3 and/or Wnt11.

In certain aspects of the present invention, screening for such compounds is accomplished by (i) identifying from a group of test compounds those that bind to a FZD 7, Wnt 3 and/or Wnt 11 polypeptide, modulate (i.e., increase or decrease) an interaction between FZD 7 and its ligand (e.g., Wnt 3, Wnt 8b and/or Wnt 11) and/or modulate (i.e., increase or decrease) transcription and/or translation of FZD 7 Wnt3 and/or Wnt11; and, optionally, (ii) further testing such compounds for their ability to modulate Wnt/FZD signaling, reduce cancer cell motility, reduce β-catenin accumulation in cancer cells and/or to treat cancer *in vitro* or *in vivo.* Test compounds that bind to FZD 7, Wnt 3 and/or Wnt 11 polypeptides, modulate an interaction between FZD 7 and its ligand (e.g., Wnt 3, Wnt 8b and/or Wnt 11), or modulate transcription and/or translation of FZD 7, Wnt3, and/or Wnt11, are referred to herein as "candidate anti-cancer agents." Candidate anti-cancer agents further tested and found to be capable of modulating *in vitro* or *in vivo* Wnt/FZD signaling, reducing cancer cell motility, reduce β-catenin accumulation in cancer cells and/or treating cancer are considered "anti-cancer agents." In the screening methods of the present invention, candidate anti-cancer agents can be, but do not necessarily have to be, tested to determine whether they are anti-cancer agents. Assays of the present invention may be carried out in biological samples, whole cell preparations and/or *ex vivo* cell-free systems.

In one aspect, the invention includes methods for screening test compounds to identify compounds that bind to FZD polypeptides, e.g., FZD 7 polypeptides, and/or to Wnt polypeptides, e.g., Wnt 3 and/or 11 polypeptides. Binding of a test compound to a FZD or Wnt polypeptide can be detected, for example, *in vitro* by reversibly or irreversibly immobilizing the test compound(s) or the Wnt or FZD polypeptide on a substrate, e.g., the surface of a well of a 96-well polystyrene microtitre plate. Methods for immobilizing polypeptides and other small molecules are well known in the art. For example, microtitre plates can be coated with a FZD or Wnt polypeptide by adding the polypeptide in a solution (typically, at a concentration of 0.05 to 1 mg/ml in a volume of 1-100 µl) to each well, and incubating the plates at room temperature to 37°C for a given amount of time, e.g., for 0.1 to 36 hours. Polypeptides not bound to the plate can be removed by shaking excess solution from the plate, and then washing the plate (once or repeatedly) with water or a buffer. Typically, the polypeptide is in water or a buffer. The plate can then be washed with a buffer that lacks the bound polypeptide. To block the free protein-binding sites on the plates, plates can be blocked with a protein that is unrelated to the bound polypeptide. For example, 300 µl of bovine serum albuminutes (BSA) at a concentration of 2 mg/ml in Tris-HCl can be used. Suitable substrates include those substrates that contain a defined cross-linking chemistry (e.g., plastic substrates, such as polystyrene, styrene, or polypropylene substrates from Coming Costar Corp. (Cambridge, MA), for example). If desired, a particle, e.g., beaded agarose or beaded sepharose, can be used as the substrate. Test compounds can then be added to the coated plate and allowed to bind to the FZD or Wnt polypeptide (e.g., at 37°C for 0.5 - 12 hours). The plate can then be rinsed as described above. Skilled practitioners will appreciate that many variations of this method are possible. For example, the method can include coating a substrate with a test compound and adding Wnt or FZD polypeptides to the substrate-bound compound.

Binding of FZD or Wnt to a second compound, e.g., a test compound described above or to a binding partner (e.g., FZD 7 to Wnt 3 and/or 11; discussed in further detail below), can be detected by any of a variety of art-known methods. For example, an antibody that specifically binds to a FZD or Wnt polypeptide (i.e., an anti-FZD antibody or an anti-Wnt antibody, e.g., a polyclonal anti-Wnt3 antibody described in the Examples section) can be used in an immunoassay. If desired, the antibody can be labeled (e.g., fluorescently or with a radioisotope) and detected directly (*see,* e.g., West and McMahon, J. Cell Biol. 74:264, 1977). Alternatively, a second antibody can be used for detection (e.g., a labeled antibody that binds to the Fc portion of the anti-FZD or anti-Wnt antibody). In an alternative detection method, the FZD or Wnt polypeptide is labeled (e.g., with a radioisotope, fluorophore, chromophore, or the like), and the label is detected. In still another method, a FZD or Wnt polypeptide is produced as a fusion protein with a protein that can be detected optically, e.g., green fluorescent protein (which can be detected under UV light). In an alternative method, the polypeptide is produced as a fusion protein with an enzyme having a detectable enzymatic activity, such as horseradish peroxidase, alkaline phosphatase, β-galactosidase, or glucose oxidase. Genes encoding all of these enzymes have been cloned and are available for use by skilled practitioners. If desired, the fusion protein can include an antigen or epitope that can be detected and measured with a polyclonal or monoclonal antibody using conventional methods. Suitable antigens include enzymes (e.g., horse radish peroxidase, alkaline phosphatase, and β-galactosidase) and non-enzymatic polypeptides (e.g., serum proteins, such as BSA and globulins, and milk proteins, such as caseins).

In various methods for identifying polypeptides (e.g., test polypeptides) that bind to FZD or Wnt polypeptides, the conventional two-hybrid assays of protein/protein interactions can be used (*see* e.g., Chien et al., Proc. Natl. Acad. Sci. USA, 88:9578, 1991; Fields et al., U.S. Pat. No. 5,283,173; Fields and Song, Nature, 340:245, 1989; Le Douarin et al., Nucleic Acids Research, 23:876, 1995; Vidal et al., Proc. Natl. Acad. Sci. USA, 93:10315-10320, 1996; and White, Proc. Natl. Acad. Sci. USA, 93:10001-10003, 1996). Generally, two-hybrid methods involve reconstitution of two separable domains of a transcription factor. One fusion protein includes the FZD or Wnt polypeptide fused to either a transactivator domain or DNA binding domain of a transcription factor (e.g., of Gal4). The other fusion protein contains a test polypeptide or a binding partner for the polypeptide included in the first fusion protein, fused to either the DNA binding domain or a transactivator domain of a transcription factor. Binding of the FZD or Wnt polypeptide to the test polypeptide or binding partner reconstitutes the transcription factor. Reconstitution of the transcription factor can be detected by detecting expression of a gene (i.e., a reporter gene) that is operably linked to a DNA sequence that is bound by the DNA binding domain of the transcription factor. Kits for practicing various two-hybrid methods are commercially available (e.g., from Clontech; Palo Alto, CA).

In another aspect, the invention includes methods for screening test compounds to identify a compound that modulates a protein-protein interaction between FZD and Wnt polypeptides. A method useful for high throughput screening of compounds capable of modulating protein-protein interactions between transcriptional regulators is described in Lepourcelet et al., Cancer Cell 5: 91-102 (2004) . Typically, a first compound is provided. The first compound is a FZD (e.g., FZD 7) or Wnt (e.g., Wnt 3, or 11) polypeptide or biologically active fragment thereof. A second compound is provided that is different from the first compound and is labeled. The second compound is a FZD (e.g., FZD 7) or Wnt (e.g., Wnt 3, or 11) polypeptide or biologically active fragment thereof. A test compound is provided. The first compound, second compound and test compound are contacted with each other. The amount of label bound to the first compound is then determined. A change in protein-protein interaction between the first compound and the second compound as assessed by label bound is indicative of the usefulness of the test compound in modulating a protein-protein interaction between the FZD and Wnt polypeptide.

In certain embodiments, the first compound provided is attached to a solid support. Solid supports include, e.g., resins (e.g., agarose and beads) and multiwell plates. In certain embodiments, the method includes a washing step after the contacting step, so as to separate bound and unbound label.

In certain embodiments, a plurality of test compounds is contacted with the first compound and second compound. The different test compounds can be contacted with the other compounds in groups or separately. In certain embodiments, each of the test compounds is contacted with both the first compound and the second compound in an individual well. For example, the method can screen libraries of test compounds. Libraries of test compounds are discussed in detail above. Libraries can include, e.g., natural products, organic chemicals, peptides, and/or modified peptides, including, e.g., D-amino acids, unconventional amino acids, and N-substituted amino acids. Typically, the libraries are in a form compatible with screening in multiwell plates, e.g., 96-well plates. The assay is particularly useful for automated execution in a multiwell format in which many of the steps are controlled by computer and carried out by robotic equipment. The libraries can also be used in other formats, e.g., synthetic chemical libraries affixed to a solid support and available for release into microdroplets.

In certain embodiments, the first compound is a FZD 7 polypeptide or fragment thereof and the second compound is a Wnt polypeptide, such as Wnt 3, or 11, or fragment thereof. In other embodiments, the first compound is a Wnt polypeptide, such as Wnt 3, or 11 polypeptide or fragment thereof, and the second compound is a FZD 7 polypeptide or fragment thereof. The solid support to which the first compound is attached can be, e.g., sepharose beads, SPA beads (microspheres that incorporate a scintillant) or a multiwell plate. SPA beads can be used when the assay is performed without a washing step, e.g., in a scintillation proximity assay. Sepharose beads can be used when the assay is performed with a washing step. The second compound can be labeled with any label that will allow its detection, e.g., a radiolabel, a fluorescent agent, biotin, a peptide tag, or an enzyme fragment. The second compound can also be radiolabeled, e.g., with ¹²⁵I or ³H.

In certain embodiments, the enzymatic activity of an enzyme chemically conjugated to, or expressed as a fusion protein with, the first or second compound, is used to detect bound protein. A binding assay in which a standard immunological method is used to detect bound protein is also included. In certain other embodiments, the interaction of Wnt and FZD polypeptides or fragments thereof is detected by fluorescence resonance energy transfer (FRET) between a donor fluorophore covalently linked to a FZD or Wnt polypeptide (e.g., a fluorescent group chemically conjugated to FZD or Wnt, or a variant of green fluorescent protein (GFP) expressed as an FZD or Wnt-GFP chimeric protein) and an acceptor fluorophore covalently linked to a substrate protein, where there is suitable overlap of the donor emission spectrum and the acceptor excitation spectrum to give efficient nonradiative energy transfer when the fluorophores are brought into close proximity through the protein-protein interaction of FZD and Wnt polypeptides.

In other embodiments, the protein-protein interaction is detected by reconstituting domains of an enzyme, e.g., beta-galactosidase (see Rossi et al, Proc. Natl. Acad. Sci. USA 94:8405-8410 (1997)).

In still other embodiments, the protein-protein interaction is assessed by fluorescence ratio imaging (Bacskai et al, Science 260:222-226 (1993)) of suitable chimeric constructs of FZD and Wnt polypeptides in cells, or by variants of the two-hybrid assay (Fearon et al, Proc Natl Acad Sci USA 89:7958-7962 (1992); Takacs et al, Proc Natl Acad Sci USA 90:10375-10379 (1993); Vidal et al, Proc Natl Acad Sci USA 93:10321-10326 (1996)) employing suitable constructs of FZD and Wnt polypeptides and tailored for a high throughput assay to detect compounds that inhibit the FZD/Wnt interaction. These embodiments have the advantage that the cell permeability of the test compounds is assured.

For example, in one assay, a FZD or Wnt polypeptide or fragment thereof is adsorbed to ELISA plates. The FZD or Wnt polypeptides are then exposed to test compounds, followed by a glutathione-S-transferase (GST)-binding partner fusion protein, e.g., a GST-FZD or -Wnt polypeptide fusion protein. Bound protein is detected with goat anti-GST antibody, alkaline phosphatase (AP)-coupled anti-goat IgG, and AP substrate. Compounds that interfere with protein-protein interactions yield reduced AP signals in the ELISA plates.

Also described herein are methods of identifying test compounds that modulate (e.g., increase or decrease) expression of a FZD and/or Wnt polypeptide. The method includes contacting a FZD and/or Wnt nucleic acid with a test compound and then measuring expression of the encoded FZD and/or Wnt polypeptide. In a related aspect, the invention features a method of identifying compounds that modulate (e.g., increase or decrease) the expression of FZD and/or Wnt polypeptides by measuring expression of a FZD polypeptide in the presence of the test compound or after the addition of the test compound in: (a) a cell line into which has been incorporated a recombinant construct including the FZD and/or Wnt nucleic acid sequence or fragment or an allelic variation thereof; or (b) a cell population or cell line that naturally selectively expresses FZD and/or Wnt, and then measuring the expression of the FZD and/or Wnt protein.

Since the FZD and Wnt nucleic acids described herein have been identified, they can be cloned into various host cells (e.g., mammalian cells, insect cells, bacteria or fungi) for carrying out such assays in whole cells.

An isolated nucleic acid molecule encoding a FZD and/or Wnt polypeptide may be used to identify a compound that modulates (e.g., increases or decreases) the expression of FZD and/or Wnt *in vivo* (e.g., in a FZD and/or Wnt-producing cell). In such embodiments, cells that express a FZD (e.g., FZD 7 and/or 8) and/or Wnt (e.g., Wnt3, Wnt8b or Wnt11) are cultured, exposed to a test compound (or a mixture of test compounds), and the level of FZD and/or Wnt expression is compared with the level of FZD and/or Wnt expression or activity in cells that are otherwise identical but that have not been exposed to the test compound(s). Standard quantitative assays of gene expression can be used.

Expression of FZD and Wnt can be measured using art-known methods, for example, by Northern blot PCR analysis or RNAse protection analyses using a nucleic acid molecule of the invention as a probe. Other examples include enzyme-linked immunosorbent assay (ELISA), radioimmunoassay (RIA) and fluorescent activated cell sorting (FACS). The level of expression in the presence of the test molecule, compared with the level of expression in its absence, will indicate whether or not the test compound modulates the expression of a FZD and/or Wnt polypeptide.

Further described are methods of screening test compounds utilizing cell systems that are sensitive to perturbation of one or several transcriptional/translational components.

The methods include identifying candidate compounds that interfere with steps in FZD and/or Wnt translational accuracy, such as maintaining a proper reading frame during translation and terminating translation at a stop codon. This method involves constructing cells in which a detectable reporter polypeptide can only be produced if the normal process of staying in one reading frame or of terminating translation at a stop codon has been disrupted. This method further involves contacting the cell with a test compound to examine whether it increases or decreases the production of the reporter polypeptide.

The cell system may be a cell-free extract and the method involves measuring transcription or translation *in vitro.* Conditions are selected so that transcription or translation of the reporter is increased or decreased by the addition of a transcription modifier or a translation modifier to the cell extract.

One method for identifying candidate compounds relies upon a transcription-responsive gene product. This method involves constructing a cell in which the production of a reporter molecule changes (i.e., increases or decreases) under conditions in which cell transcription of a FZD and/or Wnt nucleic acid changes (i.e., increases or decreases). Specifically, the reporter molecule is encoded by a nucleic acid transcriptionally linked to a sequence constructed and arranged to cause a relative change in the production of the reporter molecule when transcription of a FZD and/or Wnt nucleic acid changes. A gene sequence encoding the reporter may, for example, be fused to part or all of the gene encoding the transcription-responsive gene product and/or to part or all of the genetic elements that control the production of the gene product. Alternatively, the transcription-responsive gene product may stimulate transcription of the gene encoding the reporter, either directly or indirectly. The method further involves contacting the cell with a test compound, and determining whether the test compound increases or decreases the production of the reporter molecule in the cell.

Alternatively, the method for identifying candidate compounds can rely upon a translation-responsive gene product. This method involves constructing a cell in which cell translation of a FZD and/or Wnt nucleic acid changes (i.e., increases or decreases). Specifically, the reporter molecule is encoded by nucleic acid translationally linked to a sequence constructed and arranged to cause a relative increase or decrease in the production of the reporter molecule when transcription of a FZD and/or Wnt nucleic acid changes. A gene sequence encoding the reporter may, for example, be fused to part or all of the gene encoding the translation-responsive gene product and/or to part or all of the genetic elements that control the production of the gene product. Alternatively, the translation-responsive gene product may stimulate translation of the gene encoding the reporter, either directly or indirectly. The method further involves contacting the cell with a test compound, and determining whether the test compound increases or decreases the production of the first reporter molecule in the cell.

For these and any method described herein, a wide variety of reporters may be used, with typical reporters providing conveniently detectable signals (e.g., by spectroscopy). By way of example, a reporter gene may encode an enzyme that catalyses a reaction that alters light absorption properties.

Examples of reporter molecules include but are not limited to β-galactosidase, invertase, green fluorescent protein, luciferase, chloramphenicol acetyltransferase, beta-glucuronidase, exo-glucanase, glucoamylase and radiolabeled reporters. For example, the production of the reporter molecule can be measured by the enzymatic activity of the reporter gene product, such as β-galactosidase.

Any of the methods described herein can be used for high throughput screening of numerous test compounds to identify candidate anti-cancer agents. By high-throughput screening is meant that the method can be used to screen a large number of candidate compounds relatively easily and quickly.

Having identified a test compound as a candidate anti-cancer agent, the compound can be further tested *in vivo* or *in vitro* using techniques known in the art to confirm whether it is an anti-cancer agent, i.e., to determine whether it can modulate Wnt/FZD signaling; cancer cell motility; and/or FZD and/or Wnt expression *in vitro* (e.g., using isolated cells or cell-free systems) or *in vivo* (e.g., using an animal, e.g., rodent, model system) if desired.

*In vitro* testing of a candidate compound can be accomplished by means known to those in the art, such as assays involving the use of cells, e.g., wild type, cancerous and/or transgenic liver cells. Exemplary assays for monitoring Wnt/FZD signaling, FZD and Wnt expression and cancer cell motility, as well as useful cells that can be used in such assays, are described in the Examples section, below.

Alternatively or in addition, *in vivo* testing of candidate compounds can be performed by means known to those in the art. For example, the candidate compound(s) can be administered to a mammal, such as a rodent (e.g., mouse) or rabbit. Such animal model systems are art-accepted for testing potential pharmaceutical agents to determine their therapeutic efficacy in patients, e.g., human patients. Animals that are particularly useful for *in vivo* testing are wild type animals or non-wild type animals (e.g., mice) that over-produce FZD and/or Wnt polypeptides, e.g., animals that overexpress a FZD or Wnt transgene (e.g., a FZD 7 or Wnt3 transgene) and/or that display reduced production of FZD 8 and/or Wnt11 polypeptides. Other animals that are useful for *in vivo* testing are animals bred to develop liver cancer. Certain particularly useful transgenic mice that develop liver cancer are described in the Examples section and are included in the present invention.

In a typical *in vivo* assay, an animal (e.g., a wild type or transgenic mouse) is administered, by any route deemed appropriate (e.g., by injection), a dose of a candidate compound. Conventional methods and criteria can then be used to monitor animals for the desired activity. If needed, the results obtained in the presence of the candidate compound can be compared with results in control animals that are not treated with the test compound.

### Medicinal Chemistry

Once a compound (or agent) of interest has been identified, standard principles of medicinal chemistry can be used to produce derivatives of the compound for further rounds of testing. Derivatives can be screened for improved pharmacological properties, for example, efficacy, pharmaco-kinetics, stability, solubility, and clearance. The moieties responsible for a compound's activity in the assays described above can be delineated by examination of structure-activity relationships (SAR) as is commonly practiced in the art. A person of ordinary skill in pharmaceutical chemistry could modify moieties on a candidate compound or agent and measure the effects of the modification on the efficacy of the compound or agent to thereby produce derivatives with increased potency. For an example, see Nagarajan et al. (1988) J. Antibiot. 41: 1430-8. Furthermore, if the biochemical target of the compound (or agent) is known or determined, the structure of the target and the compound can inform the design and optimization of derivatives. Molecular modeling software is commercially available (e.g., Molecular Simulations, Inc.) for this purpose.

### IV. Antibodies

The invention features purified or isolated antibodies that bind, e.g., specifically bind, to a Wnt polypeptide, i.e., anti-Wnt antibodies. An antibody "specifically binds" to a particular antigen, e.g., a FZD 7 polypeptide, when it binds to that antigen, but recognizes and binds to a lesser extent (e.g., does not recognize and bind) to other molecules in a sample. Antibodies of the invention include monoclonal antibodies, polyclonal antibodies, humanized or chimeric antibodies, single chain antibodies, Fab fragments, F(ab')₂ fragments, and molecules produced using a Fab expression library.

An example of a type of antibody included in the present invention is the polyclonal anti-Wnt3 antibody described in the Examples section, below. Methods for producing polyclonal antibodies are well known to those of skill in the art.

As used herein, the term "antibody" refers to a protein comprising at least one, e.g., two, heavy (H) chain variable regions (abbreviated herein as VH), and at least one, e.g., two light (L) chain variable regions (abbreviated herein as VL). The VH and VL regions can be further subdivided into regions ofhypervariability, termed "complementarity determining regions" ("CDR"), interspersed with regions that are more conserved, termed "framework regions" (FR). The extent of the framework region and CDR's has been precisely defined (see, Kabat, B.A., et al. (1991) Sequences of Proteins of Immunological Interest, Fifth Edition, U.S. Department of Health and Human Services, NIH Publication No. 91-3242, and Chothia, C. et al. (1987) J. Mol. Biol. 196:901-917). Each VH and VL is composed of three CDR's and four FRs, arranged from amino-terminus to carboxy-terminus in the following order: FR1, CDR1, FR2, CDR2, FR3, CDR3, FR4.

An anti-FZD or -Wnt antibody can further include a heavy and light chain constant region, to thereby form a heavy and light immunoglobulin chain, respectively. The antibody can be a tetramer of two heavy immunoglobulin chains and two light immunoglobulin chains, wherein the heavy and light immunoglobulin chains are interconnected by, e.g., disulfide bonds. The heavy chain constant region is comprised of three domains, CH1, CH2, and CH3. The light chain constant region is comprised of one domain, CL. The variable region of the heavy and light chains contains a binding domain that interacts with an antigen. The constant regions of the antibodies typically mediate the binding of the antibody to host tissues or factors, including various cells of the immune system (e.g., effector cells) and the first component (Clq) of the classical complement system.

A "FZD binding fragment" and "Wnt binding fragment" of an antibody refers to one or more fragments of a full-length antibody that retain the ability to specifically bind to FZD or Wnt polypeptides, respectively, or to portions thereof. Examples of polypeptide binding fragments of an antibody include, but are not limited to: (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Furthermore, although the two domains of the Fv fragment, VL and VH, are encoded by separate genes, they can be joined, using recombinant methods, by a synthetic linker that enables them to be made as a single protein chain in which the VL and VH regions pair to form monovalent molecules (known as single chain Fv (scFv); see *e.g.,* Bird et al. (1988) Science 242:423-426; and Huston et al. (1988) Proc. Natl. Acad. Sci. USA 85:5879-5883). Such single chain antibodies are also encompassed within the terms "FZD binding fragment" and "Wnt binding fragment" of an antibody. These antibody fragments can be obtained using conventional techniques known to those with skill in the art.

To produce antibodies, polypeptides (or antigenic fragments (e.g., fragments of a polypeptide that appear likely to be antigenic by criteria such as high frequency of charged residues) or analogs of such polypeptides), e.g., those produced by recombinant or peptide synthetic techniques (see, e.g., *Solid Phase Peptide Synthesis,* supra; Ausubel et al., supra), can be used. In general, the polypeptides can be coupled to a carrier protein, such as KLH, as described in Ausubel et al., supra, mixed with an adjuvant, and injected into a host mammal. A "carrier" is a substance that confers stability on, and/or aids or enhances the transport or immunogenicity of, an associated molecule. For example, FZD or Wnt proteins, or fragments thereof, can be generated using standard techniques of PCR, and can be cloned into a pGEX expression vector (Ausubel et al., supra). Fusion proteins can be expressed in *E. coli* and purified using a glutathione agarose affinity matrix as described in Ausubel et al., supra.

Typically, various host animals are injected with FZD and/or Wnt polypeptides. Examples of suitable host animals include rabbits, mice, guinea pigs, and rats. Various adjuvants can be used to increase the immunological response, depending on the host species, including but not limited to Freund's (complete and incomplete adjuvant), adjuvant mineral gels such as aluminum hydroxide, surface active substances such as lysolecithin, pluronic polyols, polyanions, peptides, oil emulsions, keyhole limpet hemocyanin, dinitrophenol, BCG (bacille Calmette-Guerin) and *Corynebacterium parvum.* Such procedures result in the production of polyclonal antibodies, i.e., heterogeneous populations of antibody molecules derived from the sera of the immunized animals. Antibodies can be purified from blood obtained from the host animal, for example, by affinity chromatography methods in which FZD and/or Wnt polypeptide antigens are immobilized on a resin.

The present invention also includes anti-Wnt monoclonal antibodies. Monoclonal antibodies (mAbs), which are homogeneous populations of antibodies specific for a particular antigen, can be prepared using Wnt polypeptides and standard hybridoma technology (see, e.g., Kohler et al., Nature, 256:495, 1975; Kohler et al., Eur. J. Immunol., 6:511, 1976; Kohler et al., Eur. J. Immunol., 6:292, 1976; Hammerling et al., In Monoclonal Antibodies and T Cell Hybridomas, Elsevier, NY, 1981; Ausubel et al., supra).

Typically, monoclonal antibodies are produced using any technique that provides for the production of antibody molecules by continuous cell lines in culture, such as those described in Kohler et al., Nature, 256:495, 1975, and U.S. Patent No. 4,376,110; the human B-cell hybridoma technique (Kosbor et al., Immunology Today, 4:72, 1983; Cole et al., Proc. Natl. Acad. Sci. USA, 80:2026, 1983); and the EBV-hybridoma technique (Cole et al., Monoclonal Antibodies and Cancer Therapy, Alan R. Liss, Inc., pp. 77-96, 1983). Such antibodies can be of any immunoglobulin class including IgG, IgM, IgE, IgA, IgD, and any subclass thereof. The hybridomas producing the mAbs of this invention can be cultivated *in vitro* or *in vivo.*

Once produced, polyclonal or monoclonal antibodies can be tested for recognition, e.g., specific recognition, of FZD or Wnt polypeptides in an immunoassay, such as a Western blot or immunoprecipitation analysis using standard techniques, e.g., as described in Ausubel et al., supra. Antibodies that specifically bind to FZD or Wnt polypeptides (e.g., FZD 7, Wnt 3, and/or Wnt 11) are useful in the invention. For example, such antibodies can be used in an immunoassay to detect the polypeptide in a sample, e.g., a tissue sample, and/or to modulate FZD/Wnt signaling (e.g., to treat cancer, e.g., liver cancer).

Alternatively or in addition, a monoclonal antibody can be produced recombinantly, e.g., produced by phage display or by combinatorial methods as described in, e.g., Ladner et al. U.S. Patent No. 5,223,409; Kang et al. International Publication No. WO 92/18619; Dower et al. International Publication No. WO 91/17271; Winter et al. International Publication WO 92/20791; Markland et al. International Publication No. WO 92/15679; Breitling et al. International Publication WO 93/01288; McCafferty et al. International Publication No. WO 92/01047; Garrard et al. International Publication No. WO 92/09690; Ladner et al. International Publication No. WO 90/02809; Fuchs et al. (1991) Bio/Technology 9:1370-1372; Hay et al. (1992) Hum Antibod Hybridomas 3:81-85; Huse et al. (1989) Science 246:1275-1281; Griffths et al. (1993) EMBO J 12:725-734; Hawkins et al. (1992) J Mol Biol 226:889-896; Clackson et al. (1991) Nature 352:624-628; Gram et al. (1992) PNAS 89:3576-3580; Garrad et al. (1991) Bio/Technology 9:1373-1377; Hoogenboom et al. (1991) Nuc Acid Res 19:4133-4137; and Barbas et al. (1991) PNAS 88:7978-7982.

Anti-FZD and -Wnt antibodies can be fully human antibodies (e.g., an antibody made in a mouse which has been genetically engineered to produce an antibody from a human immunoglobulin sequence), or non-human antibodies, e.g., rodent (mouse or rat), rabbit, horse, cow, goat, primate (e.g., monkey), camel, donkey, pig, or bird antibodies.

An anti-FZD and anti-Wnt antibody can be one in which the variable region, or a portion thereof, e.g., the CDRs, is generated in a non-human organism, e.g., a rat or mouse. The anti-FZD and anti-Wnt antibody can also be, for example, chimeric, CDR-grafted, or humanized antibodies. The anti-FZD and anti-Wnt antibody can also be generated in a non-human organism, e.g., a rat or mouse, and then modified, e.g., in the variable framework or constant region, to decrease antigenicity in a human.

Techniques developed for the production of "chimeric antibodies" (Morrison et al., Proc. Natl. Acad. Sci., 81:6851, 1984; Neuberger et al., Nature, 312:604, 1984; Takeda et al., Nature, 314:452, 1984) can be used to splice the genes from a mouse antibody molecule of appropriate antigen specificity together with genes from a human antibody molecule of appropriate biological activity. A chimeric antibody is a molecule in which different portions are derived from different animal species, such as those having a variable region derived from a murine mAb and a human immunoglobulin constant region.

Alternatively, techniques described for the production of single chain antibodies (U.S. Patent 4,946,778; and U.S. Patents 4,946,778 and 4,704,692) can be adapted to produce single chain antibodies specific for a FZD or Wnt polypeptide. Single chain antibodies are formed by linking the heavy and light chain fragments of the Fv region via an amino acid bridge, resulting in a single chain polypeptide.

Antibody fragments that recognize and bind to specific epitopes can be generated by known techniques. For example, such fragments can include but are not limited to F(ab')₂ fragments, which can be produced by pepsin digestion of the antibody molecule, and Fab fragments, which can be generated by reducing the disulfide bridges of F(ab')₂ fragments. Alternatively, Fab expression libraries can be constructed (Huse et al., Science, 246:1275, 1989) to allow rapid and easy identification of monoclonal Fab fragments with the desired specificity.

Polyclonal and monoclonal antibodies (or fragments thereof) that specifically bind to a FZD and/or Wnt polypeptide can be used, for example, to detect expression of FZD and/or Wnt in various tissues of a patient. For example, a FZD 7 and/or 8 polypeptide can be detected in conventional immunoassays of biological tissues or extracts. Examples of suitable assays include, without limitation, Western blotting, ELISAs, radioimmunoassays, and the like.

### V. Pharmaceutical Compositions

Any pharmaceutically active compound, agent, nucleic acid, polypeptide, or antibody (all of which can be referred to herein as "active compounds"), can be incorporated into pharmaceutical compositions. Such compositions typically include the active compound and a pharmaceutically acceptable carrier. A "pharmaceutically acceptable carrier" can include solvents, dispersion media, coatings, antibacterial and antifungal agents, isotonic and absorption delaying agents, and the like, compatible with pharmaceutical administration. Supplementary active compounds can also be incorporated into the compositions.

A pharmaceutical composition can be formulated to be compatible with its intended route of administration. Examples of routes of administration include enteral (e.g., oral or rectal) and parenteral, e.g., intravenous (e.g., into the portal vein of the liver), intradermal, subcutaneous, transdermal, transmucosal, and pulmonary administration. Administration may be directly into the liver, e.g., by injection or by topical administration during surgery. Solutions or suspensions used for injection can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol or sorbitol and sodium chloride. Prolonged absorption of the injectable compositions can be achieved by including an agent which delays absorption, e.g., aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying, which yield a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or com starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressured container or dispenser that contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories (e.g., with conventional suppository bases such as cocoa butter and other glycerides). For transdermal administration, the active compounds are formulated into ointments, salves, gels, or creams as generally known in the art.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions can also be used as pharmaceutically acceptable carriers. These can be prepared according to methods known to those skilled in the art, for example, as described in U.S. Patent No. 4,522,811.

It may be advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index, and it can be expressed as the ratio LD50/ED50. Compounds which exhibit high therapeutic indices are preferred. While compounds that exhibit toxic side effects may be used, care should be taken to design a delivery system that targets such compounds to the site of affected tissue, e.g., liver, in order to minimize potential damage to healthy cells and, thereby, reduce side effects.

The data obtained from cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound that achieves a half-maximal inhibition of symptoms) as determined in cell culture. Such information can be used to more accurately determine useful doses in humans. Levels in plasma may be measured, for example, by high performance liquid chromatography.

The terms "effective amount" and "effective to treat," as used herein, refer to an amount or concentration of a compound described herein utilized for a period of time (including acute or chronic administration and periodic or continuous administration) that is effective within the context of its administration for causing an intended effect or physiological outcome. For compounds described herein, an effective amount, e.g., of a polypeptide (i.e., an effective dosage), ranges from about 0.001 to 500 mg/kg body weight, e.g. about 0.01 to 50 mg/kg body weight, e.g. about 0.1 to 20 mg/kg body weight. The polypeptide can be administered one time per week for between about 1 to 10 weeks, e.g. between 2 to 8 weeks, about 3 to 7 weeks, or for about 4, 5, or 6 weeks. The skilled artisan will appreciate that certain factors influence the dosage and timing required to effectively treat a patient, including but not limited to the type of patient to be treated, the severity of the disease or disorder, previous treatments, the general health and/or age of the patient, and other diseases present. Moreover, treatment of a patient with a therapeutically effective amount of a compound can include a single treatment or, preferably, can include a series of treatments.

With respect to antibodies, partially human antibodies and fully human antibodies have a longer half life within the human body than other antibodies. Accordingly, lower dosages and less frequent administration are possible. Modifications such as lipidation can be used to stabilize antibodies and to enhance uptake and tissue penetration. A method for lipidation of antibodies is described by Cruikshank et al. ((1997) J. Acquired Immune Deficiency Syndromes and Human Retrovirology 14:193).

If the compound is a small molecule, exemplary doses include milligram or microgram amounts of the small molecule per kilogram of subject or sample weight (e.g., about 1 microgram per kilogram to about 500 milligrams per kilogram, about 100 micrograms per kilogram to about 5 milligrams per kilogram, or about 1 microgram per kilogram to about 50 micrograms per kilogram. It is furthermore understood that appropriate doses of a small molecule depend upon the potency of the small molecule with respect to the expression or activity to be modulated. When one or more of these small molecules is to be administered to an animal (e.g., a human) to modulate expression or activity of a FZD or Wnt polypeptide or nucleic acid, a physician, veterinarian, or researcher may, for example, prescribe a relatively low dose at first, subsequently increasing the dose until an appropriate response is obtained. In addition, it is understood that the specific dose level for any particular animal subject will depend upon a variety of factors including the activity of the specific compound employed, the age, body weight, general health, gender, and diet of the subject, the time of administration, the route of administration, the rate of excretion, any drug combination, and the degree of expression or activity to be modulated.

Nucleic acid molecules (e.g., FZD 7, FZD 8, Wnt 3, Wnt8b and/or Wnt 11 DNA) can be inserted into vectors and used as gene therapy vectors. Gene therapy vectors can be delivered to a subject by, for example, intravenous injection, local administration (see, e.g., U.S. Patent 5,328,470) or by stereotactic injection (see, e.g., Chen et al. (1994) Proc. Natl. Acad. Sci. USA 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system. Exemplary constructs that can potentially be used in gene therapy methods are described in the Examples section, below.

The pharmaceutical compositions can be included in a container, pack, or dispenser together with instructions for administration.

### VI. Cancer and Treatments Therefor

The term "cancer" refers to animal cells having the capacity for autonomous growth. Examples of such cells include cells having an abnormal state or condition characterized by rapidly proliferating cell growth. The term is meant to include cancerous growths, e.g., tumors; oncogenic processes, metastatic tissues, and malignantly transformed cells, tissues, or organs, irrespective of histopathologic type or stage of invasiveness. Also included are malignancies of the various organ systems, such as respiratory, cardiovascular, renal, reproductive, hematological, neurological, hepatic, gastrointestinal, and endocrine systems; as well as adenocarcinomas which include malignancies such as most colon cancers, renal-cell carcinoma, prostate cancer and/or testicular tumors, non-small cell carcinoma of the lung, cancer of the small intestine, and cancer of the esophagus. Cancer that is "naturally arising" includes any cancer that is not experimentally induced by implantation of cancer cells into a subject, and includes, for example, spontaneously arising cancer, cancer caused by exposure of a patient to a carcinogen(s), cancer resulting from insertion of a transgenic oncogene or knockout of a tumor suppressor gene, and cancer caused by infections, e.g., viral infections. The term "carcinoma" is art recognized and refers to malignancies of epithelial or endocrine tissues. The term includes carcinosarcomas, which include malignant tumors composed of carcinomatous and sarcomatous tissues. An "adenocarcinoma" refers to a carcinoma derived from glandular tissue or in which the tumor cells form recognizable glandular structures. The term "hapatocellular carcinoma" (HCC) refers to cancer that arises from hepatocytes, the major cell type of the liver.

The term "patient" is used throughout the specification to describe an animal, human or non-human, rodent or non-rodent, to whom treatment according to the methods of the present invention is provided Veterinary and human clinical applications are contemplated. The term "patient" includes, but is not limited to, birds, reptiles, amphibians, and mammals, e.g., humans, other primates, pigs, rodents such as mice and rats, rabbits, guinea pigs, hamsters, cows, horses, cats, dogs, sheep and goats. Preferred subjects are humans, farm animals, and domestic pets such as cats and dogs. The term "treat(ment)," is used herein to denote delaying the onset of, inhibiting, alleviating the effects of, or prolonging the life of a patient suffering from, a condition, e. g., cancer.

Cancers that may be treated using the methods and compositions of the present invention include, but are not limited to, cancers of the liver, stomach, colon, rectum, mouth/pharynx, esophagus, larynx, pancreas, lung, small bowel, and bile ducts, among others.

Individuals considered at risk for developing liver cancer may benefit particularly from the invention, primarily because prophylactic treatment can begin before there is any evidence of a tumor. Individuals "at risk" include, e.g., individuals exposed to carcinogens, e.g., by consumption, e.g., by inhalation and/or ingestion, at levels that have been shown statistically to promote cancer in susceptable individuals. Also included are individuals exposed to a virus, e.g., a hepatitis virus, e.g., hepatitis B virus (HBV). Also included are individuals at risk due to exposure to ultraviolet radiation, or their environment, occupation, and/or heredity, as well as those who show signs of a precancerous condition. Similarly, individuals in very early stages of cancer or development of metastases (i.e., only one or a few aberrant cells are present in the individual's body or at a particular site in an individual's tissue)) may benefit from such prophylactic treatment.

Skilled practitioners will appreciate that a patient can be diagnosed by a physician (or veterinarian, as appropriate for the patient being diagnosed) as suffering from or at risk for cancer using the methods described herein, optionally using additional methods, e.g., assessing a patient's medical history, performing other diagnostic tests and/or by employing imaging techniques.

One strategy for treating patients suffering from or at risk for cancer is to modulate Wnt/FZD signaling in the patient. The goal is to increase signaling where signaling is too low and to decrease signaling where signaling is too high. Modulation of Wnt/FZD signaling falls into two basic categories: decreasing (i.e., reducing, e.g., eliminating) Wnt/FZD signaling and increasing (i.e., supplementing or providing) Wnt/FZD signaling where there is insufficient or no activity. Whether Wnt/FZD signaling should be inhibited or increased depends upon the intended application. Wnt/FZD signaling can be modulated using the active compounds (e.g., anti-Wnt antibodies, siRNAs, candidate compounds and/or anti-cancer agents) described herein. Compounds that decrease Wnt/FZD signaling activity, e.g., by decreasing expression of FZD 7 and/or Wnt3 and/or interfering with an interaction between FZD 7 and its ligand (e.g., Wnt 3, 8b and/or 11) can be used, e.g., as treatments for cancer, e.g., liver cancer. Compounds that increase activity, e.g., by increasing expression of FZD 8 can also be used, e.g., as treatments for cancer, e.g., liver cancer.

### Decreasing Wnt/FZD Signaling

Art-known methods for decreasing the expression of a particular protein in a patient can be used to decrease Wnt/FZD signaling. For example, an antisense nucleic acid effective to inhibit expression of an endogenous FZD or Wnt gene, e.g., FZD 7 or Wnt3 gene, can be utilized. As used herein, the term "antisense oligonucleotide" or "antisense" describes an oligonucleotide that is an oligoribonucleotide, oligodeoxyribonucleotide, modified oligoribonucleotide, or modified oligodeoxyribonucleotide which hybridizes under physiological conditions to DNA comprising a particular gene or to an mRNA transcript of that gene and, thereby, inhibits the transcription of that gene and/or the translation of that mRNA.

Antisense molecules are designed so as to interfere with transcription or translation of a target gene (e.g., a gene encoding FZD 7 or Wnt 3, 8b or 11) upon hybridization with the target gene or transcript. The antisense nucleic acid can include a nucleotide sequence complementary to an entire FZD or Wnt RNA or only a portion of the RNA. On one hand, the antisense nucleic acid needs to be long enough to hybridize effectively with FZD or Wnt RNA. Therefore, the minimum length is approximately 12 to 25 nucleotides. On the other hand, as length increases beyond about 150 nucleotides, effectiveness at inhibiting translation may increase only marginally, while difficulty in introducing the antisense nucleic acid into target cells may increase significantly. Accordingly, an appropriate length for the antisense nucleic acid may be from about 15 to about 150 nucleotides, e.g., 20, 25, 30, 35, 40, 45, 50, 60, 70, or 80 nucleotides. The antisense nucleic acid can be complementary to a coding region of FZD or Wnt mRNA or a 5' or 3' non-coding region of a FZD or Wnt mRNA, or both. One approach is to design the antisense nucleic acid to be complementary to a region on both sides of the translation start site of the FZD or Wnt mRNA.

Based upon the sequences disclosed herein, one of skill in the art can easily choose and synthesize any of a number of appropriate antisense molecules for use in accordance with the present invention. For example, a "gene walk" comprising a series of oligonucleotides of 15-30 nucleotides complementary to and spanning the length of a FZD or Wnt mRNA can be prepared, followed by testing for inhibition of FZD or Wnt expression. Optionally, gaps of 5-10 nucleotides can be left between the oligonucleotides to reduce the number of oligonucleotides synthesized and tested.

The antisense nucleic acid can be chemically synthesized, e.g., using a commercial nucleic acid synthesizer according to the vendor's instructions. Alternatively, the antisense nucleic acids can be produced using recombinant DNA techniques. An antisense nucleic acid can incorporate only naturally occurring nucleotides. Alternatively, it can incorporate variously modified nucleotides or nucleotide analogs to increase its in vivo half-life or to increase the stability of the duplex formed between the antisense molecule and its target RNA. Examples of nucleotide analogs include phosphorothioate derivatives and acridine-substituted nucleotides. Given the description of the targets and sequences, the design and production of suitable antisense molecules is within ordinary skill in the art. For guidance concerning antisense nucleic acids, see, e.g., Goodchild, "Inhibition of Gene Expression by Oligonucleotides," in Topics in Molecular and Structural Biology, Vol. 12: Oligodeoxynucleotides (Cohen, ed.), MacMillan Press, London, pp. 53-77 (1989).

Delivery of antisense oligonucleotides can be accomplished by any method known to those of skill in the art. For example, delivery of antisense oligonucleotides for cell culture and/or ex vivo work can be performed by standard methods such as the liposome method or simply by addition of membrane-permeable oligonucleotides.

Delivery of antisense oligonucleotides for *in vivo* applications can be accomplished, for example, via local injection of the antisense oligonucleotides at a selected site, e.g., a liver. This method has previously been demonstrated for psoriasis growth inhibition and for cytomegalovirus inhibition. *See,* for example, Wraight et al., (2001). Pharmacol Ther. 90(1):89-104; Anderson et al., (1996) Antimicrob Agents Chemother 40: 2004-2011; and Crooke et al., (1996) J Pharmacol Exp Ther 277: 923-937.

Similarly, RNA interference (RNAi) techniques can be used to inhibit FZD or Wnt expression, in addition or as an alternative to the use of antisense techniques. For example, small interfering RNA (siRNA) duplexes directed against FZD or Wnt nucleic acids could be synthesized and used to prevent expression of the encoded protein(s). Exemplary Wnt3 siRNAs are described in the Examples section, below.

Another approach to inhibiting Wnt/FZD signaling involves administering to a patient a compound, e.g., a candidate compound or anti-cancer agent, that binds to FZD polypeptides (e.g., FZD 7 polypeptides) and/or their binding partners (e.g., Wnt 3, 8b and/or 11), thereby preventing interaction between the two. Such compounds and agents may, for example, bind to the FZD polypeptide (e.g., to the CRD domain of the FZD polypeptide) and/or to the Wnt polypeptide (e.g., to a binding domain of the Wnt polypeptide) in such a way that interaction between the proteins is prevented. Such candidate compounds and anti-cancer agents can be identified using screening methods described herein. Examples of compounds that can bind to a Wnt polypeptide, e.g., Wnt 3, 8b and/or 11, are a FZD 7 receptor or truncated form thereof, and an anti-Wnt antibody (or FZD-binding fragment thereof), e.g., the anti-Wnt3 antibody described in the Examples section, below.

Yet another approach to inhibiting Wnt/FZD signaling involves administering to a patient a vector (e.g., a gene therapy vector) that encodes a mutated (e.g., truncated) form of a FZD receptor, e.g., a FZD 7 receptor. Expression of the mutated form of the receptor by the patient's cells that incorporate the construct can interfere with Wnt/FZD signaling in the cells. For example, a construct that encodes a secreted and soluble form of a FZD receptor (e.g., a FZD 7 receptor) can be used. Expression of such a construct by target cells would cause the cells to secrete a soluble form of the FZD receptor that would bind Wnt polypeptides, rendering them unable to bind to intact FZD receptors on the cell surface. Alternatively or in addition, a construct that encodes a membrane bound but inactive form of a FZD receptor (i.e., a mutant FZD receptor unable to perform some function performed by a counterpart wild-type FZD receptor) can be used. Expression of such a construct by target cells may bind up Wnt polypeptides or interfere with Wnt/FZD signaling via an internal mechanism not involving Wnt polypeptides. Still another approach to inhibiting Wnt/FZD signaling involves administering to a patient a vector (e.g., a gene therapy vector) that encodes a Wnt11 polypeptide, which is a suppressor of the canonical Wnt pathway in HCC. The vector can be derived from a non-replicating linear or circular DNA or RNA vector, or from an autonomously replicating plasmid or viral vector. Methods for constructing suitable expression vectors are known in the art, and useful materials are commercially available.

### Increasing Wnt/FZD Signaling

New or supplemental Wnt/FZD signaling can be provided *in vivo* by increasing expression of FZD polypeptides (e.g., FZD 8 polypeptides) and/or Wnt polypeptides (e.g., Wnt3 polypeptides) in the patient. For example, a FZD or Wnt polypeptide can be generated directly within an organism, e.g., a human, by expressing within the cells of the organism a nucleic acid construct containing a nucleotide sequence encoding a FZD polypeptide (e.g., a FZD 8 polypeptide) and/or Wnt polypeptide (e.g., Wnt3 polypeptide). Any appropriate expression vector suitable for transfecting the cells of the organism of interest can be used for such purposes.

### VII. Transgenic Animals

The present invention also features transgenic animals that develop liver cancer and overexpress FZD 7 in their liver cells. Such animals represent model systems for the study of liver cancer and for the development of therapeutic agents that can modulate Wnt/FZD signaling and treat cancer.

Transgenic animals can be, for example, farm animals (pigs, goats, sheep, cows, horses, rabbits, and the like), rodents (such as rats, guinea pigs, and mice), non-human primates (for example, baboons, monkeys, and chimpanzees), and domestic animals (for example, dogs and cats).

Any technique known in the art can be used to introduce transgenes into animals to produce the founder lines of transgenic animals. Such techniques include, but are not limited to, pronuclear microinjection (U.S. Pat. No. 4,873,191); retrovirus mediated gene transfer into germ lines (Van der Putten et al., Proc. Natl. Acad. Sci., USA 82:6148, 1985); gene targeting into embryonic stem cells (Thompson et al., Cell 56:313, 1989); and electroporation of embryos (Lo, Mol. Cell. Biol. 3:1803, 1983). Especially useful are the methods described in Yang et al. (Proc. Natl Acac. Sci. USA 94:3004-3009, 1997).

For a review of techniques that can be used to generate and assess transgenic animals, skilled artisans can consult Gordon (Intl. Rev. Cytol. 115:171-229, 1989), and may obtain additional guidance from, for example: Hogan et al. Manipulating the Mouse Embryo, Cold Spring Harbor Press, Cold Spring Harbor, NY, 1986); Krimpenfort et al. (Bio/Technology 9: 86, 1991), Palmiter et al. (Cell 41:343, 1985), Kraemer et al. (Genetic Manipulation of the Early Mammalian Embryo, Cold Spring Harbor Press, Cold Spring Harbor, NY, 1985), Hammer et al.(Nature 315:680, 1985), Purcel et al. (Science, 244:1281, 1986), Wagner et al. (U.S. Patent No. 5,175,385), and Krimpenfort et al. (U.S. Patent No. 5,175,384).

### EXAMPLES

The invention is illustrated in part by the following examples, which are not to be taken as limiting the invention in any way.

### Example 1. Identification of natual Wnt ligands for Inhibition of HCC growth

### Methods

### Preparation of cell surface HSPG and fractionation by heparin-agarose chromatography.

Huh7 cells were grown to 70% confluency in 10% FBS (Sigma, St. Louis, MO) MEM (Mediatech, Herndon, VA, USA). The media were changed to 0% FBS MEM 24 hrs after incubation, and the cells were treated with or without heparin (50 ug/ml) in 0.1% FBS MEM for 12 hrs. Cells were washed 5 times with ice-cold PBS and cell layers were incubated with crystallized trypsin (20 ug/ml) in Tris-buffered saline/EDTA for 10 min on ice. Trypsin activity was stopped by the addition of soybean trypsin inhibitor at a final concentration of 100 ug/ml. Contaminated cells were removed by centrifugation at 400 x g for 5 min at 4°C from trypsinate.

The trypsinate was subjected to heparin-agarose chromatography. In brief, trypsinate was incubated with heparin (4%) agarose beads for overnight at 4°C and beads were collected by centrifugation at 2000 x g for 4 min, washed beads with 20 volumes of 0.1 M NaCl in PBS. Eluted fractions were collected with 0.25, 0.5, 0.75, and 1.0 M NaCl in PBS.

### Two-dimensional gel electrophoresis, in-gel digestion and peptide mapping.

Fractionated samples from heparin-agarose chromatography were prepared by precipitation and rehydration with IPG buffer for isoelectric focusing (IEF). IEF was carried out using ZOOM IPGRunner (Invitrogen™, Carlsbad, CA), according to the manufacturer's protocol. ZOOM strips (pH 3-10) were rehydrated with samples for overnight, then a step voltage ramping method was applied as follows: 200 V for 20 min, 450 V for 15 min, 750 V for 15 min, and 2000 V for 120 min. Focused gels were subjected to SDS-PAGE using ZOOM gels (Invitrogen) as second dimensional electrophoresis. Following electrophoresis, gels were stained using SilverQuest Silver Staining kit (Invitrogen).

Protein spots excised from silver-stained gels were destained and dried before enzymatic digestion with sequence grade modified trypsin (Promega, Madison, WI). Tryptic peptides were desalted and concentrated with ZipTip_{C18} (Millipore, Bedford, MA). The concentrated tryptic peptides were applied to SEND ProteinChip and performed peptide mapping using PBSII (Ciphergen, Fremont, CA). Peptide mass fingerprinting was conducted with the database search tool MS-fit in the program Protein Prospector, available at http://prospector.ucsf.edu. A number of restrictions were applied to the initial search based on localization of the spot in the 2-D gel: species = homo sapiens, pI range = 6-9.5, mass range = 35 - 50 kDa.

### RT-PCR Analysis.

Total cellular RNA was extracted by using TRIzol^{®} Reagent (Invitrogen) from HepG2, Hep3B, Huh7 and Focus cell lines. First-strand cDNA was synthesized from 250 ng of total RNA with random hexamers and AMV RT using First Strand cDNA Synthesis Kit for RT-PCR (AMV) (Roche Diagnostics, Indianapolis, IN) in 20 µl of the reaction mixture. PCR was performed in a thermocycler (MJ Research Inc., Waltham, MA) using 50 ng of cDNA and High Fidelity PCR Master (Roche Diagnostics). The primer pairs for each Wnt ligand are listed in Table 1, below. The final concentration of each primer was 250 nM. After initial denaturation at 94°C for 4 min, reactions were subjected to 35 cycles of the following thermal program: 94°C for 30 s, 55°C for 30 s, and 72°C for 1 min, followed by a final elongation step at 72°C for 10 min. The amplified products were analyzed on ethidium bromide-stained 2% agarose gel.

### qRT-PCR assay.

Extraction of total RNA and RT reaction from the liver tissues and HCC cell lines were conducted as described above. To determine the mRNA expression levels of Wnt3, Wnt11 and FZD7, qRT-PCR was performed using iCycler iQ Multi-Color Real Time PCR Detection System (Bio-Rad, Hercules, CA) with a mixture composed of SYBR Green PCR Master Mix (Applied Biosystems, Foster City, CA), 400 nM of each primer and 5 ng of cDNA (equivalent total RNA) from unknown samples. The thermal cycling conditions comprised an initial step at 95°C for 10 min, followed by 40 cycles at 95°C for 15 s, 60°C for 30 s, and 72°C for 30 s. The primer sequences for Wnt3, Wnt11, FZD7 and 18SrRNA were as follows:
(1) Wnt3, 5'-ACTTCGGCGTGTTAGTGTCC-3' (forward) (SEQ ID NO:68) and 5'-CATTTGAGGTGCATGTGGTC-3' (reverse) (SEQ ID NO:69);
(2) Wnt11, 5'-TTCCGATGCTCCTATGAAGG-3' (forward) (SEQ ID NO:70) and 5'-AGACACCCCATGGCACTTAC-3' (reverse) (SEQ ID NO:71);
(3) FZD7, 5'-GCCGCTTCTACCACAGACT-3' (forward) (SEQ ID NO:72) and 5'-TTCATACCGCAGTCTCCCC-3' (reverse) (SEQ ID NO:73);
(4) 18SrRNA, 5'-GGACACGGACAGGATTGACA-3' (forward) (SEQ ID NO:74) and 5'-ACCCACGGAATCGAGAAAGA-3' (reverse) (SEQ ID NO:75). The sizes of amplicons were 130 bp for Wnt3, 133 bp for Wnt11, 54 bp for FZD7 and 50 bp for 18SrRNA. After visualization of the PCR products by 1.5% agarose gel electrophoresis and ethidium bromide, PCR products were excised and cloned into the pCR 2.1 Vector (Invitrogen). Sequencing was performed in both directions using T7 forward and M13 reverse primers. After confirmation of the nucleotide sequences, standards for the real-time PCR were prepared with 10-fold dilutions of the each PCR products cloned into the pCR 2.1 Vector. The copy numbers of Wnt3, Wnt1 1 and FZD7 mRNAs were quantified in unknown samples by measuring the Ct values followed by normalization to 18SrRNA after comparison with standard curves for each gene. Experiments were performed in duplicate.

### Preparation of plasmids and anti-Wnt3 Ab.

Because 22 amino acids from the C-terminal end of the human Wnt3 plasmid were missing in the original plasmid (22), it was extended to the full-length sequence using three runs of PCR amplification based on the sequence of human Wnt3, which was cloned into pcDNA™3.1/myc-His A (Invitrogen) via HindIII and EcoRI restriction enzyme sites. The sequences of primers were
5'-TAGTTAAGCTTACCATGGAGCCCCACCTGCTC-3' (forward) (SEQ ID NO:76),
5'-GCAGCTGACGTAGCAGCACCAGTGGAAGATGCAGTG-3' (reverse-1) (SEQ ID NO:77),
5'-GTAGATGCGAATACACTCCTGGCAGCTGACGTAGCA-3' (reverse-2) (SEQ ID NO:78),
5'-TGCTTGAATTCCTTGCAGGTGTGCACGTCGTAGATGCGAATACA-3' (reverse -3) (SEQ ID NO:79).

Rabbit polyclonal antibodies were prepared against a synthetic peptide corresponding to amino acids 259-274 of human Wnt3 (²⁵⁹LRAKYSLFKPPTERDL²⁷⁴) (SEQ ID NO:80). The peptide sequence did not share significant homology with other members of Wnt family or other known proteins. The specificity of antibody was verified by Western blot analysis using HCC cell lines transfected with myc-tagged Wnt3 plasmid.

### Cell Culture

Hep3B, Huh7 and Focus cell lines were grown in MEM, supplemented with 10% FBS and 1X minimum essential medium nonessential amino acid solution (Sigma). HepG2 cells were excluded because of the deletion mutation in the β-catenin gene (31). Transfection experiments were performed at 70% confluency using LipofectAMINE 2000 (Invitrogen) or TransIT-LT1 (Mirus, Madison, WI) according to the manufacture's instructions. Tcf transcriptional activity was analyzed using Luciferase Assay System (Promega, Madison, WI) after transfecting cells with pSUPER8xTOPFLASH or pSUPER8xFOPFLASH (32). Raw data for luciferase activity were normalized using β-galactosidase activity as a transfection control. Experiments were carried out in triplicate and repeated three times to verify results.

### Effects of Wnt3 overexpression in HCC cells.

The HCC cells were transfected with pcDNA3.1/*myc*-His A (control plasmid) or Wnt3 plasmids, pSUPER8xTOPFLASH or pSUPER8xFOPFLASH, and β-galactosidase plasmid. Twenty-four hours after transfection, the cells were incubated with 0% FBS MEM for 24 h, and then stimulated with 1% FBS MEM. The cells were harvested at 2 h and 24 h after the stimulation, and subjected to the luciferase assay and Western blot analysis for Wnt3 and β-catenin.

### Effects of anti-Wnt3 Antibodies.

For blocking experiments, cells were seeded in 12-well plates and transfected with pSUPER8xTOPFLASH or pSUPER8xFOPFLASH with β-galactosidase plasmid as described above. After serum-deprivation for 24 h, the cells were incubated with 1% FBS MEM containing either anti-Wnt3 Ab or control Ab (10 µg/ml), and harvested 24 h after incubation. Normal rabbit IgG (Upstate, Waltham, MA) was used as a control antibody.

### Effects of siRNA.

Control siRNA and Wnt3 siRNAs
(WNT3-1: 5'-GGAAAAAUGCCACUGCAUC-3' (SEQ ID NO:81),
WNT3-2: 5'-GGAGUGUAUUCGCAUCUAC-3'(SEQ ID NO:82),
WNT3-3: 5'-GGCUUAUCUUUGCACAUGU-3' (SEQ ID NO:83)) were purchased from Ambion (Austin, TX) and transfected into HCC cells at a concentration of 10 or 100 nM with pSUPER8xTOPFLASH or pSUPER8xFOPFLASH, and β-galactosidase plasmid using TransIT-LT1 or DharmaFECT 4 (Dharmacon, Chicago, IL). Forty-eight hours after transfection, the mRNA expression levels of Wnt3 and Tcf transcriptional activity were measured by qRT-PCR and luciferase assay, respectively.

### Effect of blocking anti-Wnt3 Ab on wound healing.

Focus cells were plated in a 6-well plate. Confluent monolayer cells were wounded with sterile plastic 200 µl micropipette tips. The cells were then treated with media with either anti-Wnt3 Ab or rabbit IgG, and photographed at different time points.

### Western blot analysis

For total protein extraction, the cells were homogenized in lysis buffer (30 mM Tris, pH 7.5, 150 mM sodium chloride, 1% NP-40, 0.5% sodium deoxycholate, 0.1 % SDS, 10% glycerol, and 2mM EDTA) with proteinase inhibitors (Roche Diagnostics) and sonicated. Protein concentration was determined using the BCA Protein Assay Kit (Pierce, Rockford, IL) with BSA as standard. Equal amounts of proteins (150 µg) were separated using 12-15% SDS-PAGE and transferred to PVDF membranes (PerkinElmer, Wellesley, MA). The membranes were blocked with 5% BSA in Tris-buffered saline containing 0.1% Tween 20 and then incubated overnight at 4°C with a mouse monoclonal anti-myc Ab diluted at 1:1,000 (Santa Cruz Biotechnology Inc., Santa Cruz, CA), a rabbit polyclonal anti-Wnt3 Ab diluted at 1:200, a mouse monoclonal anti-β-catenin Ab diluted at 1:1,000 (Transduction Laboratories, San Diego, CA), or a rabbit polyclonal anti-hsp90 Ab diluted at 1:2,000 (Santa Cruz Biotechnology Inc.). After washing with Tris-buffered saline containing 0.1 % Tween 20, the membrane was incubated for 1.5 hour at room temperature with a secondary HRP antibody diluted 1:10,000 and visualized using the chemiluminescence imaging Western Lightning (PerkinElmer).

### Human HCC tissues.

Seventeen pairs of HCC and matched, uninvolved, peritumoral liver tissues were obtained from South Korea and South Africa. Twelve pairs of samples were from Korean patients who underwent surgical resection. Nine of the 12 patients were males and the median age was 52 (range, 22-67). Eleven patients were positive for hepatitis B surface antigen (HBsAg) and the etiology was unknown in the remaining 1 patient. Seven patients (58%) had underlying liver cirrhosis. Five liver tissues, obtained from the Korean patients who underwent hepatic resection for single metastasis of colorectal cancers to the liver, served as the controls for normal liver tissues. Four patients were males and the median age was 46 (range 37-57). Histologic examination did not show any pathology in the surrounding, peritumoral liver tissues.

### Immunohistochemistry for β-catenin.

Formalin-fixed, paraffin-embedded sections were deparaffinized in xylene and rehydrated by decreasing concentrations of ethanol. Sections were immersed in 10 mM sodium citrate buffer (pH 6.0), boiled in a microwave oven for 10 min, and cooled to room temperature for epitope retrieval. Slides were then processed using a Universal DakoCytomation LSAB^{®}+ Kit, Peroxidase (DAKO Corp., Carpinteria, CA) according to the manufacturer's instructions. Endogenous peroxidase, avidin and biotin activities were blocked by incubation with 3% hydrogen peroxide and Endogenous Avidin/Biotin Blocking Kit (Zymed Laboratories Inc., South San Francisco, CA). Sections were incubated at 4°C overnight with a 1:500 dilution of anti-human β-catenin monoclonal Ab. For negative controls, the primary antibody was replaced with PBS. The β-catenin expression patterns were classified into two groups according to the presence of nuclear staining. Cytoplasmic staining in tumor tissues was also compared to that in peritumoral tissues.

### Mutational analysis for exon-3 of the β-catenin gene.

Exon 3 mutation of β-catenin gene was analyzed by the method of Wong et al. (11) with some modifications. Briefly, a 218-bp fragment of exon 3 of the β-catenin gene was amplified from cDNA (50 ng) from HCCs and surrounding peritumoral liver tissues using Expand High Fidelity PCR System (Roche Diagnostics). The sequences of primers were 5'-GATTTGATGGAGTTGGACATGG-3' (forward) (SEQ ID NO:84) and 5'-TGTTCTTGAGTGAAGGACTGAG-3' (reverse) (SEQ ID NO:85). The thermal cycling conditions comprised an initial denaturating step at 94°C for 3 min, followed by 35 cycles at 95°C for 30 s, 58°C for 30 s, and 72°C for 30 s. After visualization of the PCR products, PCR products were cloned into the pCR 2.1 Vector (Invitrogen) and subsequently sequenced using T7 forward and M13 reverse primers. At least 5 clones from each PCR product were analyzed for the sequencing.

### Results

### Identification of Wnt ligands in HCC cell lines.

Since Wnt proteins are mainly associated with ECM in cell surface, an attempt was made to purify cell surface HSPG including Wnt protein(s) as an associated form in Huh7. Huh7-HSPG prepared by trypsin was subjected to heparin-agarose affinity resin for pre-fractionation. Eluted fractions were applied to SDS-PAGE and compared the protein bands between heparin-treated and non-treated samples. From a 0.25 M NaCl eluted fraction, an approximately 45 kDa protein band was distinguishable in the heparin-untreated fraction (data not shown). As the estimated molecular weight of Wnt proteins is in the range of 35-45 kDa, this protein band was considered to potentially include Wnt proteins. To define this protein band, two-dimensional electrophoresis was performed. As shown in Fig. 1A, there were several silver-stained protein spots showing different levels of protein expression between heparin-treated and untreated samples. Protein spots of 35-55 kDa and pI 5-9.5 were considered candidates for Wnt ligand proteins. Two up-regulated protein spots in heparin-untreated samples were investigated since Wnt proteins can be released by heparin treatment. Excised protein spots from silver-stained two-dimensional gels were subjected to in-gel digestion, followed by peptide mapping by mass spectrometry. Data analysis revealed that nine peptides matched human Wnt11 protein, as shown in Fig. 1B. To further corroborate this finding, Wnt mRNA expression was examined by RT-PCR in HCC cell lines using 19 pairs of primers specific for all the known human Wnt ligands. As shown in Fig. 1C, only Wnt3 and Wnt11 mRNA, among all 19 Wnt genes examined, were expressed in HCC cell lines. None of other known Wnt mRNAs were detected by RT-PCR. After Wnt3 and Wnt11 were identified in HCC cell lines, the mRNA expression levels were examined using quantitative real-time RT-PCR (qRT-PCR) assay in HCC cell lines. The expression levels (mean ± SE) of Wnt3 mRNA in HCC cell lines were 370.0 ± 10.3 in HepG2, 381.3 ± 12.7 in Hep3B, 95.2 ± 6.3 in Huh7 and 210.4 ± 9.5 copies per 10⁹ 18S ribosomal RNA (18SrRNA). The Wnt11 mRNA expression levels were 8,499.3 ± 845.0 in HepG2, 290.9 ± 40.1 in Hep3B and 3.57 ± 0.2 copies per 10⁹ 18SrRNA in Huh7 cells. Wnt11 mRNAs could not be detected in Focus cells with real-time RT-PCR, consistent with the result of conventional RT-PCR (Fig. 2B).

### Expression of Wnt3, Wnt11 and FZD7 mRNAs in human HCC tissues.

Thirteen of 17 (76.5%) HCC and 10 of 17 (58.8%) peritumoral tissues showed increased Wnt3 mRNA expression levels compared with those in normal controls, when the cut-off level was defined as a value of mean ± 3 SD in normal liver tissues. Only 1 peritumoral tissue showed decreased expression compared with normal liver tissues. Among 17 paired samples, 12 (70.6%) showed increased Wnt3 mRNA expression in tumors compared with corresponding, peritumoral tissues, but it was not statistically significant (P = .435 by Wilcoxon signed-ranks test) (Fig. 3A). In contrast to Wnt3, 11 of 17 (64.7%) paired samples showed decreased expression of Wnt11 mRNA in tumors compared with corresponding, peritumoral tissues, which was not still statistically significant (P = .227 by Wilcoxon signed-ranks test). However, 7 of 17 tumor tissues (41.2%) showed decreased expression even below the lower cut-off level of normal liver tissues, while none did in peritumoral tissues (P = .0036 by Fischer's exact test). Five of 17 (29.4%) tumor tissues showed increased expression of Wnt11 mRNA compared with normal liver tissues, while 9 of 17 (52.9%) peritumoral tissues did. Both HCC and peritumoral tissues showed increased FZD7 mRNA expression in 10 of 17 (58.8%) samples compared with normal liver tissues (Figure 3B). Twelve of 17 (70.6%) paired samples showed increased expression of FZD7 mRNA in tumors compared with corresponding peritumoral tissues, and this difference was statistically significant (P = .031 by Wilcoxon signed-ranks test) (Fig. 3C).

### Nuclear accumulation of β-catenin.

Immunohistochemical staining showed nuclear accumulation of β-catenin in 7 of 17 (41%) HCC tissues. There was no nuclear accumulation of β-catenin in surrounding, peritumoral tissues (Fig. 4). All of the 7 samples with nuclear accumulation of β-catenin also showed increased staining for β-catenin in the cytoplasm. Among the 10 tumor tissues without the nuclear accumulation of β-catenin, 1 sample showed increased cytoplasmic staining for β-catenin, compared to its corresponding peritumoral tissue.

By sequencing analysis, mutations on exon 3 of β-catenin gene were found in 4 of 17 (23.5%) HCC samples, while none in surrounding, peritumoral tissues. They were all single missense mutations affecting codons 35, 37 and 45 (two I35S, one S37C, and one S45F). Three of the 7 (42.9%) samples that showed nuclear accumulation of β-catenin by immunohistochemical staining had mutations at the region responsible for phosphorylation and ubiquitination of β-catenin. One sample that had a mutation in β-catenin gene (I35S) but did not have nuclear accumulation of β-catenin had increased cytoplasmic staining in immunohistochemical staining. All of the 4 remaining samples that had nuclear accumulation of β-catenin but did not have any mutation in β-catenin gene had increased FZD7 mRNA levels compared with paired peritumoral tissues. The FZD7 mRNA levels were also increased by 7-74 folds compared with the mean value of normal liver tissues. However, the mRNA expression levels of Wnt3 and/or Wnt11 were not related with β-catenin nuclear accumulation.

### Effects of Wnt3 overexpression in HCC cell lines.

To determine the effect of Wnt3 on the canonical Wnt pathway in HCC, changes of T-cell factor (Tcf) transcriptional activity were examined after transfection with Wnt3 plasmid. Transfection with Wnt3 plasmid resulted in marked increases of both mRNA (data not shown) and protein expression levels, as demonstrated in Fig. 5A. The Tcf transcriptional activities also showed about 3-fold increases in Focus cells, compared to those of controls, which was statistically significant (P < 0.01). However, the Tcf transcriptional activities were not changed in Hep3B cells, and even decreased in Huh7 cells, especially 24 h after stimulation (Fig. 5B). Consistent with these results, the cellular β-catenin level was increased in Focus cells after transfection with Wnt3 plasmid, while not changed or decreased in Hep3B or Huh7 cells, as demonstrated by Western blot analysis (Fig. 5A).

### Antagonizing Wnt signaling by anti-Wnt3 Ab or Wnt3 siRNAs.

Next, the effects of inhibition by anti-Wnt3 Ab or siRNAs on the canonical pathway in HCC cell lines was examined, because the baseline Tcf transcriptional activities and cellular β-catenin levels were already high in Huh7 and Hep3B cells, compared to Focus cells (Fig. 5). Incubation with polyclonal anti-Wnt3 Ab resulted in decreases of luciferase activities by 60% in Huh7, 26% in Hep3B, and 40% in Focus cells (Fig. 6A). To confirm this inhibitory effect on the canonical pathway, siRNAs for Wnt3 were used. First, the inhibition efficiencies of 3 different kinds of siRNAs were evaluated in Huh7 and Hep3B cells using qRT-PCR assay. It was found that siRNA Wnt3-3 was the most efficient (data not shown) with an average decrease of mRNA levels by 50-60% at a concentration of 100 nM (Fig. 6B). Consistent with these reductions of mRNAs, the Tcf transcriptional activities were also decreased by 48.5% in Huh7, 33% in Hep3B, and 43.5% in Focus cells (Fig. 6C). Therefore, it was concluded that inhibition of Wnt3 could result in suppression of the canonical pathway in HCC cell lines.

### Effects of anti-Wnt3 Ab treatment on wound healing in HCC cells.

Whether inhibition of Tcf transcriptional activity could lead to functional changes of HCC cell behavior was also investigated. Wound healing assay using Focus cells showed delayed wound healing in cells treated with anti-Wnt3 Ab, and these changes were most prominent at 24 h. At 24 h, most of the wound was covered with migrating and/or proliferating cells in Focus cells treated with control Ab, while it persisted in cells treated with anti-Wnt3 Ab (Fig. 7).

This study analyzed the expression pattern of Wnt ligands in the liver and HCCs. mRNAs of Wnt3 and Wnt11 were found to be expressed in most of the HCC cell lines tested using conventional RT-PCR and qRT-PCR methods. Wnt11 expression at its protein level was also demonstrated using proteomics technology. Consistent with these observations in HCC cell lines, Wnt3 and Wnt11 mRNA expression was also confirmed in human liver tissues that included HCCs.

The expression patterns of Wnt3, Wnt11, and FZD7 was determined in human liver tissues including HCC and corresponding peritumoral tissues. mRNA levels of FZD7 in tumor tissues were more elevated than those in corresponding peritumoral tissues and normal liver tissues. Although there was no statistically significant difference in Wnt3 mRNA expression between HCC and peritumoral tissues, 71% of HCCs showed increased Wnt3 expression compared to their corresponding peritumoral tissues. Furthermore, 77% of HCCs and 59% of peritumoral tissues showed increased Wnt3 mRNA expression levels above the value of mean ± 3 SD in normal liver tissues. These findings suggest that upregulation of Wnt3 might be an early event during hepatocarcinogenesis and/or play an important role in hepatocyte regeneration during hepatic inflammation and necrosis.

Further, 65% of paired HCC samples showed decreased expression of Wnt11 mRNA in tumors compared with corresponding peritumoral tissues. 41% of tumor tissues showed decreased expression levels of Wnt11 even below the lower cut-off level of normal liver tissues. These findings were compatible with Wnt11's role as a suppressor of the canonical pathway.

Wnt3 overexpression was also found to activate the canonical Wnt pathway in Focus cells, as evidenced by a 3-fold increase of Tcf transcriptional activity and increased cellular β**-**catenin levels. However, the Tcf transcriptional activities were not changed in Hep3B cells and even decreased slightly in Huh7 cells, even though the Wnt3 mRNA and protein expressions were markedly increased after transfection.

Inhibition of Wnt3 by polyclonal anti-Wnt3 Ab or siRNAs decreased the Tcf transcriptional activities in all 3 HCC cell lines. These changes were most prominent in Huh7 cells that had the highest baseline Tcf transcriptional activities. Further, treatment of Focus cells with anti-Wnt3 Ab inhibited wound healing, suggesting that the functional consequence of this inhibition was decreased cell migration and proliferation.

Nuclear and/or cytoplasmic accumulation of β-catenin was observed in 8 of 17 (47%) HCC tissues and half of these cases had β-catenin gene mutations. The remaining 4 cases with β-catenin accumulation but without mutations had marked elevated levels of FZD7 in tumors, suggesting that FZD7 upregulation is directly related with the activation of the canonical Wnt signaling pathway in these tumors. The expression levels of Wnt3 or Wnt11 were related neither with the β-catenin accumulation nor with that of FZD7.

In conclusion, Wnt3 and Wnt11 was identified in HCC cell lines and in human liver tissues including HCC tissues. Wnt3 mRNA expression levels were upregulated in both HCC and peritumoral tissues compared to those in the normal liver tissues. Wnt11 mRNA expression was downregulated in HCC tissues. Inhibition of Wnt3 by anti-Wnt3 Ab or siRNA resulted in a decrease of the canonical Wnt signaling pathway and diminished wound healing, while Wnt3 stimulation increased the Tcf transcriptional activity in Focus cells. These findings suggest that Wnt3 is the natural Wnt ligand related to overexpression of FZD7 and activation of the canonical Wnt signaling pathway without β-catenin mutations during hepatocarcinogenesis.

### REFERENCES

1. Davila, J.A., Morgan, R.O., Shaib, Y., McGlynn, K.A., and El-Serag, H.B. 2004. Hepatitis C infection and the increasing incidence of hepatocellular carcinoma: a population-based study. Gastroenterology 127:1372-1380.
2. Du, S.J., Purcell, S.M., Christian, J.L., McGrew, L.L., and Moon, R.T. 1995. Identification of distinct classes and functional domains of Wnts through expression of wild-type and chimeric proteins in Xenopus embryos. Mol Cell Biol 15:2625-2634.
3. Liang, H., Chen, Q., Coles, A.H., Anderson, S.J., Pihan, G., Bradley, A., Gerstein, R., Jurecic, R., and Jones, S.N. 2003. Wnt5a inhibits B cell proliferation and functions as a tumor suppressor in hematopoietic tissue. Cancer Cell 4:349-360.
4. Maye, P., Zheng, J., Li, L., and Wu, D. 2004. Multiple mechanisms for Wnt11-mediated repression of the canonical Wnt signaling pathway. J Biol Chem 279:24659-24665.
5. Iozzo, R.V., Eichstetter, I., and Danielson, K.G. 1995. Aberrant expression of the growth factor Wnt-5A in human malignancy. Cancer Res 55:3495-3499.
6. Lejeune, S., Huguet, E.L., Hamby, A., Poulsom, R., and Harris, A.L. 1995. Wnt5a cloning, expression, and up-regulation in human primary breast cancers. Clin Cancer Res 1:215-222.
7. Weeraratna, A.T., Jiang, Y., Hostetter, G., Rosenblatt, K., Duray, P., Bittner, M., and Trent, J.M. 2002. Wnt5a signaling directly affects cell motility and invasion of metastatic melanoma. Cancer Cell 1:279-288.
8. Kinzler, K.W., and Vogelstein, B. 1996. Lessons from hereditary colorectal cancer. Cell 87:159-170.
9. Devereux, T.R., Stem, M.C., Flake, G.P., Yu, M.C., Zhang, Z.Q., London, S.J., and Taylor, J.A. 2001. CTNNB1 mutations and beta-catenin protein accumulation in human hepatocellular carcinomas associated with high exposure to aflatoxin B1. Mol Carcinog 31:68-73.
10. Hsu, H.C., Jeng, Y.M., Mao, T.L., Chu, J.S., Lai, P.L., and Peng, S.Y. 2000. Beta-catenin mutations are associated with a subset of low-stage hepatocellular carcinoma negative for hepatitis B virus and with favorable prognosis. Am J Pathol 157:763-770.
11. Wong, C.M., Fan, S.T., and Ng, I.O. 2001. beta-Catenin mutation and overexpression in hepatocellular carcinoma: clinicopathologic and prognostic significance. Cancer 92:136-145.
12. Huang, H., Fujii, H., Sankila, A., Mahler-Araujo, B.M., Matsuda, M., Cathomas, G., and Ohgaki, H. 1999. Beta-catenin mutations are frequent in human hepatocellular carcinomas associated with hepatitis C virus infection. Am J Pathol 155:1795-1801.
13. Laurent-Puig, P., Legoix, P., Bluteau, O., Belghiti, J., Franco, D., Binot, F., Monges, G., Thomas, G., Bioulac-Sage, P., and Zucman-Rossi, J. 2001. Genetic alterations associated with hepatocellular carcinomas define distinct pathways of hepatocarcinogenesis. Gastroenterology 120:1763-1773.
14. Satoh, S., Daigo, Y., Furukawa, Y., Kato, T., Miwa, N., Nishiwaki, T., Kawasoe, T., Ishiguro, H., Fujita, M., Tokino, T., et al. 2000. AXIN1 mutations in hepatocellular carcinomas, and growth suppression in cancer cells by virus-mediated transfer of AXIN1. Nat Genet 24:245-250.
15. Merle, P., de la Monte, S., Kim, M., Herrmann, M., Tanaka, S., Von Dem Bussche, A., Kew, M.C., Trepo, C., and Wands, J.R. 2004. Functional consequences of frizzled-7 receptor overexpression in human hepatocellular carcinoma. Gastroenterology 127:1110-1122.
16. Merle, P., Kim, M., Herrmann, M., Gupte, A., Lefrancois, L., Califano, S., Trepo, C., Tanaka, S., Vitvitski, L., Monte, S.D., et al. 2005. Oncogenic role of the frizzled-7/beta-catenin pathway in hepatocellular carcinoma. J Hepatol.
17. Lin, X., and Perrimon, N. 1999. Dally cooperates with Drosophila Frizzled 2 to transduce Wingless signalling. Nature 400:281-284.
18. Willert, K, Brown, J.D., Danenberg, E., Duncan, A.W., Weissman, I.L., Reya, T., Yates, J.R, 3rd, and Nusse, R 2003. Wnt proteins are lipid-modified and can act as stem cell growth factors. Nature 423:448-452.
19. Bradley, R.S., and Brown, A.M. 1990. The proto-oncogene int-1 encodes a secreted protein associated with the extracellular matrix. Embo J 9:1569-1575.
20. Reichsman, F., Smith, L., and Cumberledge, S. 1996. Glycosaminoglycans can modulate extracellular localization of the wingless protein and promote signal transduction. J Cell Biol 135:819-827.
21. Dhoot, G.K, Gustafsson, M.K., Ai, X., Sun, W., Standiford, D.M., and Emerson, C.P., Jr. 2001. Regulation of Wnt signaling and embryo patterning by an extracellular sulfatase. Science 293:1663-1666.
22. Roelink, H., Wang, J., Black, D.M., Solomon, E., and Nusse, R 1993. Molecular cloning and chromosomal localization to 17q21 of the human WNT3 gene. Genomics 17:790-792.
23. Shimizu, H., Julius, M.A., Giarre, M., Zheng, Z., Brown, A.M., and Kitajewski, J. 1997. Transformation by Wnt family proteins correlates with regulation of beta-catenin. Cell Growth Differ 8:1349-1358.
24. Katoh, M. 2001. Molecular cloning and characterization of human WNT3. Int J Oncol 19:977-982.
25. Gregorieff, A., Pinto, D., Begthel, H., Destree, O., Kielman, M., and Clevers, H. 2005. Expression pattern of Wnt signaling components in the adult intestine. Gastroenterology 129:626-638.
26. Kirikoshi, H., Sekihara, H., and Katoh, M. 2001. Molecular cloning and characterization of human WNT11. Int J Mol Med 8:651-656.
27. Zhu, H., Mazor, M., Kawano, Y., Walker, M.M., Leung, H.Y., Armstrong, K., Waxman, J., and Kypta, R.M. 2004. Analysis of Wnt gene expression in prostate cancer: mutual inhibition by WNT11 and the androgen receptor. Cancer Res 64:7918-7926.
28. Smolich, B.D., McMahon, J.A., McMahon, A.P., and Papkoff, J. 1993. Wnt family proteins are secreted and associated with the cell surface. Mol Biol Cell 4:1267-1275.
29. Cha, M.Y., Kim, C.M., Park, Y.M., and Ryu, W.S. 2004. Hepatitis B virus X protein is essential for the activation of Wnt/beta-catenin signaling in hepatoma cells. Hepatology 39:1683-1693.
30. Veeman, M.T., Slusarski, D.C., Kaykas, A., Louie, S.H., and Moon, R.T. 2003. Zebrafish prickle, a modulator of noncanonical Wnt/Fz signaling, regulates gastrulation movements. Curr Biol 13:680-685.
31. Carruba, G., Cervello, M., Miceli, M.D., Farruggio, R., Notarbartolo, M., Virruso, L., Giannitrapani, L., Gambino, R, Montalto, G., and Castagnetta, L. 1999. Truncated form of beta-catenin and reduced expression of wild-type catenins feature HepG2 human liver cancer cells. Ann N Y Acad Sci 886:212-216.
32. Korinek, V., Barker, N., Morin, P.J., van Wichen, D., de Weger, R., Kinzler, K.W., Vogelstein, B., and Clevers, H. 1997. Constitutive transcriptional activation by a beta-catenin-Tcf complex in APC-/- colon carcinoma. Science 275:1784-1787.

**Table 1. Primer Pairs Used for the Detection of Wnt Ligand mRNA**

| **Wnt** | **Sequence** | **SEQ ID NO** |
|---|---|---|
| GAPDH | Sense: 5'-GAAATCCCATCACCATCTTCCAG-3' | 28 |
| | Anti-sense: 5'-ATGAGTCCTTCCACGATACCAAAG-3' | 29 |
| Wnt1 | Sense: 5'-TGTTGCCTGGCTGGGTTTC-3' | 30 |
| | Anti-sense: 5'-CTGTAAGCAGGTTCGTGGAG-3' | 31 |
| Wnt2 | Sense: 5'-GTGGATGCAAAGGAAAGGAA-3' | 32 |
| | Anti-sense: 5'-AGCCAGCATGTCCTGAGAGT-3' | 33 |
| Wnt2b | Sense: 5'-ACCCAAGATGGTGCCAACTTC-3' | 34 |
| | Anti-sense: 5'-CACAACCGTCTGTTCCTTTTGATG-3' | 35 |
| Wnt3 | Sense: 5'-GGAGTGTATTCGCATCTACGACG-3' | 36 |
| | Anti-sense: 5'-CGAGTTGGGTCTGGGTCATTTAC-3' | 37 |
| Wnt3a | Sense: 5'-CCCCACTCGGATACTTCTTACTCC-3' | 38 |
| | Anti-sense: 5'-CTCCTGGATGCCAATCTTGATG-3' | 39 |
| Wnt4 | Sense: 5'-TTTGTGGATGTGCGGGAGAG-3' | 40 |
| | Anti-sense: 5'-ATCTGTGTGCGGCTTGAACTG-3' | 41 |
| Wnt5a | Sense: 5'-ACACCTCTTTCCAAACAGGCC-3' | 42 |
| | Anti-sense: 5'-GGATTGTTAAACTCAACTCTC-3' | 43 |
| Wnt5b | Sense: 5'-GGAGCGAGAGAAGAACTTTGCC-3' | 44 |
| | Anti-sense: 5'-GAAGCAGCACCAGTGGAACTTG-3' | 45 |
| Wnt6 | Sense: 5'-CTTGGTTATGGACCCTACCCAGGCATC-3' | 46 |
| | Anti-sense: 5'-CACTGCAGCAGCTCGCCCATAGAA-3' | 47 |
| Wnt7a | Sense: 5'-GCTGCCTGGGCCACCTCTTTCTCA-3' | 48 |
| | Anti-sense: 5'-CCCGGTGGTACAGGCCTTGCTTCT-3' | 49 |
| Wnt7b | Sense: 5'-TCAACGAGTGCCAGTACCAG-3' | 50 |
| | Anti-sense: 5'-CCCTCGGCTTGGTTGTAGTA-3' | 51 |
| Wnt8a | Sense: 5'-TCCAGTTTGCTTGGGAACGC-3' | 52 |
| | Anti-sense: 5'-CCATCACAGCCACAGTTTTCG-3' | 53 |
| Wnt8b | Sense: 5'-CATCTGTCTTTTCACCTGTGTCCTC-3' | 54 |
| | Anti-sense: 5'-AATGCTGTCTCCCGATTGGC-3' | 55 |
| Wnt10a | Sense: 5'-TCTGGGTGCTCCTGTTCTTCCTAC-3' | 56 |
| | Anti-sense: 5'-ATTGGTGTTGGCATTCGTGG-3' | 57 |
| Wnt10b | Sense: 5'-ACTGTCCCGAGGCAAGAGTTTC-3' | 58 |
| | Anti-sense: 5'-GCATTTCCGCTTCAGGTTTTC-3' | 59 |
| Wnt11 | Sense: 3'-TGCTGACCTCAAGACCCGATAC-3' | 60 |
| | Anti-sense: 3'-TGTCGCTTCCGTTGGATGTC-3' | 61 |
| Wnt14 | Sense: 5'-TGCCAGTTCCAGTTCCGCTTTG-3' | 62 |
| | Anti-sense: 5'-TTCACACCCACGAGGTTGTTG-3' | 63 |
| Wnt15 | Sense: 5'-TGAGTGCCAGTTTCAGTTCCG-3' | 64 |
| | Anti-sense: 5'-CTTGTTTCCTCTCTTGGACCCC-3' | 65 |
| Wnt16 | Sense: 5'-CTGCTCCGATGATGTCCAGTATG-3' | 66 |
| | Anti-sense: 5'-CATTCTCTGCCTTGTGTCCCTG-3' | 67 |

A number of embodiments of the invention have been described. Nevertheless, it will be understood that various modifications may be made. Accordingly, other embodiments are within the scope of the following claims.

## Claims

1. A method for identifying an anti-liver cancer agent, the method comprising:
selecting a test compound that binds to a polypeptide comprising an amino acid sequence of a Wnt 3 or Wnt 11 protein or a FZD-binding fragment thereof; and
determining whether the test compound is capable of:
(a) reducing Wnt/FZD 7 signaling in a liver cell;
(b) reducing liver cancer cell motility;
(c) reducing β-catenin accumulation in a liver cancer cell; or
(d) treating liver cancer *in vitro* or in a non-human animal *in vivo;*
wherein a test compound that is capable of at least one of (a) to (d) is an anti-liver cancer agent.

2. The method of claim 1, wherein
(a) the selection of the test compound comprises:
contacting the test compound with the polypeptide comprising the amino acid sequence of a Wnt 3 or Wnt 11 protein or a FZD-binding fragment thereof;
detecting binding between the polypeptide and the test compound; and
selecting the test compound if it binds to the polypeptide; and/or
(b) the polypeptide is provided as:
(i) a naturally occurring polypeptide;
(ii) a recombinant polypeptide;
(iii) a polypeptide expressed on the surface of a cell; or
(iv) an isolated polypeptide; and/or
(c) the polypeptide comprises an amino acid sequence of a Wnt3 protein; or
(d) the polypeptide comprises an amino acid sequence of a Wnt3 protein, wherein said amino acid sequence comprises any one of SEQ ID NOs:8 to 12 and at least one non-Wnt sequence, and/or
(e) the polypeptide comprises an amino acid sequence of a Wnt11 protein; or
(f) the polypeptide comprises an amino acid sequence of a Wnt11 protein, wherein the said amino acid sequence comprises any one of SEQ ID NOs: 22 to 26 and at least one non-Wnt sequence.

3. A compound for use in the treatment of liver cancer or for use in reducing the motility of liver cancer in a patient, wherein the compound comprises:
(a) an anti-Wnt3 antibody;
(b) an anti-Wnt3 antibody that binds to a polypeptide comprising the amino acid sequence LRAKYSLFKPPTERDL (SEQ ID NO:80);
(c) an siRNA that reduces expression of Wnt3 in the patient; or
(d) an siRNA that reduces expression of Wnt3 in the patient and comprises a sequence selected from the group consisting of: WNT3-1: 5'-GGAAAAAUGCCACUGCAUC-3' (SEQ ID NO:81), WNT3-2:5'-GGAGUGUAUUCGCAUCUAC-3'(SEQ ID NO:82), and WNT3-3: 5'-GGCUUAUCUUUGCACAUGU-3' (SEQ ID NO:83).

4. A method for identifying a candidate anti-liver cancer agent, the method comprising:
(a) providing a first polypeptide that:
(i) comprises a FZD 7 polypeptide or a fragment thereof; and
(ii) displays Wnt-binding ability;
(b) providing a second polypeptide that:
(i) comprises a Wnt 3 or Wnt 11 polypeptide or a fragment thereof; and
(ii) displays FZD 7 binding ability;
(c) contacting the first and second polypeptides in the presence of a test compound; and
(d) comparing the level of binding between the first and second polypeptides in the presence of the test compound with the level of binding in the absence of the test compound, wherein a reduced level of binding in the presence of the test compound than in its absence indicates that the test compound is a candidate anti-cancer agent, the method optionally further comprising:
(e) determining whether the candidate anti-liver cancer agent is capable of:
(i) reducing Wnt/FZD 7 signaling in a cell that expresses FZD 7;
(ii) reducing liver cancer cell motility;
(iii) reducing β-catenin accumulation in a liver cancer cell; or
(iv) treating liver cancer *in vitro* or in a non-human animal *in vivo;*
wherein a candidate that is capable of at least one of (i) to (iv) is an anti-liver cancer agent.

5. The method of claim 4, wherein
(a) the test compound is selected from the group consisting of polypeptides, ribonucleic acids, small molecules and deoxyribonucleic acids; or
(b) wherein the first polypeptide is a first fusion protein comprising a FZD polypeptide fused to:
(i) a transcription activation domain of a transcription factor or
(ii) a DNA-binding domain of a transcription factor; the second polypeptide is a second fusion protein comprising a Wnt polypeptide fused to
(iii) a transcription activation domain of a transcription factor or
(iv) a DNA-binding domain of a transcription factor, wherein the Wnt polypeptide is fused to a domain different from that fused to the FZD polypeptide; and binding of the first and second polypeptides is detected as reconstitution of a transcription factor; or
(c) wherein the second polypeptide comprises a Wnt3 polypeptide or fragment thereof; or
(d) wherein the second polypeptide comprises any one of SEQ ID NOs:8 to 12 and at least one non-Wnt sequence; or
(e) wherein the second polypeptide comprises a Wnt11 polypeptide or fragment thereof; or
(f) wherein the second polypeptide comprises any one of SEQ ID NOs: 22 to 26 and at least one non-Wnt sequence.

6. A method of determining whether a liver cell is, or is at risk for becoming, a cancer cell, the method comprising:
(a) providing a test liver cell obtained from a patient;
(b) determining whether the cell's level of Wnt3 expression is higher than that of a control cell; and
(c) classifying the test cell as a cancer cell or at risk for becoming a cancer cell, if the test cell's level of Wnt3 expression is higher than that of the control cell.

7. A method of determining whether a test tissue sample comes from a patient that is suffering from or at risk for liver cancer, the method comprising:
(a) providing a liver test tissue sample obtained from the patient, including a sample that is tumorous tissue or peritumorous tissue; and
(b) determining whether the level of Wnt3 expression in the test tissue sample is higher than that in a comparable tissue sample obtained from a healthy individual, wherein a higher level of expression in the test tissue sample is an indication that the patient is suffering from or is at risk for cancer; and optionally further comprising
(c) determining whether the level of Wnt11 expression in the test tissue sample is lower than that in a tissue sample obtained from a healthy individual, wherein a lower level of expression of Wnt11 is an indication that the patient is suffering from or at risk for cancer.

8. The method of claim 6 or 7:
(a) wherein determining the level of Wnt3 expression includes determining the amount of Wnt3 mRNA in the test cell or test tissue sample; or
(b) wherein determining the level of Wnt3 expression includes determining the amount of Wnt3 mRNA in the test cell or tissue sample using a Northern blot assay or an RT-PCR assay; or
(c) wherein determining the level of Wnt3 expression includes determining the amount of Wnt3 protein in the test cell or test tissue sample; or
(d) wherein determining the level of Wnt3 expression includes determining the amount of Wnt3 protein in the test cell or tissue sample using an antibody, including an antibody that binds to the amino acid sequence LRAKYSLFKPPTERDL (SEQ ID NO:80); or
(e) further comprising determining whether the test cell's or test tissue's level of Wnt11 expression is lower than that of a control cell or control tissue, wherein a lower level of expression of Wnt11 indicates that
(i) the test cell is, or is at risk for becoming, a cancer cell or
(ii) the patient is suffering from or is at risk for cancer.

9. An antibody that binds to the amino acid sequence LRAKYSLFKPPTERDL (SEQ ID NO:80).

10. A pharmaceutical composition comprising the antibody of claim 9 and a pharmaceutically acceptable carrier.

11. The antibody of claim 9 for use in therapy.

12. The antibody of claim 9 for use for treating liver cancer.

13. A composition comprising an siRNA comprising a sequence selected from the group consisting of: WNT3-1: 5'-GGAAAAAUGCCACUGCAUC-3' (SEQ ID NO:81), WNT3-2: 5'-GGAGUGUAUUCGCAUCUAC-3' (SEQ ID NO:82), and WNT3-3: 5'-GGCUUAUCUUUGCACAUGU-3' (SEQ ID NO:83) for use in treating liver cancer.

14. A composition comprising a Wnt11 polypeptide or a genetic construct encoding a Wnt11 polypeptide for use for treating liver cancer.

15. The composition of claim 14, wherein the composition is administered directly to the liver of the patient *in situ.*

16. The antibody of claim 9, wherein the antibody is a monoclonal antibody, a polyclonal antibody, a chimeric antibody, a humanized antibody, a single chain antibody, a Fab fragment, or a F(ab')₂ fragment.

## Patentansprüche

1. Verfahren zur Identifizierung eines Antileberkrebsmittels, wobei das Verfahren folgendes umfasst:
Auswählen einer Testverbindung, die an ein Polypeptid bindet, welches eine Aminosäuresequenz von einem Wnt 3- oder Wnt 11-Protein oder einem FZD-bindenden Fragment davon umfasst; und
Bestimmen, ob die Testverbindung zu folgendem in der Lage ist:
(a) Herabsetzen der Wnt/FZD 7-Signalgebung in einer Leberzelle;
(b) Herabsetzen der Leberkrebszellmotilität;
(c) Herabsetzen der β-Catenin-Akkumulation in einer Leberkrebszelle; oder
(d) Behandeln von Leberkrebs *in vitro* oder in einem nichtmenschlichen Tier *in vivo;*
wobei eine Testverbindung, die zumindest zu einem von (a) bis (d) in der Lage ist, ein Mittel gegen Leberkrebs ist.

2. Verfahren nach Anspruch 1, wobei
(a) die Auswahl der Testverbindung folgendes umfasst:
Kontaktieren der Testverbindung mit dem Polypeptid, welches die Aminosäuresequenz von einem Wnt 3- oder Wnt 11-Protein oder von einem FZD-bindenden Fragment davon umfasst; Nachweisen der Bindung zwischen dem Polypeptid und der Testverbindung; und
Auswählen der Testverbindung, wenn sie an das Polypeptid bindet; und/oder
(b) das Polypeptid bereitgestellt wird als:
(i) ein natürlich vorkommendes Polypeptid;
(ii) ein rekombinantes Polypeptid;
(iii) ein auf der Oberfläche einer Zelle exprimiertes Polypeptid; oder
(iv) ein isoliertes Polypeptid; und/oder
(c) das Polypeptid eine Aminosäuresequenz eines Wnt3-Proteins umfasst; oder
(d) das Polypeptid eine Aminosäuresequenz eines Wnt3-Proteins umfasst, wobei die Aminosäuresequenz eine von SEQ ID NO: 8 bis 12 und mindestens eine Nicht-Wnt-Sequenz umfasst, und/oder
(e) das Polypeptid eine Aminosäuresequenz eines Wnt11-Proteins umfasst; oder
(f) das Polypeptid eine Aminosäuresequenz eines Wnt11-Proteins umfasst, wobei die Aminosäuresequenz eine von SEQ ID NO: 22 bis 26 und mindestens eine Nicht-Wnt-Sequenz umfasst.

3. Verbindung zur Verwendung bei der Behandlung von Leberkrebs oder zur Verwendung bei der Herabsetzung der Motilität von Leberkrebs in einem Patienten, wobei die Verbindung umfasst:
(a) einen anti-Wnt3-Antikörper;
(b) einen anti-Wnt3-Antikörper, der an ein Polypeptid bindet, das die Aminosäuresequenz LRAKYSLFKPPTERDL (SEQ ID NO: 80) umfasst;
(c) eine siRNA, die die Wnt3-Expression in dem Patienten herabsetzt; oder
(d) eine siRNA, die die Wnt3-Expression in dem Patienten herabsetzt und eine Sequenz einschließt, die aus der Gruppe ausgewählt ist bestehend aus:
WNT3-1: 5'-GGAAAAAUGCCACUGCAUC-3' (SEQ ID NO: 81),
WNT3-2: 5'-GGAGUGUAUUCGCAUCUAC-3' (SEQ ID NO: 82) und
WNT3-3 : 5'-GGCUUAUCUUUGCACAUGU-3' (SEQ ID NO: 83).

4. Verfahren zur Identifizierung eines Antileberkrebsmittel-Kandidaten, wobei das Verfahren folgendes umfasst:
(a) Bereitstellen eines ersten Polypeptids, das:
(i) ein FZD 7-Polypeptid oder ein Fragment davon umfasst; und
(ii) die Fähigkeit des Bindens an Wnt zeigt;
(b) Bereitstellen eines zweiten Polypeptids, das:
(i) ein Wnt 3- oder Wnt 11-Polypeptid oder ein Fragment davon umfasst; und
(ii) die Fähigkeit des Bindens an FZD 7 zeigt;
(c) Kontaktieren des ersten und zweiten Polypeptids in Gegenwart einer Testverbindung; und
(d) Vergleichen des Bindungsniveaus zwischen dem ersten und dem zweiten Polypeptid in Gegenwart der Testverbindung mit dem Bindungsniveau in Abwesenheit der Testverbindung,
wobei ein in Gegenwart der Testverbindung kleineres Bindungsniveau als in ihrer Abwesenheit angibt, dass die Testverbindung ein Antileberkrebsmittel-Kandidat ist, wobei das Verfahren gegebenenfalls weiterhin folgendes umfasst:
(e) Bestimmen, ob der Antileberkrebsmittel-Kandidat zu folgendem in der Lage ist:
(i) Herabsetzen der Wnt/FZD 7-Signalgebung in einer Zelle, die FZD 7 exprimiert;
(ii) Herabsetzen der Leberkrebszellmotilität;
(iii) Herabsetzen der Akkumulation von β-Catenin in einer Leberkrebszelle; oder
(iv) Behandeln von Leberkrebs *in vitro* oder in einem nichtmenschlichen Tier *in vivo;*
wobei ein Kandidat, der zu mindestens einem von (i) bis (iv) in der Lage ist, ein Mittel gegen Leberkrebs ist.

5. Verfahren nach Anspruch 4, wobei:
(a) die Testverbindung aus der Gruppe ausgewählt ist bestehend aus Polypeptiden, Ribonukleinsäuren, kleinen Molekülen und Desoxyribonukleinsäuren; oder
(b) wobei das erste Polypeptid ein erstes Fusionsprotein ist, das ein FZD-Polypeptid umfasst, das mit folgendem fusioniert ist:
(i) einer Transkriptionsaktivierungsdomäne eines Transkriptionsfaktors oder
(ii) einer DNA-Bindungsdomäne eines Transkriptionsfaktors; wobei das zweite Polypeptid ein zweites Fusionsprotein ist, das ein Wnt-Polypeptid umfasst, das an folgendes fusioniert ist:
(iii) eine Transkriptionsaktivierungsdomäne eines Transkriptionsfaktors oder
(iv) eine DNA-Bindungsdomäne eines Transkriptionsfaktors, wobei das Wnt-Polypeptid an eine Domäne fusioniert ist, die von derjenigen verschieden ist, die an das FZD-Polypeptid fusioniert ist; und das Binden des ersten und zweiten Polypeptids als Rekonstitution eines Transkriptionsfaktors nachgewiesen wird; oder
(c) wobei das zweite Polypeptid ein Wnt3-Polypeptid oder ein Fragment davon umfasst; oder
(d) wobei das zweite Polypeptid eine von SEQ ID NO: 8 bis 12 und mindestens eine Nicht-Wnt-Sequenz umfasst; oder
(e) wobei das zweite Polypeptid ein Wnt11-Polypeptid oder ein Fragment davon umfasst; oder
(f) wobei das zweite Polypeptid eine von SEQ ID NO: 22 bis 26 und mindestens eine Nicht-Wnt-Sequenz umfasst.

6. Verfahren zur Bestimmung, ob eine Leberzelle eine Krebszelle ist, oder ob bei ihr das Risiko besteht, zu einer Krebszelle zu werden, wobei das Verfahren folgendes umfasst:
(a) Bereitstellen einer Testleberzelle, die von einem Patienten erhalten wurde;
(b) Bestimmen, ob das Wnt3-Expressionsniveau der Zelle höher ist als dasjenige in einer Kontrollzelle; und
(c) Klassifizieren der Testzelle als eine Krebszelle oder als solche mit dem Risiko, zu einer Krebszelle zu werden, wenn das Wnt3-Expressionsniveau der Testzelle höher ist als dasjenige der Kontrollzelle.

7. Verfahren zur Bestimmung, ob eine Testgewebeprobe von einem Patienten stammt, der an Leberkrebs leidet oder bei dem das Risiko dafür besteht, wobei das Verfahren folgendes umfasst:
(a) Bereitstellen einer von dem Patienten erhaltenen Leber-Testgewebeprobe, einschließlich einer Probe, die ein Tumor- oder Peritumorgewebe ist; und
(b) Bestimmen, ob das Wnt3-Expressionsniveau in der Testgewebeprobe höher ist als dasjenige in einer vergleichbaren Gewebeprobe, die von einem gesunden Individuum erhalten wurde, wobei ein höheres Expressionsniveau in der Testgewebeprobe ein Anzeichen dafür ist, dass der Patient an Krebs leidet oder bei ihm das Risiko dafür besteht; und gegebenenfalls weiterhin folgendes umfassend:
(c) Bestimmen, ob das Wnt11-Expressionsniveau in der Testgewebeprobe geringer ist als dasjenige in einer von einem gesunden Individuum erhaltenen Gewebeprobe, wobei ein geringeres Wnt11-Expressionsniveau ein Hinweis darauf ist, dass der Patient an Krebs leidet, oder dass bei ihm das Risiko dafür besteht.

8. Verfahren nach Anspruch 6 oder 7:
(a) wobei das Bestimmen des Wnt3-Expressionsniveaus die Bestimmung der Menge von Wnt3-mRNA in der Testzelle oder in der Testgewebeprobe umfasst; oder
(b) wobei das Bestimmen des Wnt3-Expressionsniveaus das Bestimmen der Menge an Wnt3-mRNA in der Testzelle oder in der Gewebeprobe unter Verwendung eines Northern Blot- oder RT-PCR-Tests umfasst; oder
(c) wobei das Bestimmen des Wnt3-Expressionsniveaus das Bestimmen der Menge an Wnt3-Protein in der Testzelle oder in der Testgewebeprobe umfasst; oder
(d) wobei das Bestimmen des Wnt3-Expressionsniveaus das Bestimmen der Menge an Wnt3-Protein in der Testzelle oder in der Gewebeprobe unter Verwendung eines Antikörpers, einschließlich eines Antikörpers, der an die Aminosäuresequenz LRAKYSLFKPPTERDL (SEQ ID NO: 80) bindet, umfasst; oder
(e) weiterhin umfassend das Bestimmen, ob das Wnt11-Expressionsniveau der Testzelle oder des Testgewebes geringer ist als dasjenige einer Kontrollzelle oder eines Kontrollgewebes, wobei ein geringeres Wnt11-Expressionsniveau angibt, dass
(i) die Testzelle eine Krebszelle ist, oder dass bei ihr das Risiko besteht, zu einer Krebszelle zu werden, oder
(ii) der Patient an Krebs leidet, oder dass bei ihm das Risiko für Krebs besteht.

9. Antikörper, der an die Aminosäuresequenz LRAKYSLFKPPTERDL (SEQ ID NO: 80) bindet.

10. Pharmazeutische Zusammensetzung, umfassend den Antikörper nach Anspruch 9 und einen pharmazeutisch verträglichen Träger.

11. Antikörper nach Anspruch 9 zur Verwendung in der Therapie.

12. Antikörper nach Anspruch 9 zur Behandlung von Leberkrebs.

13. Zusammensetzung umfassend eine siRNA umfassend eine Sequenz, die aus der Gruppe ausgewählt ist bestehend aus: WNT3-1: 5'-GGAAAAAUGCCACUGCAUC-3' (SEQ ID NO: 81), WNT3-2: 5'-GGAGUGUAUUCGCAUCUAC-3' (SEQ ID NO: 82), und WNT3-3: 5'-GGCUUAUCUUUGCACAUGU-3' (SEQ ID NO: 83) zur Verwendung bei der Behandlung von Leberkrebs.

14. Zusammensetzung umfassend ein Wnt11-Polypeptid oder ein genetisches Konstrukt, welches ein Wnt11-Polypeptid kodiert, zur Verwendung bei der Behandlung von Leberkrebs.

15. Zusammensetzung nach Anspruch 14, wobei die Zusammensetzung direkt an die Leber des Patienten *in situ* verabreicht wird.

16. Antikörper nach Anspruch 9, wobei der Antikörper ein monoklonaler Antikörper, ein polyklonaler Antikörper, ein chimärer Antikörper, ein humanisierter Antikörper, ein einzelkettiger Antikörper, ein Fab-Fragment, oder ein F(ab')₂-Fragment ist.

## Revendications

1. Méthode d'identification d'un agent anti-cancer hépatique, la méthode comprenant les étapes suivantes:
sélectionner un composé d'essai qui se lie à un polypéptide comprenant une séquence d'acides aminés d'une protéine Wnt 3 ou Wnt 11 ou d'un fragment de celles-ci se liant au FZD; et
déterminer si le composé d'essai est capable de:
(a) réduire la Wnt/FZD 7 signalant dans une cellule hépatique;
(b) réduire la motilité de cellule de cancer hépatique;
(c) réduire l'accumulation de β-caténine dans une cellule de cancer hépatique; ou
(d) traiter le cancer hépatique *in vitro* ou dans un animal non-humain *in vivo;*
dans laquelle un composé d'essai qui est capable pour le moins de l'un de (a) à (d) est un agent anti-cancer hépatique.

2. Méthode selon la revendication 1, dans laquelle
(a) la sélection du composé d'essai comprend les étapes consistant à:
contacter le composé d'essai avec le polypeptide comprenant la séquence d'acides aminés d'une protéine Wnt 3 ou Wnt 11 ou d'un fragment de celles-ci se liant au FZD; détecter la liaison entre le polypeptide et le composé d'essai; et
sélectionner le composé d'essai s'il se lie au polypeptide; et/ou
(b) le polypeptide est fourni à titre:
(i) d'un polypeptide existant naturellement;
(ii) d'un polypeptide recombinant;
(iii) d'un polypeptide exprimé sur la surface d'une cellule; ou
(iv) d'un polypeptide isole; et/ou
(c) le polypeptide comprend une séquence d'acides aminés d'une protéine Wnt3; ou
(d) le polypeptide comprend une séquence d'acides aminés d'une protéine Wnt3, dans laquelle ladite séquence d'acides aminés comprend l'une quelconque de SEQ ID NOs: 8 à 12 et au moins une séquence non-Wnt, et/ou
(e) le polypeptide comprend une séquence d'acides aminés d'une protéine Wnt11; ou
(f) le polypéptide comprend une séquence d'acides aminés d'une protéine Wnt11, dans laquelle ladite séquence d'acides aminés comprend l'une quelconque de SEQ ID NOs: 22 à 26 et au moins une séquence non-Wnt.

3. Composé pour l'utilisation dans le traitement du cancer hépatique ou pour l'utilisation dans la réduction de la motilité de cancer hépatique chez un patient, dans lequel le composé comprend:
(a) un anticorps anti-Wnt3;
(b) un anticorps anti-Wnt3 qui se lie à un polypéptide comprenant la séquence d'acides aminés LRAKYSLFKPPTERDL (SEQ ID NO: 80);
(c) un siARN qui réduit l'expression de Wnt3 chez le patient; ou
(d) un siARN qui réduit l'expression de Wnt3 chez le patient et comprend une séquence sélectionnée parmi le groupe consistant en:
WNT3-1: 5'-GGAAAAAUGCCACUGCAUC-3' (SEQ ID NO: 81),
WNT3-2: 5'-GGAGUGUAUUCGCAUCUAC-3' (SEQ ID NO: 82) et
WNT3-3: 5'-GGCUUAUCUUUGCACAUGU-3' (SEQ ID NO: 83).

4. Méthode d'identification d'un agent anti-cancer hépatique candidate, la méthode comprenant les étapes consistant à:
(a) fournir un premier polypéptide qui:
(i) comprend un polypeptide FZD 7 ou un fragment de de celui-ci; et
(ii) présente la capacité de se lier à Wnt;
(b) fournir un deuxième polypeptide qui:
(i) comprend un polypeptide Wnt 3 ou Wnt 11 ou un fragment de ceux-ci; et
(ii) présente la capacité de se lier à FZD 7;
(c) contacter le premier et le deuxième polypeptide en présence d'un composé d'essai; et
(d) comparer le niveau de liaison entre le premier et le deuxième polypéptide en présence du composé d'essai avec le niveau de liaison en absence du composé d'essai,
dans laquelle un niveau de liaison réduit en présence du composé d'essai qu'en son absence indique que le composé d'essai est un agent anti-cancer hépatique candidate, la méthode, en cas échéant, comprenant en outre l'étape consistant à:
(e) déterminer si l'agent anti-cancer hépatique candidate est capable de:
(i) réduire la signalisation Wnt/FZD 7 dans une cellule qui exprime le FZD 7;
(ii) réduire la motilité de cellule de cancer hépatique;
(iii) réduire l'accumulation de β-caténine dans une cellule de cancer hépatique; ou
(iv) traiter le cancer hépatique *in vitro* ou dans un animal non-humain *in vivo;*
dans laquelle un candidate qui est capable pour le moins de l'un de (i) à (iv) est un agent anti-cancer hépatique.

5. Méthode selon la revendication 4, dans laquelle:
(a) le composé d'essai est sélectionné parmi le groupe consistant en les polypeptides, les acides ribonucléiques, les petites molécules et les acides désoxyribonucléiques; ou
(b) dans laquelle le premier polypeptide est une première protéine de fusion comprenant un polypeptide FZD fusionné à:
(i) un domaine d'activation de la transcription d'un facteur de transcription ou
(ii) un domaine de liaison à l'ADN d'un facteur de transcription; le deuxième polypeptide est une deuxième protéine de fusion comprenant un polypeptide Wnt fusionné à
(iii) un domaine d'activation de transcription d'un facteur de transcription ou
(iv) un domaine de liaison à l'ADN d'un facteur de transcription, dans laquelle le polypeptide Wnt est fusionné à un domaine qui est différent à celui qui est fusionné au polypeptide FZD; et la liaison du premier et deuxième polypeptide est détectée en tant que reconstitution d'un facteur de transcription; ou
(c) dans laquelle le deuxième polypeptide comprend un polypeptide Wnt3 ou un fragment de celui-ci;
ou
(d) dans laquelle le deuxième polypeptide comprend l'une quelconque de SEQ ID NOs: 8 à 12 et au moins une séquence non-Wnt; ou
(e) dans laquelle le deuxième polypeptide comprend un polypeptide Wnt11 ou un fragment de celui-ci;
ou
(f) dans laquelle le deuxième polypeptide comprend l'une quelconque de SEQ ID NOs: 22 à 26 et au moins une séquence non-Wnt.

6. Méthode de détermination si une cellule hépatique est, ou subit le risque de devenir, une cellule cancéreuse, la méthode comprenant les étapes consistant à:
(a) fournir une cellule d'essai hépatique obtenu d'un patient;
(b) déterminer si le niveau d'expression de Wnt3 de la cellule est plus élevé que celui dans une cellule de contrôle; et
(c) classifier la cellule d'essai en tant qu'une cellule de cancer ou comme subir le risque de devenir une cellule de cancer, si le niveau d'expression de Wnt3 de la cellule d'essai est plus élevé que celui de la cellule de contrôle.

7. Méthode de détermination si un échantillon de tissu d'essai vient d'un patient qui souffre ou subit le risque de cancer hépatique, la méthode comprenant les étapes consistant à:
(a) fournir un échantillon de tissu hépatique d'essai obtenu du patient, incluant un échantillon qui est un tissu tumoral ou peritumoral; et
(b) déterminer si le niveau d'expression de Wnt3 dans l'échantillon de tissu d'essai est plus élevé que celui dans un échantillon de tissu comparable obtenu d'un individu en bonne santé, dans laquelle un niveau d'expression plus élevé dans l'échantillon de tissu d'essai est une indication que le patient souffre ou subit le risque de cancer; et en cas échéant comprenant en outre l'étape consistant à
(c) déterminer si le niveau d'expression de Wnt11 dans l'échantillon de tissu d'essai est plus faible que celui dans un échantillon de tissu obtenu d'un individu en bonne santé, dans laquelle un niveau d'expression de Wnt11 plus faible est une indication que le patient souffre ou subit le risque de cancer.

8. Méthode selon la revendication 6 ou 7:
(a) dans laquelle la détermination du niveau d'expression de Wnt3 comprend la détermination du niveau de Wnt3 mARN dans la cellule d'essai ou dans l'échantillon de tissu d'essai; ou
(b) dans laquelle la détermination du niveau d'expression de Wnt3 comprend la détermination du niveau de Wnt3 mARN dans la cellule d'essai ou dans l'échantillon de tissu en utilisant un essai de Northern Blot ou un essai RT-PCR; ou
(c) dans laquelle la détermination du niveau d'expression de Wnt3 comprend la détermination du niveau de protéine Wnt3 dans la cellule d'essai ou dans l'échantillon de tissu d'essai; ou
(d) dans laquelle la détermination du niveau d'expression de Wnt3 comprend la détermination du niveau de Wnt3 mARN dans la cellule d'essai ou dans l'échantillon de tissu en utilisant un anticorps, incluant un anticorps qui se lie à la séquence d'acides aminés LRAKYSLFKPPTERDL (SEQ ID NO: 80); ou
(e) comprenant en outre la détermination si le niveau d'expression de Wnt11 de la cellule d'essai ou du tissu d'essai est plus faible que celui d'une cellule de contrôle ou d'un tissu de contrôle, dans laquelle un niveau d'expression plus faible de Wnt11 indique que
(i) la cellule d'essai est une cellule de cancer ou subit le risque de devenir une cellule de cancer ou
(ii) le patient souffre ou subit le risque du cancer.

9. Anticorps qui se lie à la séquence d'acides aminés LRAKYSLFKPPTERDL (SEQ ID NO: 80).

10. Composition pharmaceutique comprenant l'anticorps selon la revendication 9 et un véhicule pharmaceutiquement acceptable.

11. Anticorps selon la revendication 9 pour l'utilisation en thérapie.

12. Anticorps selon la revendication 9 pour le traitement du cancer hépatique.

13. Composition comprenant un siARN comprenant une séquence sélectionnée parmi le groupe consistant en: WNT3-1: 5'-GGAAAAAUGCCACUGCAUC-3' (SEQ ID NO: 81), WNT3-2: 5'-GGAGUGUAUUCGCAUCUAC-3'(SEQ ID NO: 82), et WNT3-3: 5'-GGCUUAUCUUUGCACAUGU-3'(SEQ ID NO: 83) pour l'utilisation dans le traitement du cancer hépatique.

14. Composition comprenant un polypeptide Wnt11 ou une construction génétique codant un polypéptide Wnt11 pour l'utilisation pour le traitement du cancer hépatique.

15. Composition selon la revendication 14, dans lequel la composition est administrée directement au foie du patient *in situ.*

16. Anticorps selon la revendication 9, dans lequel l'anticorps est un anticorps monoclonal, un anticorps polyclonal, un anticorps chimérique, un anticorps humanisé, un anticorps à chaine simple, un fragment Fab, ou un fragment F(ab')₂.
